(19) Europäisches Patentamt
European Patent Office
Office européen des brevets

(11) EP 4 403 556 A1

(12) EUROPEAN PATENT APPLICATION
published in accordance with Art. 153(4) EPC

(43) Date of publication:
**24.07.2024 Bulletin 2024/30**

(21) Application number: **22869437.8**

(22) Date of filing: **16.09.2022**

(51) International Patent Classification (IPC):
***C07D 487/04*** *(2006.01)* ***A61P 35/00*** *(2006.01)*
***A61K 31/519*** *(2006.01)*

(52) Cooperative Patent Classification (CPC):
**A61K 31/519; A61P 35/00; C07D 487/04**

(86) International application number:
**PCT/CN2022/119451**

(87) International publication number:
**WO 2023/041071 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.09.2021 CN 202111117969**
**02.03.2022 CN 202210197158**
**02.03.2022 CN 202210197277**

(71) Applicant: **Shandong New Time Pharmaceutical Co., Ltd.**
**Feixian LINYI CITY, Shandong 273400 (CN)**

(72) Inventors:
- **ZHANG, Guimin**
  **Linyi, Shandong 276006 (CN)**
- **ZHANG, Hao**
  **Linyi, Shandong 276006 (CN)**
- **YAO, Jingchun**
  **Linyi, Shandong 276006 (CN)**
- **YUAN, Jiang**
  **Linyi, Shandong 276006 (CN)**
- **ZHAO, Guifang**
  **Linyi, Shandong 276006 (CN)**
- **LI, Rui**
  **Linyi, Shandong 276006 (CN)**
- **LI, Guangyan**
  **Linyi, Shandong 276006 (CN)**
- **ZHAO, Yun**
  **Linyi, Shandong 276006 (CN)**
- **LIANG, Hongbao**
  **Linyi, Shandong 276006 (CN)**
- **ZHANG, Zhenjun**
  **Linyi, Shandong 276006 (CN)**
- **ZHU, Xiangxia**
  **Linyi, Shandong 276006 (CN)**

(74) Representative: **Held, Stephan**
**Meissner Bolte Patentanwälte**
**Rechtsanwälte Partnerschaft mbB**
**Widenmayerstraße 47**
**80538 München (DE)**

(54) **EGFR INHIBITOR, PREPARATION METHOD THEREFOR AND USE THEREOF**

(57) Disclosed are a compound

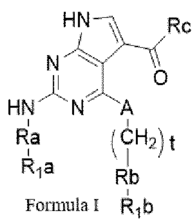

Formula I

of formula I or a pharmaceutically acceptable salt thereof, and use thereof in regulating and controlling an EGFR tyrosine kinase activity or preventing and treating an EGFR-related disease. The EGFR inhibitor of formula I has an inhibitory activity on EGFR D770-N771 ins NPG and NPG/T790M kinases, and an inhibitory effect on an EGFR-Del19/T790M/C797S kinase and cell proliferation of a KC-0122:Ba/F3EGFR-L858R/T790M/C797S three-mutation cell line and a KC-0116:Ba/F3EGFR-Del19/T790M/C797S three-mutation cell line.

EP 4 403 556 A1

## Description

### Technical Field

The present invention relates to the field of pharmaceutical chemistry and pharmacotherapeutics, and an EGFR inhibitor and a preparation method therefor, and further relates to use of the compound in preparing an anti-cancer drug.

### BACKGROUND ART

*EGFR* mutation mainly occurs in No. 18-21 exons, wherein deletion mutation of No. 19 exon (Del19) and L858R point mutation of No. 21 exon are the most common EGFR mutation subtypes, account for 90% of all mutation types, and are called sensitive mutation of the *EGFR* gene. The first generation of EGFR inhibitors (gefitinib, erlotinib, and icotinib) are developed for the *EGFR* mutation. However, after a majority of patients with non-small cell lung cancer (NSCLC) take the medicines, they will develop an acquired drug resistance within 6-12 months. T790M mutation is the primary cause of the drug resistance.

To overcome the T790M drug resistance, researchers develop second-generation EGFR inhibitors such as afatinib, dacomitinib, and neratinib. However, the second-generation inhibitors have poor selectivity, also show strong inhibitory activity on wild-type EGFR (EGFR-WT), have low maximum tolerated dose and narrow therapeutic window, and are therefore limited in clinical use.

In order to overcome the problem of poor selectivity of the second-generation EGFR-TKIs, third-generation EGFR inhibitors represented by osimertinib are successfully developed. However, the acquired resistance of the third-generation inhibitors is still unavoidable. EGFR-C797S mutation accounts for about 20% of the many resistance mechanisms and is one of the major resistance mechanisms. When the C797S mutation occurs in cis, no medicine is available clinically at present. Therefore, overcoming the drug resistance of C797S and developing fourth-generation EGFR-TKIs with high efficiency and low toxicity become the consensus of domestic and foreign counterparts, and are also the key points and difficulties in the research and development of new drugs for NSCLC. At present, although the fourth-generation EGFR small molecules are reported successively, most of them are in a preclinical research stage, few compounds enter clinical trials, and no medicines are on the market.

It is of great clinical significance and application prospect to research and develop a drug-resistant novel fourth-generation EGFR inhibitor for selectively inhibiting the C797S mutation.

### SUMMARY

The present invention discloses an EGFR inhibitor, a preparation method for and a medical use thereof. The method for preparing the inhibitor in the present invention has a mild and easily-controlled condition, convenient and simple post-treatment, and also practicability and universality at the same time. Pharmacological experiment results show that the compound of the present invention has a significant inhibitory activity on EGFR D770-N771 ins NPG and EGFR D770-N771 ins NPG/T790M kinases. The compound has an excellent inhibitory activity on EGFR-Del19/T790M/C797S kinases, an inhibitory activity on a KC-0122:Ba/F3 EGFR-L858R/T790M/C797S three-mutation cell line and a KC-0116:Ba/F3EGFR-Del19/T790M/C797S three-mutation cell line, and has a stronger inhibitory activity on single-mutation and double-mutation cells at the same time.

An objective of the present invention is to provide a compound show in formula I, a pharmaceutically acceptable salt thereof, or a stereoisomer thereof,

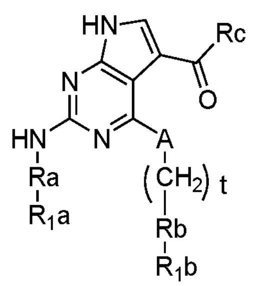

式I

wherein the Rc is

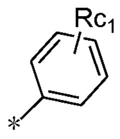 or 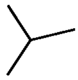 ;

the number of the $Rc_1$ is 0, 1, 2, or 3; each $Rc_1$ is independently selected from at least one of halogen and $C_{1-3}$ alkyl; the Ra is a substituted or unsubstituted phenyl, or a substituted or unsubstituted

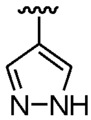 ;

the substituents are each independently selected from halogen, $C_{1-3}$ alkyl, and $C_{1-3}$ alkoxy; the number of the respective substituents of the phenyl or the

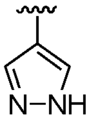

is 0, 1, or 2; the halogen is F, Cl, Br, and I;
the $R_1a$ is selected from H,

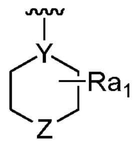 , 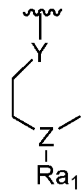 ,

or $C_{1-3}$ alkyl; the Y and the Z are each independently selected from N, O, and C, wherein the N and the C are substituted by 0 or 1 $C_{1-3}$ alkyl and the insufficient valence bonds are complemented by H; and the $Ra_1$ is H or $C_{1-3}$ alkyl;
the A is O or N, wherein the N is substituted by at least one of H and $C_{1-3}$ alkyl;
the t is selected from 0, 1, 2, or 3;
the Rb is selected from groups in one or more groups of the following 1)-5):

1) a substituted or unsubstituted 5- to 8-membered spiroheterocycle, wherein a heteroatom in the spiroheterocycle is O, S, or N; the substituent of the spiroheterocycle is one or more independent substituted or unsubstituted amino, $C_{1-3}$ alkyl, $C_{1-3}$ alkoxy, and OH; and the substituent of the amino is H or one or two $C_{1-3}$ alkyls;
2) a substituted or unsubstituted $C_8$-$C_{11}$ bridge ring, wherein the substituent of the bridge ring is one or more independent substituted or unsubstituted amino, $C_{1-3}$ alkyl, $C_{1-3}$ alkoxy, and OH; and the substituent of the amino is H or one or two $C_{1-3}$ alkyls;
3) a substituted or unsubstituted amino, wherein the substituent of the amino is H or one or two $C_{1-3}$ alkyls, or $-(CH_2)_a-E-(CH_2)_b-$, and N in the amino is linked with both ends of the $-(CH_2)_a-E-(CH_2)_b-$ to form a ring; the E is C or O, and the a is 1, 2, 3, or 4; and the b is 1, 2, 3, or 4;
4)

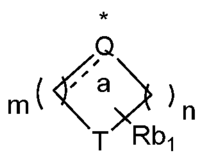 ,

wherein the number of the double bonds a is 0, 1, 2, or 3; the position of the a is any position with a reasonable valence bond in the ring; the insufficient valence bonds are complemented by H;

the m is 0, 1, 2, or 3;
the n is 0, 1, 2, or 3;
the Q and the T are each independently N, O, S, or C; and the insufficient valence bonds are complemented by H; and

5)

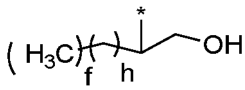,

wherein the f is 1, 2, or 3; and the h is 0, 1, or 2;

the number of the $Rb_1$ is 0, 1, or 2; the $Rb_1$ is amino, hydroxy, $C_{1-3}$ alkyl, $C_{1-3}$ alkoxy, $C_{1-3}$ alkyl substituted by hydroxy, $SO_2R_2b$,

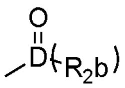, or 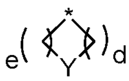;

the $R_2b$ is a substituted or unsubstituted $C_{1-3}$ alkyl, a substituted or unsubstituted phenyl, $C_{3-6}$ cycloalkyl, $C_{2-5}$ alkenyl, or

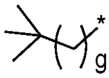;

the substituent of the $C_{1-3}$ alkyl or the phenyl is halogen or hydroxyl; the D is selected from C, P, or S; the g is 1, 2, or 3; the e and the d are each independently selected from 0, 1, 2, or 3; and the Y is selected from C, O, or N; and the $R_1b$ is H, $NH_2$, hydroxyl, $C_{1-3}$ alkoxy, $C_{1-3}$ alkyl substituted by hydroxyl, $SO_2R_2b$, $C_{1-3}$ alkyl,

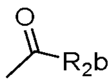, 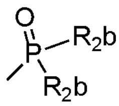, or 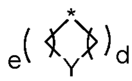;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

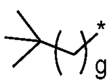;

the substituent of the $C_{1-3}$ alkyl or the phenyl is halogen or hydroxyl; the g is 1, 2, or 3; the e and the d are each independently selected from 0, 1, 2, or 3; the Y is selected from C, O, or N; the $C_{1-3}$ alkyl is methyl, ethyl, propyl, or isopropyl; the $C_{1-3}$ alkoxy is methoxy, ethoxy, propoxy, or isopropoxy; and the halogen is F, Cl, Br, or I.

In some examples, the compound shown in formula I, a stereoisomer thereof, or a pharmaceutically acceptable salt thereof, wherein the substituent Rc thereof is selected from any one of the following groups:

the number of the $Rc_1$ is 0, 1, 2, or 3; each $Rc_1$ is independently selected from at least one of F, Cl, Br, I, and $C_{1-3}$ alkyl;

**the** Rc is

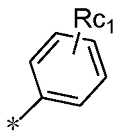 or 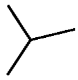 ;

the number of the $Rc_1$ is 1, 2, or 3; and each $Rc_1$ is independently selected from at least one of F, Cl, Br, methyl, ethyl, and propyl;

the Rc is

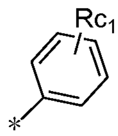 or 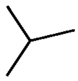 ,

and the number of the $Rc_1$ is 0, 1, 2, or 3; and each $Rc_1$ is independently selected from at least one of F, Cl, and $C_{1-3}$ alkyl;

the Rc is

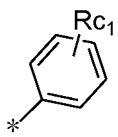 or 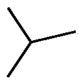 ,

and the number of the $Rc_1$ is 0, 1, 2, or 3; and each $Rc_1$ is independently selected from at least one of F, Cl, and methyl;

the Rc is

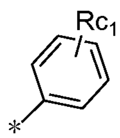 ,

and the number of the $Rc_1$ is 0, 1, 2, or 3; each $Rc_1$ is independently selected from at least one of F and $C_{1-3}$ alkyl; and further the $C_{1-3}$ alkyl is selected from methyl or ethyl;

**the** Rc is

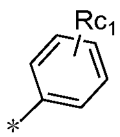 ,

and the number of the $Rc_1$ is 0, 1, 2, or 3; and each $Rc_1$ is independently selected from at least one of F, Cl, and methyl;

**the** Rc is

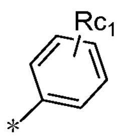 ,

and the number of the $Rc_1$ is 1, 2, or 3; and each $Rc_1$ is independently selected from at least one of F, Cl, and methyl;

**the** Rc is

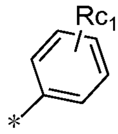

,

and the number of the $Rc_1$ is 0, 1, or 3; and each $Rc_1$ is independently selected from at least one of F and methyl;

the Rc is

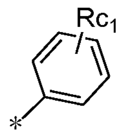

,

and the number of the $Rc_1$ is 1 or 3; and each $Rc_1$ is independently selected from at least one of F;

the Rc is

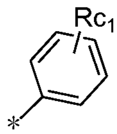

and the $Rc_1$ is a single F;

the Rc is

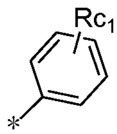

and the $Rc_1$ is F; and the number of the $Rc_1$ is 3, wherein at least two are respectively at the ortho-position and para-position of the benzene ring;

the Rc is

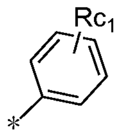

and the $Rc_1$ is F; and the number of the $Rc_1$ is 0, 1, or 3;

**the** Rc is

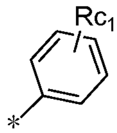

and the number of the $Rc_1$ is 0 or the $Rc_1$ is 1 F or 3 Fs or one methyl;

the Rc is

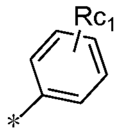

and the number of the $Rc_1$ is 0 or the $Rc_1$ is 1 F or 3 Fs or one methyl, and the preferable position is the para-position of the benzene ring;

**the** Rc is

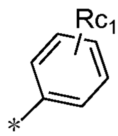

,

and the number of the $Rc_1$ is 1, 2, or 3; and each $Rc_1$ is independently selected from at least one of F and Cl;

the Rc is

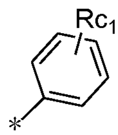

,

and the number of the $Rc_1$ is 1 or 2; and each $Rc_1$ is independently selected from at least one of F and Cl;

the Rc is

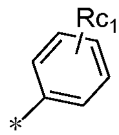

,

and the number of the $Rc_1$ is 1 or 2; and each $Rc_1$ is independently selected from F or a combination of F and Cl;

the Rc is

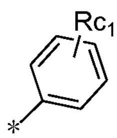

and the $Rc_1$ is 1 or 2 or 3 Fs;

the Rc is

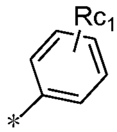

and the $Rc_1$ is 3 Fs;

the Rc is

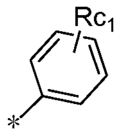

and the Rc1 is 2 Fs; and

the Rc is

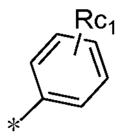
,

and the Rc1 is 1 F and at the para-position of the benzene ring.

In some examples, the compound shown in formula I, a stereoisomer thereof, or a pharmaceutically acceptable salt thereof, the substituent Ra thereof is selected from any one of the following groups:

the Ra is a substituted or unsubstituted phenyl, or a substituted or unsubstituted

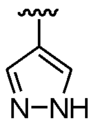
;

the substituents are each independently selected from F, $C_{1-3}$ alkyl, and $C_{1-3}$ alkoxy; the number of the respective substituents of the phenyl or the

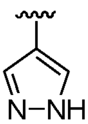

is 0, 1, or 2; the halogen is F, Cl, Br, and I; the substituent of the phenyl or the

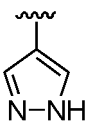

in the Ra is F, Cl, $C_{1-3}$ alkyl, and $C_{1-3}$ alkoxy; and the number of the respective substituents of the phenyl or the

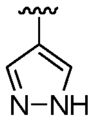

is 0, 1, or 2;
each substituent of the phenyl or the

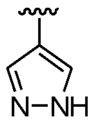

in the Ra is independently selected from F, Cl, methyl, ethyl, methoxy, and ethoxy; and the number of the respective substituents of the phenyl or the

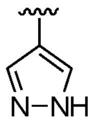

is 0 or 1;
the substituent of the phenyl or the

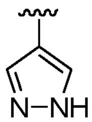

in the Ra is F, methyl, and methoxy; and the number of the respective substituents of the phenyl or the

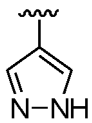

is 0 or 1;
the Ra is phenyl;
the substituent of the phenyl or the

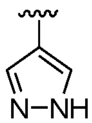

in the Ra is F, methyl, and methoxy; and the number of the respective substituents of the phenyl or the

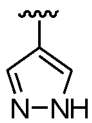

is 1; and
the Ra is a substituted or unsubstituted phenyl, or a substituted or unsubstituted

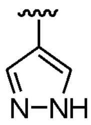 ;

the substituent of the phenyl or the

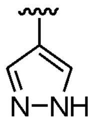

in the Ra is methyl; and the number of the respective substituents of the phenyl or the

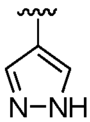 is 1.

In some examples, the compound shown in formula I, a stereoisomer thereof, or a pharmaceutically acceptable salt thereof, wherein the substituent $R_1a$ thereof is selected from any one of the following groups:

the $R_1a$ is selected from H,

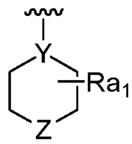 , 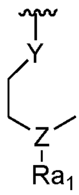 ,

or $C_{1-3}$ alkyl; the Y and the Z are each independently selected from N, O, and C, and the insufficient valence bonds are complemented by H; and the $Ra_1$ is H or $C_{1-3}$ alkyl;

the $R_1a$ is selected from H,

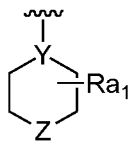 , 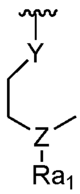 ,

or $C_{1-3}$ alkyl; the Y is C, O, or N; the Z is N or O, wherein the N is substituted by 0 or 1 $C_{1-3}$ alkyl, and the insufficient valence bonds are complemented by H; and the $Ra_1$ is H or $C_{1-3}$ alkyl;

the $R_1a$ is selected from H,

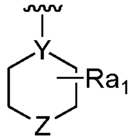, 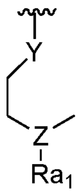,

or $C_{1-3}$ alkyl; the Y is C, O, or N; and the Z is N or O, wherein the N is substituted by 0 or 1 $C_{1-3}$ alkyl and the insufficient valence bonds are complemented by H;

the $R_1a$ is selected from H,

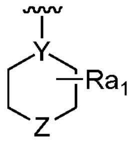, 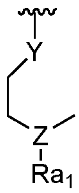,

or $C_{1-3}$ alkyl; the Y is C or N; the Z is N or O, wherein the N is substituted by 0 or 1 methyl and the insufficient valence bonds are complemented by H; and the $Ra_1$ is H, $CH_3$, or $CH_2CH_3$;

the $R_1a$ is selected from H or

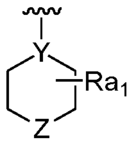;

the Y is N; the Z is N or O; the insufficient valence bonds are complemented by H; and the $Ra_1$ is H or $CH_3$;

the $R_1a$ is selected from

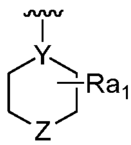;

the Y is N; the Z is N or O; the insufficient valence bonds are complemented by H; and the $Ra_1$ is H or $CH_3$;

the $R_1a$ is selected from H,

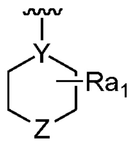, 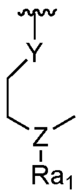,

or $C_{1-3}$ alkyl; the Y is C or N; the Z is N or O; and the $Ra_1$ is H or $C_{1-3}$ alkyl;

the $R_1a$ is selected from H,

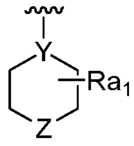,

or $C_{1-3}$ alkyl; the Y is N; the Z is N or O; and the $Ra_1$ is H or methyl;

the $R_1a$ is selected from H,

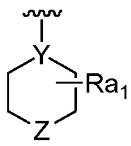,

or $C_{1-3}$ alkyl; the Y is C or N; the Z is N; and the $Ra_1$ is H or methyl; and

the $R_1a$ is selected from

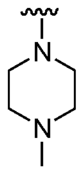, 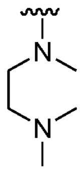, 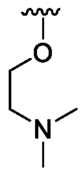, 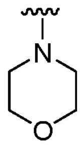, 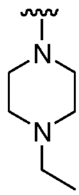, and 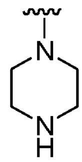.

In some examples, the compound shown in formula I, a stereoisomer thereof, or a pharmaceutically acceptable salt thereof, wherein the substituent $R_1a$ thereof is selected from any one of the following groups:

the $Ra_1$ is H or $C_{1-3}$ alkyl;
the $Ra_1$ is H, $CH_3$, or $CH_2CH_3$;
the $Ra_1$ is $CH_3$ or $CH_2CH_3$;
the $Ra_1$ is methyl; and
the $Ra_1$ is ethyl.

In some examples, the compound shown in formula I, a stereoisomer thereof, or a pharmaceutically acceptable salt thereof, wherein the substituent A thereof is selected from any one of the following groups:

the A is O or N, wherein the N is substituted by at least one of H and $C_{1-3}$ alkyl;
the A is O or N, wherein the N is substituted by at least one of H or methyl;
the A is O or N, wherein the N is substituted by H; and
the A is N, wherein the N is substituted by H.

In some examples, the compound shown in formula I, a stereoisomer thereof, or a pharmaceutically acceptable salt thereof, wherein the t is selected from any one of the following groups:

the t is selected from 0, 1, or 2;
the t is selected from 0 or 2;
the t is selected from 0;
the t is selected from 1;
the t is selected from 2; and
the t is selected from 3.

In some examples, the compound shown in formula I, a stereoisomer thereof, or a pharmaceutically acceptable salt thereof, wherein the number of the $Rb_1$ is 0, 1, or 2; the $Rb_1$ is amino, hydroxy, $C_{1-3}$ alkyl, $C_{1-3}$ alkoxy, $C_{1-3}$ alkyl substituted by hydroxy, $SO_2R_2b$,

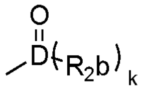 , or 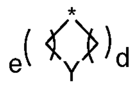 ;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

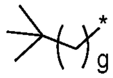 ;

the substituent of the $C_{1-3}$ alkyl or the phenyl is halogen or hydroxyl; the D is selected from C or P; the k is 1 or 2; the g is 1, 2, or 3; the e and the d are each independently selected from 0, 1, 2, or 3; and the Y is selected from C, O, or N.

In some examples, the compound shown in formula I, a stereoisomer thereof, or a pharmaceutically acceptable salt thereof, wherein the number of the $Rb_1$ is 0, 1, or 2; the $Rb_1$ is amino, hydroxy, $C_{1-3}$ alkyl, $C_{1-3}$ alkoxy, or $C_{1-3}$ alkyl substituted by hydroxy.

In some examples, the compound shown in formula I, a stereoisomer thereof, or a pharmaceutically acceptable salt thereof, wherein the Rb is selected from groups in one or more groups of the following 1)-5):

1) a substituted or unsubstituted 5- to 8-membered spiroheterocycle, wherein a heteroatom in the spiroheterocycle is O;
2) a substituted or unsubstituted 10 bridge ring, wherein the substituent of the bridge ring is one or more independent amino, $C_{1-3}$ alkyl, $C_{1-3}$ alkoxy, and OH;
3) a substituted or unsubstituted amino, wherein the substituent of the amino is one or two $C_{1-3}$ alkyls, or $-(CH_2)_a-E-(CH_2)_b-$, and N in the amino is linked with both ends of the $-(CH_2)_a-E-(CH_2)_b-$ to form a ring; the E is C or O, and the a is 1, 2, or 3; and the b is 1, 2, or 3;
4)

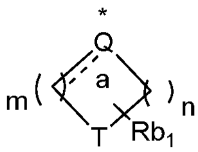 ,

wherein the number of the double bonds a is 0, 1, 2, or 3; the position of the a is any position with a reasonable valence bond in the ring; the insufficient valence bonds are complemented by H; the m is 0, 1, 2, or 3; the n is 0, 1, 2, or 3; the Q and the T are each independently N, O, S, or C; and the insufficient valence bonds are complemented by H; and
5)

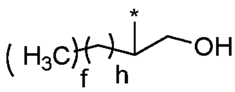 ,

wherein the f is 1, 2, or 3; and the h is 0 or 1; and

the number of the $Rb_1$ is 0, 1, or 2; the $Rb_1$ is amino, hydroxy, $C_{1-3}$ alkyl, $C_{1-3}$ alkoxy, $C_{1-3}$ alkyl substituted by hydroxy, $SO_2R_2b$,

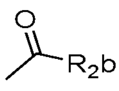 , 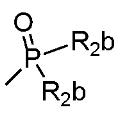 , or 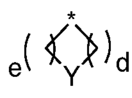 ;

each $R_2b$ is independently a substituted or unsubstituted $C_{1-3}$ alkyl, a substituted or unsubstituted phenyl, $C_{3-6}$ cycloalkyl,

$C_{2-5}$ alkenyl, or

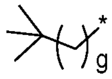;

the substituent of the $C_{1-3}$ alkyl or the phenyl is halogen or hydroxyl; the g is 1, 2, or 3; the e and the d are each independently selected from 0, 1, 2, or 3; and the Y is selected from C, O, or N.

In some examples, the compound shown in formula I, a stereoisomer thereof, or a pharmaceutically acceptable salt thereof, wherein the Rb is selected from

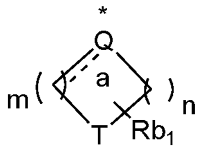;

the number of the double bonds a is 0, 1, 2, or 3; the position of the a is any position with a reasonable valence bond in the ring; the insufficient valence bonds are complemented by H; the m is 0, 1, 2, or 3; the n is 0, 1, 2, or 3; the Q and the T are each independently N, O, S, or C; and the insufficient valence bonds are complemented by H.

In some examples, the compound shown in formula I, a stereoisomer thereof, or a pharmaceutically acceptable salt thereof, wherein the number of the $Rb_1$ is 0 or 1; the $Rb_1$ is amino, hydroxy, hydroxymethyl, $C_{1-3}$ alkyl, $C_{1-3}$ alkoxy, $C_{1-3}$ alkyl substituted by hydroxy, $SO_2R_2b$,

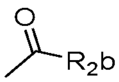, or 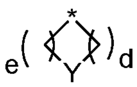;

each $R_2b$ is independently a substituted or unsubstituted $C_{1-3}$ alkyl, cyclopropyl, or $C_{2-3}$ alkenyl; the substituent of the $C_{1-3}$ alkyl is halogen or hydroxyl; the e and the d are each independently selected from 0, 1, 2, or 3; and the Y is selected from C, O, or N.

In some examples, the compound shown in formula I, a stereoisomer thereof, or a pharmaceutically acceptable salt thereof, wherein the number of the $Rb_1$ is 0, 1, or 2; and the $Rb_1$ is selected from any one of the following groups:

the $Rb_1$ is amino, hydroxy, $C_{1-3}$ alkyl, $C_{1-3}$ alkoxy, or $C_{1-3}$ alkyl substituted by hydroxy;
the $Rb_1$ is $C_{1-3}$ alkyl or $C_{1-3}$ alkoxy;
the $Rb_1$ is amino, hydroxy, $C_{1-3}$ alkyl, $C_{1-3}$ alkoxy, or $C_{1-3}$ alkyl substituted by hydroxy;
the $Rb_1$ is amino, hydroxy, or $C_{1-3}$ alkyl substituted by hydroxy;
the $Rb_1$ is amino or hydroxymethyl;
the $Rb_1$ is hydroxymethyl;

the $Rb_1$ is amino;
the $Rb_1$ is hydroxy;
the $Rb_1$ is hydroxyethyl; and
the $Rb_1$ is methyl, ethyl, or propyl.

In some examples, the compound shown in formula I, a stereoisomer thereof, or a pharmaceutically acceptable salt thereof, wherein the Rb is selected from any one of the following groups:

the Rb is

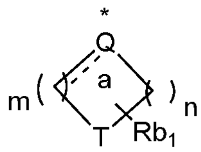,

wherein the Q and the T are both C; the a is 0 and the insufficient valence bonds are complemented by H; the m is 2; and the n is 2;

the Rb is

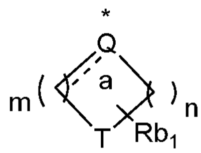,

wherein the Q is C; the T is C and O; the a is 0 or 1 and the insufficient valence bonds are complemented by H; the m is 1, 2, or 3; and the n is 0, 1, or 2;

the Rb is

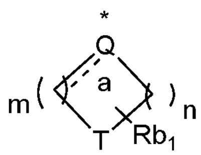,

wherein the Q is C; the T is O or N; the a is 0 and the insufficient valence bonds are complemented by H; the m is 2 or 3; and the n is 1 or 2;

the Rb is

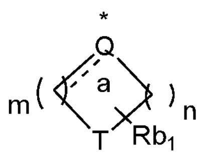,

wherein the Q is C; the T is O; the a is 0 and the insufficient valence bonds are complemented by H; the m is 3; and the n is 1;

the Rb is

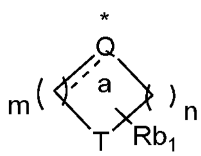,

wherein the Q and the T are both C; the a is 0 and the insufficient valence bonds are complemented by H; the m is 1; and the n is 1;

the Rb is

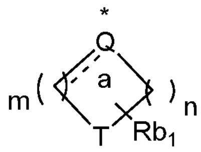

,

wherein the Q and the T are both C; the a is 1 and the insufficient valence bonds are complemented by H; the m is 2; and the n is 1;

the Rb is

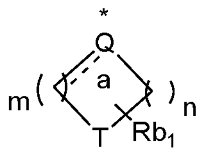

,

wherein the Q is C; the T is C; the a is 0 and the insufficient valence bonds are complemented by H; the m is 1; and the n is 0;

the Rb is

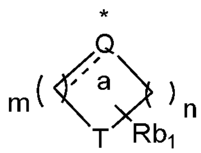

,

wherein the Q is C or N; the T is C, O, or N; the a is 0, 1, or 3 and the insufficient valence bonds are complemented by H; the m is 1, 2, or 3; and the n is 1 or 2;

the Rb is

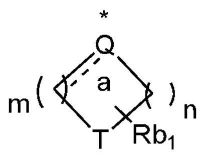

,

wherein the Q is C; the T is N; the a is 0 and the insufficient valence bonds are complemented by H; the m is 2 or 3; and the n is 1;

the Rb is

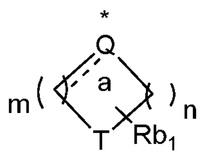

,

wherein the Q is C; the T is O or N; the a is 0 and the insufficient valence bonds are complemented by H; the m is

3; and the n is 1;

the Rb is

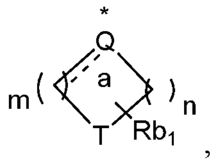

wherein the Q is C; the T is O or N; the a is 0 and the insufficient valence bonds are complemented by H; the m is 2; and the n is 2;

the Rb is

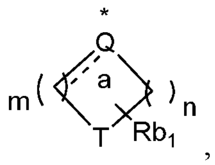

wherein the Q is C; the T is C or O; the a is 0 and the insufficient valence bonds are complemented by H; the m is 2; and the n is 2;

the Rb is

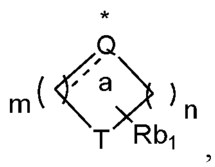

wherein the Q is C; the T is C, O, or N; the a is 0 and the insufficient valence bonds are complemented by H; the m is 2 or 3; and the n is 1 or 2;

the Rb is

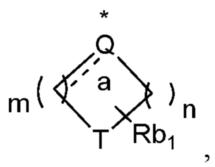

wherein the Q is C; the T is C or N; the a is 0 and the insufficient valence bonds are complemented by H; the m is 2 or 3; and the n is 1 or 2; and

the Rb is

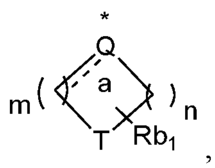

wherein the Q is C or N; the T is C, O, or N; the a is 0, 1, or 3 and the insufficient valence bonds are complemented by H; the m is 1, 2, or 3; and the n is 1 or 2.

In some examples, the compound shown in formula I, a stereoisomer thereof, or a pharmaceutically acceptable salt thereof, wherein the $R_1b$ is selected from any one of the following groups: the $R_1b$ is H, $NH_2$, hydroxyl, $C_{1-3}$ alkoxy, $C_{1-3}$ alkyl substituted by hydroxyl, $SO_2R_2b$, $C_{1-3}$ alkyl,

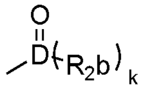, or 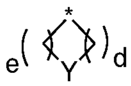;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

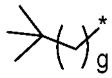;

the substituent of the $C_{1-3}$ alkyl or the phenyl is halogen or hydroxyl; the D is selected from C or P; the k is 1 or 2; the g is 1, 2, or 3; the e and the d are each independently selected from 0, 1, 2, or 3; and the Y is selected from C, O, or N;

the $R_1b$ is H, $NH_2$, hydroxymethyl, hydroxyethyl, hydroxypropyl, methyl, ethyl, propyl, isopropyl, OH, $SO_2R_2b$, $((CH_3)_2CH_2$,

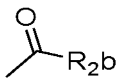, 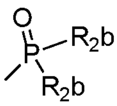, or 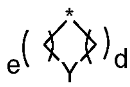;

each $R_2b$ is independently a substituted or unsubstituted $C_{1-3}$ alkyl, a substituted or unsubstituted phenyl, $C_{3-5}$ cycloalkyl, $C_{2-3}$ alkenyl, or

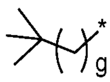;

the substituent of the phenyl is halogen or hydroxyl; the D is selected from C, P, or S; the k is 1 or 2; the g is 1, 2, or 3; and the g is 1, 2, or 3;
the $R_1b$ is H, $NH_2$, hydroxymethyl, hydroxyethyl, hydroxypropyl, methyl, ethyl, propyl, isopropyl, OH, $SO_2R_2b$, $((CH_3)_2CH_2$,

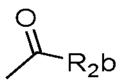, 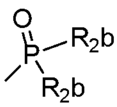, or 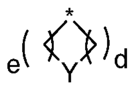;

each $R_2b$ is independently a substituted or unsubstituted $C_{1-3}$ alkyl, a substituted or unsubstituted phenyl, $C_{3-5}$ cycloalkyl, $C_{2-3}$ alkenyl, or

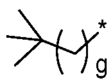;

the substituent of the phenyl is halogen or hydroxyl; the D is selected from C or P; the k is 1 or 2; the g is 1, 2, or 3; and the g is 1, 2, or 3;
the $R_1b$ is H, $NH_2$, $HOCH_2$, OH, $SO_2R_2b$, $CH_3$, $CH_3CH_2$, $(CH_3)_2CH_2$,

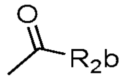,

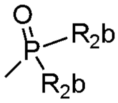, or 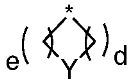;

each $R_2b$ is independently a substituted or unsubstituted $C_{1-3}$ alkyl, cyclopropyl, cyclobutyl, cyclopentyl, cyclooctyl, ethenyl, propenyl, or

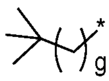;

and the g is 1, 2, or 3;
the $R_1b$ is H, $NH_2$, $HOCH_2$, OH, $SO_2R_2b$, $CH_3$, $CH_3CH_2$, $(CH_3)_2CH_2$,

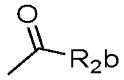,

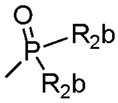, or 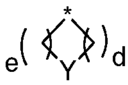;

each $R_2b$ is independently methyl, ethyl, propyl, isopropyl, hydroxymethyl, hydroxyethyl, hydroxypropyl, cyclopropyl, cyclobutyl, cyclopentyl, cyclooctyl, ethenyl, propenyl, or

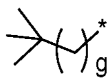;

and the g is 1, 2, or 3;
the $R_1b$ is H, amino, hydroxy, $C_{1-3}$ alkyl substituted by hydroxy, $C_{1-3}$ alkoxy,

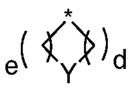,

or $SO_2R_2b$, and each $R_2b$ is independently selected from $C_{1-3}$ alkyl or $C_{3-6}$ cycloalkyl; and the e and the d are each independently selected from 1, 2, or 3; and the Y is selected from C, N, or O;
the $R_1b$ is H, amino, hydroxy, $C_{1-3}$ alkyl, $C_{1-3}$ alkyl substituted by hydroxy, $C_{1-3}$ alkoxy, or $SO_2R_2b$, and each $R_2b$ is independently selected from $C_{1-3}$ alkyl;
the $Rb_1$ is H, amino, hydroxy, $C_{1-3}$ alkyl, $C_{1-3}$ alkoxy, or $C_{1-3}$ alkyl substituted by hydroxy;
the $Rb_1$ is H, amino, hydroxy, or $C_{1-3}$ alkyl substituted by hydroxy; and
the $Rb_1$ is H, amino, or $C_{1-3}$ alkyl substituted by hydroxy.

In some examples, the compound shown in formula I, a stereoisomer thereof, or a pharmaceutically acceptable salt thereof, wherein the $C_{1-3}$ alkyl is methyl, ethyl, propyl, or isopropyl; the $C_{1-3}$ alkoxy is methoxy, ethoxy, propoxy, or isopropoxy; and the halogen is F, Cl, Br, or I.

In some examples, the compound shown in formula I, a stereoisomer thereof, or a pharmaceutically acceptable salt thereof, wherein the $C_{1-3}$ alkyl is methyl and ethyl.

In some examples, the compound shown in formula I, a stereoisomer thereof, or a pharmaceutically acceptable salt thereof, wherein the $C_{1-3}$ alkoxy is methoxy, ethoxy, propoxy, or isopropoxy

In some examples, the compound shown in formula I, a stereoisomer thereof, or a pharmaceutically acceptable salt thereof, wherein the halogen is F, Cl, Br, or I.

In some examples, the compound shown in formula I, a stereoisomer thereof, or a pharmaceutically acceptable salt thereof, wherein the halogen is F or/and Cl.

In some examples, the $Rc_1$ is at least one of H, F, Cl, and $CH_3$.

In some examples, the number of the $Rc_1$ is 1, 2, or 3.

In some examples, the position of the $Rc_1$ is one, two, or three of the positions 2, 3, 4, 5, and 6 on the benzene ring.

In some examples, the substituent of phenyl or

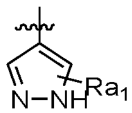

of the Ra is F and $CH_3O$-.

In some examples, the Rb is a substituted or unsubstituted 5- to 8-membered spiroheterocycle, a substituted or unsubstituted $C_8$-$C_{11}$ bridge ring, a substituted or unsubstituted amino,

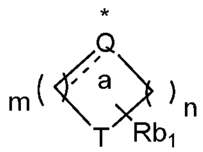 , or 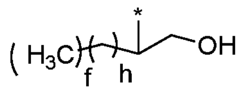 ;

a heteroatom in the spiroheterocycle is O, S, or N; the substituent of the spiroheterocycle or the bridge ring is one or more independent $NH_2$, $C_{1-3}$ alkyl, $C_{1-3}$ alkoxy, and OH; and the substituent of the amino is one or two $C_{1-3}$ alkyls; the m is 0, 1, 2, or 3; the n is 0, 1, 2, or 3; the Q and the T are each independently N, O, S, or C; the insufficient valence bonds are complemented by H; the f is 1, 2, or 3; and the h is 0, 1, or 2.

In some examples, the f is 2 or 3.

In some examples, the $Rb_1$ is methyl.

In some examples, each $R_2b$ is independently methyl, cyclopropyl, ethenyl, and 2,2,2-trimethyl-ethyl.

In some examples, the Rb is a substituted or unsubstituted 5- to 8-membered spiroheterocycle, a substituted or unsubstituted $C_8$-$C_{11}$ bridge ring, a substituted or unsubstituted amino,

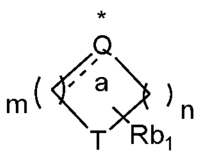 , or 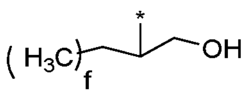 ;

a heteroatom in the spiroheterocycle is O, S, or N; the substituent of the spiroheterocycle or the bridge ring is one or more $NH_2$, $HOCH_2$, OH, and $CH_3$; and the substituent of the amino is one or two $C_{1-3}$ alkyls,

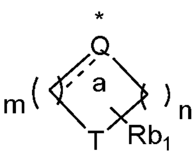 , 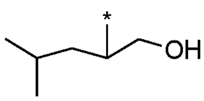 , 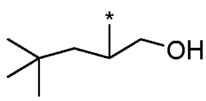 , 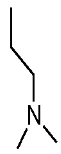 ,

and a substituted or unsubstituted 7-membered spiroheterocycle or $C_{10}$ bridge ring.

In some examples, the Rb is

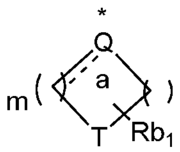, 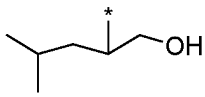, 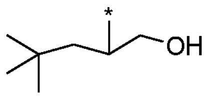,

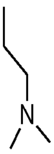,

and substituted or unsubstituted 3-oxaspiro[3,3]heptanyl or adamantyl.

In some examples, the Rb is

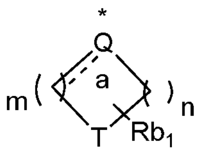.

In some examples, the Rb is

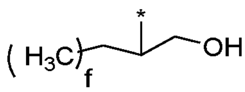;

and the f is 1, 2, or 3.

In some examples, the Rb is

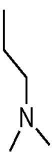.

In some examples, the Rb is adamantyl.

In some examples, the Rb is 3-oxaspiro[3,3]heptanyl.

In some examples, the Rc is

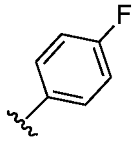;

the Ra is a substituted or unsubstituted phenyl, or a substituted or unsubstituted

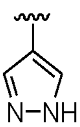;

the substituents are each independently selected from F, methyl, and methoxy; and the number of the respective substituents of the phenyl or the

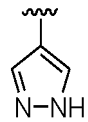

is independently 0 or 1; the $R_1a$ is selected from H,

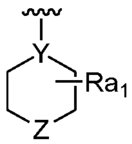
,
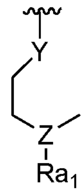
,

or methyl; the Y is selected from Nand O; and the Z is N or O;

the $Ra_1$ is H, methyl, or ethyl; the A is N, wherein N is substituted by one H; the t is 0; and the Rb is selected from groups in one or more groups of the following 1)-4):

1) a substituted or unsubstituted 7-membered spiroheterocycle, wherein a heteroatom in the spiroheterocycle is O;
2) a substituted or unsubstituted adamantyl, wherein the substituent of the adamantyl is one or more OHs;
3) a substituted or unsubstituted amino, wherein the substituent of the amino is one or two $C_{1-3}$ alkyls; and
4)

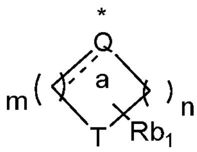
,

wherein the number of the double bonds a is 0 or 1; the position of the a is any position with a reasonable valence bond in the ring; the m is 0, 1, 2, or 3; the n is 0, 1, or 2; the Q is C; the T is N, O or C; and the insufficient valence bonds are complemented by H;

the number of the $Rb_1$ is 0, 1, or 2; the $Rb_1$ is amino, hydroxy, or hydroxymethyl; and the substituent of the $C_{1-3}$ alkyl is halogen or hydroxyl; and
the $R_1b$ is H, amino, hydroxy, or hydroxymethyl.

In some examples, the Rc is

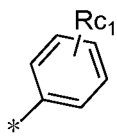
,

and the $Rc_1$ is F and located at the para-position on the benzene ring; the Ra is a substituted or unsubstituted phenyl, or a substituted or unsubstituted

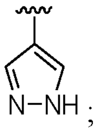

the substituents are each independently $C_{1-3}$ alkyl; the number of the respective substituents of the phenyl or the

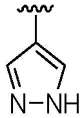

is 0 or 1; the $R_1a$ is selected from H,

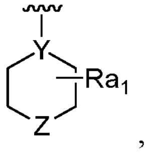

or $C_{1-3}$ alkyl; the Y is C or N; the Z is N; the $Ra_1$ is H, $CH_3$, and $CH_2CH_3$; the A is N and the N is substituted by one H; the t is selected from 0; the Rb is

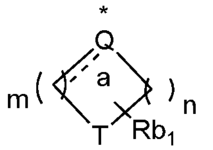

wherein the Q and the T are both C; the a is 0 and the insufficient valence bonds are complemented by H; the m is 2; and the n is 2; and

the number of the $Rb_1$ is 0, 1, or 2; the $Rb_1$ is $NH_2$, hydroxymethyl, or hydroxyethyl; and the $R_1b$ is H, $NH_2$, or $HOCH_2$.

In some examples, the Rb is selected from groups in one or more groups of the following 1)-4):

1) a substituted or unsubstituted 7-membered spiroheterocycle, wherein a heteroatom in the spiroheterocycle is O;
2) a substituted or unsubstituted adamantyl, wherein the substituent of the adamantyl is one or more OHs;
3) a substituted or unsubstituted amino, wherein the substituent of the amino is one or two C1-3 alkyls; and
4)

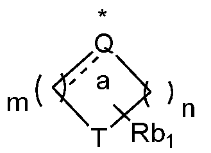

wherein the number of the double bonds a is 0 or 1; the position of the a is any position with a reasonable valence bond in the ring; the m is 0, 1, 2, or 3; the n is 0, 1, or 2; the Q is C; the T is N, O or C; and the insufficient valence bonds are complemented by H.

As shown in formula I, the Rc is a substituted or unsubstituted phenyl, and as shown in formula II

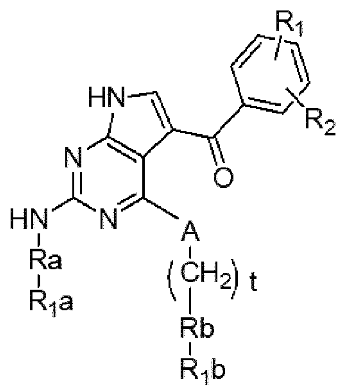

Formula II

,

the $R_1$ and the $R_2$ are each independently selected from at least one of H, halogen, and $C_{1-3}$ alkyl.

In some examples, the $R_1$ and the $R_2$ are each independently selected from at least one of H, F, Cl, Br, methyl, and ethyl.

In some examples, the $R_1$ and the $R_2$ are each independently selected from at least one of H, F, Cl, and methyl.

As shown in formula I, the Rc is a substituted or unsubstituted phenyl, the $R_a$ is selected from a substituted or unsubstituted benzene ring, and the Rb is selected from

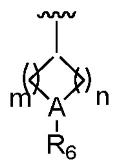

;

the $R_1$b is H and as shown in formula III

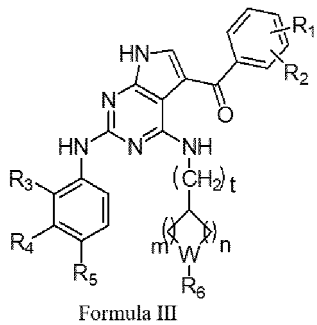

Formula III

,

wherein the m is selected from 0, 1, or 2; the n is selected from 0, 1, or 2; the t is selected from 0, 1, and 2; and the W is selected from C, O, or N;

the $R_1$ is selected from H, F, and Cl;
the $R_2$ is selected from H, F, and Cl;
the $R_3$ is selected from H and $C_{1-3}$ alkoxy;
the $R_4$ is selected from Hand F; and
the $R_5$ is selected from the range defined above for the $R_1$a.

In some examples, the $R_5$ is selected from

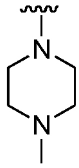, 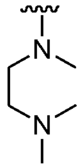, 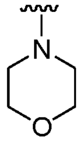, 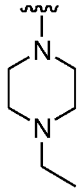,

and

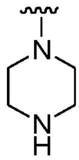.

In some examples, the $R_5$ is

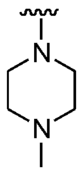.

In some examples, the $R_6$ is selected from H, $NH_2$, hydroxyl, $C_{1-3}$ alkoxy, $C_{1-3}$ alkyl substituted by hydroxyl, $SO_2R_2b$, $C_{1-3}$ alkyl,

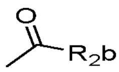, or 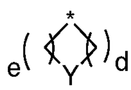;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

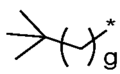;

the substituent of the $C_{1-3}$ alkyl or the phenyl is halogen or hydroxyl; the g is 1, 2, or 3; the e and the d are each independently selected from 0, 1, 2, or 3; and the Y is selected from C, O, or N.

In some examples, the $R_3$ is selected from H, methoxy, and ethoxy.

In some examples, the $R_6$ is H, $NH_2$, $HOCH_2$, OH, $SO_2R_2b$, $CH_3$, $CH_3CH_2$, $(CH_3)_2CH_2$,

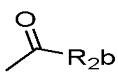, or 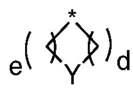;

each $R_2b$ is independently methyl, ethyl, propyl, isopropyl, hydroxymethyl, hydroxyethyl, hydroxypropyl, cyclopropyl, cyclobutyl, cyclopentyl, cyclooctyl, ethenyl, propenyl, or

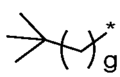;

and the g is 1, 2, or 3.

In some examples, the $R_6$ is H, amino, hydroxy, $C_{1-3}$ alkyl substituted by hydroxy, $C_{1-3}$ alkoxy,

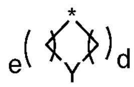,

or $SO_2R_2b$, and each $R_2b$ is independently selected from $C_{1-3}$ alkyl or $C_{3-6}$ cycloalkyl; and the e and the d are each independently selected from 1, 2, or 3; and the Y is selected from C, N, or O;

In some examples, the $R_6$ is H, amino, hydroxy, $C_{1-3}$ alkyl, $C_{1-3}$ alkyl substituted by hydroxy, $C_{1-3}$ alkoxy, or $SO_2R_2b$ and each $R_2b$ is independently selected from $C_{1-3}$ alkyl.

In some examples, the $R_6$ is H, amino, hydroxy, $C_{1-3}$ alkyl, $C_{1-3}$ alkoxy, or $C_{1-3}$ alkyl substituted by hydroxy.

In some examples, the $R_6$ is H, amino, hydroxy, or $C_{1-3}$ alkyl substituted by hydroxy.

In some examples, the $R_6$ is H, amino, or $C_{1-3}$ alkyl substituted by hydroxy.

In some examples, the $R_6$ is selected from H, OH, $CH_2OH$,

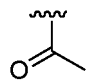, 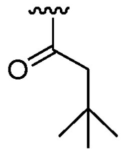,

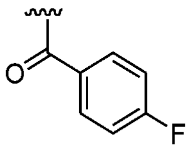, 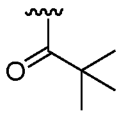, 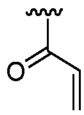, 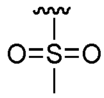, 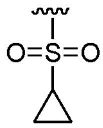, and 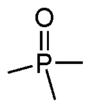.

In some examples, the $R_6$ is $CH_2OH$.

In some examples, as shown in formula I, the Ra is a substituted or unsubstituted

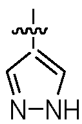,

and the substituent is at least one of H, $C_{1-3}$ alkoxy, and $C_{1-3}$ alkyl; and further, the substituent is one, two or three of H, methoxy, ethoxy, propoxy, isopropoxy, methyl, ethyl, and propyl.

In some examples, as shown in formula I, the Ra is

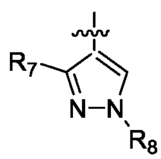

and the Rb is selected from

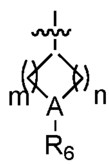;

the $R_1$b H and as shown in formula IV

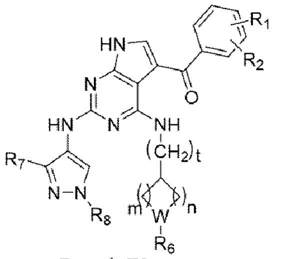

Formula IV

,

wherein the m is selected from 0, 1, or 2; the n is selected from 0, 1, or 2; the t is selected from 0, 1, and 2; and the W is C, O, or N;

the $R_1$ is selected from H, F, and Cl;
the $R_2$ is selected from H, F, and Cl;
the $R_7$ is selected from H or $C_{1-3}$ alkoxy; the $R_6$ is selected from H, OH, $CH_2OH$,

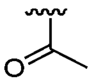, 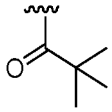, 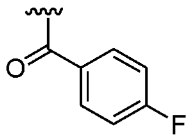, 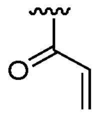, 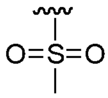, 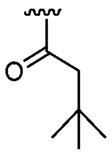, 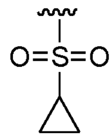, and 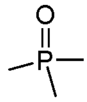;

and
the $R_8$ is selected from $C_{1-3}$ alkyl or piperidin substituted by $C_{1-3}$ alkyl.

In some examples, the $R_8$ is methyl.
In some examples, the $R_8$ is ethyl.
In some examples, the $R_8$ is methylpiperidin. In some examples, the Rb is

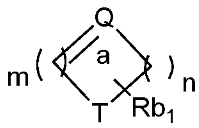, 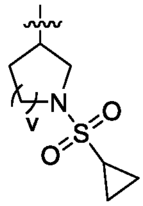, 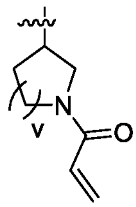, and 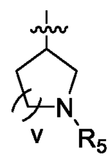,

and each v is independently 1, 2, or 3.
In some examples, the substituent of the adamantyl is at the position 2 or 3 of the adamantyl.

Some descriptions

In the description of the general formula in the present application, functional groups, substituents, or bonds are emphasized. If there are insufficient valence bonds, not specifically described, are by default complemented by H.
The linking position between the groups, not specifically described, is any position with a reasonable valence bond.
The substituent of the bridge ring is one or more independent amino groups, $C_{1-3}$ alkyl, $C_{1-3}$ alkoxy, and OH. It is independently explained that when a plurality of substituents are present, each substituent may be the same or different, such as a plurality of amino or a plurality of $C_{1-3}$ alkyl, a plurality of $C_{1-3}$ alkoxy, or a plurality of OHs, and a combination of one amino, one methyl, one ethoxy, one hydroxyl, and one propyl as a bridge ring.
"Each $R_2$b is independently selected from" only refers that if there are multiple $R_2$b in the same formula, each $R_2$b may be the same or different, and independent of each other.

"$R_1$b is selected from any one of the following groups" refers any one group of the following groups of "$R_1$b is". The selection ends before a new paragraph (space before paragraph).

Some new starting line top-written formats herein belong to the intra-paragraph content. The line division is for clarity of form and convenience of reading, and should not be used as a basis for the isolated content similar to a new paragraph.

"$R_1$b is" means that both may be the same or different groups. When the groups are the same, further substituents may be the same or different, for example

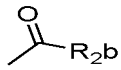
,

, the $R_2$b in the $Rb_1$ and the $R_2$b in the $R_1$b may be the same group or different groups.

In some examples, the compound is selected from the following specific compounds:

(R)-(4-((1-(cyclopropylsulfonyl)piperidin-3-yl)amino)-2-((4-(4-methylpiperazin-1-yl )phenyl)amino)-7H-pyrrolo[2,3-d]pyrimidin-5-yl)(4-fluorophenyl)methanone,
(R)-(4-(1-(cyclopropylsulfonyl)piperidin-3-yl)amino)-2-((1-methyl-1H-pyrazol-4-yl)amino)-7 H-pyrrolo[2,3-d]pyrimidin-5-yl)(4-fluorophenyl)methanone,
(R)-1-(4-((1-(cyclopropylsulfonyl)piperidin-3-yl)amino)-2-((-4-(4-methylpiperazin-1-yl)phen yl)amino)-7H-pyrrolo[2,3-d]pyrimidin-5-yl)-2-methylpropan-1-one,
(S)-(4-(1-(cyclopropylsulfonyl)piperidin-3-yl)amino)-2-((4-(4-methylpiperazin-1-yl)phenyl)a mino)-7H-pyrrolo[2,3-d]pyrimidin-5-yl)(4-fluorophenyl)methanone,
(R)-1-(3-((5-(4-fluorobenzoyl)-2-((4-(4-methylpiperazin-1-yl)phenyl)amino)-7H-pyrrolo[2,3-d]pyrimidin-4-yl)amino)piperidin-1-yl)prop-2-en-1-one,
(4-fluorophenyl)(2-((4-(4-methylpiperazin-1-yl)phenyl)amino)-4-((tetrahydro-2H-pyran-4-yl) amino)-7H-pyrrolo[2,3-d]pyrimidin-5-yl)methanone,
(4-fluorophenyl)(2-((4-(4-methylpiperazin-1-yl)phenyl)amino)-4-(1-(tetrahydro-2H-pyran-4-y l)piperidin-4-yl)amino)-7H-pyrrolo[2,3-d]pyrimidin-5-yl)methanone,
(4-fluorophenyl)(4-(((1r,4r)-4-(hydroxymethyl)cyclohexyl)amino)-2-((2-methoxy-4-morpholi nophenyl)amino)-7H-pyrrolo[2,3-d]pyrimidin-5-yl)methanone,
(3,4-difluorophenyl)(4-((1r,4r)-4-(hydroxymethyl)cyclohexyl)amino)-2-((4-morpholinopheny l)amino)-7H-pyrrolo[2,3-d]pyrimidin-5-yl)methanone,
(3-fluorophenyl)(4-(((1r,4r)-4-(hydroxymethyl)cyclohexyl)amino)-2-((4-morpholinophenyl)a mino)-7H-pyrrolo[2,3-d]pyrimidin-5-yl)methanone,
(4-fluorophenyl)(4-((1-isopropylpiperidin-4-yl)amino)-2-((4-morpholinophenyl)amino)-7H-p yrrolo[2,3-d]pyrimidin-5-yl)methanone,
(3,5-difluorophenyl)(4-(((1r,4r)-4-(hydroxymethyl)cyclohexyl)amino)-2-((4-morpholinophen yl)amino)-7H-pyrrolo[2,3-d]pyrimidin-5-yl)methanone, and
(2-chloro-4-fluorophenyl)(4-(((1r,4r)-4-(hydroxymethyl)cyclohexyl)amino)-2-((4-morpholino phenyl)amino)-7H-pyrrolo[2,3-d]pyrimidin-5-yl)methanone.

In some examples, the compound is selected from the following specific compounds:

(S)-1-(3-((5-(4-fluorobenzoyl)-2-((4-(4-methylpiperazin-1-yl)phenyl) amino-d]pyrimidin-4-yl)amino)pyrrolidin-1-yl)-2,2-dimethylpropan-1-one,
(4-fluorophenyl)(4-(((1r,4r)-4-hydroxycyclohexyl)amino)-2-((4-(4-methylpiperazin-1-yl)phe nyl)amino)-7H-pyrrolo[2,3-d]pyrimidin-5-yl)methanone,
(4-fluorophenyl)(4-((1-(hydroxymethyl)cyclopropyl)amino)-2-((4-(4-methylpiperazin-1-yl)ph enyl)amino)-7H-pyrrolo[2,3-d]pyrimidin-5-yl)methanone,
(2-((3-fluoro-4-(4-methylpiperazin-1-yl)phenyl)amino)-4-(((1r,4r)-4-(hydroxymethyl)cyclohe xyl)amino)-7H-pyrrolo[2,3-d]pyrimidin-5-yl)(4-fluorophenyl)methanone,
(4-fluorophenyl)(4-((3R,6S)-6-(hydroxymethyl)tetrahydro-2H-pyran-3-yl)amino)-2-((4-morp holinophenyl)amino)-7H-pyrrolo[2,3-d]pyrimidin-5-yl)methanone,
(4-fluorophenyl)(4-((3R,6S)-6-(hydroxymethyl)tetrahydro-2H-pyran-3-yl)amino)-2-((4-(4-me thylpiperazin-1-yl)phenyl)amino)-7H-pyrrolo[2,3-d]pyrimidin-5-yl)methanone,
(2,4-difluorophenyl)(4-(((1r,4r)-4-(hydroxymethyl)cyclohexyl)amino)-2-((4-morpholinophen yl)amino)-7H-pyrrolo[2,3-d]pyrimidin-5-yl)methanone,
(4-((2-oxaspiro[3.3]hept-6-yl)amino)-2-((4(4-methylpiperazin-1-yl)phenyl)amino)-7H-pyrrolo[2,3-d]pyrimidin-5-yl)(4-fluorophenyl)methanone,
(4-fluorophenyl)(4-(((1s,4s)-4-(hydroxymethyl)cyclohexyl)amino)-2-((4-morpholinophenyl)amino)-7H-pyrrolo[2,3-d]pyrimidin-5-yl)methanone,
(2-((3-fluoro-4-(4-methylpiperazin-1-yl)phenyl)amino)-4-(((1s,4s)-4-(hydroxymethyl)cyclohexyl)amino)-7H-pyrrolo[2,3-d]pyrimidin-5-yl)(4-fluorophenyl)methanone,
(4-fluorophenyl)(4-(((1s,4s)-4-(hydroxymethyl)cyclohexyl)amino)-2-((2-methoxy-4-(4-methylpiperazin-1-yl)phenyl)amino)-7H-pyrrolo[2,3-d]pyrimidin-5-yl)methanone,
(2-((4-(2-(dimethylamino)ethoxy)phenyl)amino)-4-(((1s,4s)-4-(hydroxymethyl)cyclohexyl)amino)-7H-pyrrolo[2,3-d]pyrimidin-5-yl)(4-fluorophenyl)methanone,
(4-fluorophenyl)(4-(((1s,4s)-4-(hydroxymethyl)cyclohexyl)amino)-2-((3-methoxy-1-methyl-1H-pyrazol-4-yl)amino)-7H-pyrrolo[2,3-d]pyrimidin-5-yl)methanone,
(4-fluorophenyl)(4-((1S,4R)-4-(hydroxymethyl)cyclopent-2-en-1-yl)amino)-2-((4-(4-methylpiperazin-1-yl)phenyl)amino)-7H-pyrrolo[2,3-d]pyrimidin-5-yl)methanone,
(4-fluorophenyl)(4-((1R,4S)-4-(hydroxymethyl)cyclopent-2-en-1-yl)amino)-2-((4-(4-methylpiperazin-1-yl)phenyl)amino)-7H-pyrrolo[2,3-d]pyrimidin-5-yl)methanone,
(4-fluorophenyl)(4-(((1r,3s,5R,7S)-3-hydroxyadamantan-1-yl)amino)-2-((4-(4-methylpiperazin-1-yl)phenyl)amino)-7H-pyrrolo[2,3-d]pyrimidin-5-yl)methanone,
(4-fluorophenyl)(4-(((1s,3R,4s,5S,7s)-4-hydroxyadamantan-1-yl)amino)-2-((4-(4-methylpiperazin-1-yl)phenyl)amino)-7H-pyrrolo[2,3-d]pyrimidin-5-yl)methanone, and
(4-fluorophenyl)(4-(((1r,4r)-4-(hydroxymethyl)cyclohexyl)amino)-2-((1-methyl-1H-pyrazol-4-yl)amino)-7H-pyrrolo[2,3-d]pyrimidin-5-yl)methanone.

In some examples, the compound is selected from the following specific compounds:

(4-fluorophenyl)(4-(((1r,4r)-4-(hydroxymethyl)cyclohexyl)amino)-2-((4-(4-methylpiperazin-1-yl)phenyl)amino)-7H-pyrrolo[2,3-d]pyrimidin-5-yl)methanone,
(4-fluorophenyl)(2-((4-(4-methylpiperazin-1-yl)phenyl)amino)-4-((tetrahydro-2H-pyran-4-yl)methyl)amino)-7H-pyrrolo[2,3-d]pyrimidin-5-yl)methanone,
(4-((2-(dimethylamino)ethyl)amino)-2-((4-(4-methylpiperazin-1-yl)phenyl)amino)-7H-pyrrolo[2,3-d]pyrimidin-5-yl)(4-fluorophenyl)methanone,
(4-fluorophenyl)(4-(((1r,4r)-4-(hydroxymethyl)cyclohexyl)amino)-2-(4-morpholinophenyl)amino)-7H-pyrrolo[2,3-d]pyrimidin-5-yl)methanone, and
(4-(((1r,4r)-4-(hydroxymethyl)cyclohexyl)amino)-2-(4-morpholinophenyl)amino)-7H-pyrrolo[2,3-d]pyrimidin-5-yl)(p-tolyl)methanone.

In some examples, the compound is selected from the following specific compounds:

(4-(cyclohexylamino)-2-((4-(4-methylpiperazin-1-yl)phenyl)amino)-7H-pyrrolo[2,3-d]pyrimidin-5-yl)(4-fluorophenyl)methanone,
(4-fluorophenyl)(4-((2-hydroxycyclohexyl)amino)-2-((4-(4-methylpiperazin-1-yl)phenyl)amino)-7H-pyrrolo[2,3-d]pyrimidin-5-yl)methanone,
(R)-(4-fluorophenyl)(2-((4-(4-methylpiperazin-1-yl)phenyl)amino)-4-(1-(methylsulfonyl)pyrrolidin-3-yl)amino)-7H-pyrrolo[2,3-d]pyrimidin-5-yl)methanone,
(R)-(4-fluorophenyl)(2-((4-(4-methylpiperazin-1-yl)phenyl)amino)-4-(1-(methylsulfonyl)piperidin-3-yl)amino)-7H-pyrrolo[2,3-d]pyrimidin-5-yl)methanone,
(S)-(4-fluorophenyl)(2-((4-(4-methylpiperazin-1-yl)phenyl)amino)-4-(tetrahydro-2H-pyran-3-yl)amino)-7H-pyrrolo[2,3-d]pyrimidin-5-yl)methanone,
(R)-(4-fluorophenyl)(2-((4-(4-methylpiperazin-1-yl)phenyl)amino)-4-(tetrahydro-2H-pyran-3-yl)amino)-7H-pyrrolo[2,3-d]pyrimidin-5-yl)methanone,
(4-fluorophenyl)(2-((4-(4-methylpiperazin-1-yl)phenyl)amino)-4-((tetrahydro-2H-pyran-4-yl)methyl)amino)-7H-pyrrolo[2,3-d]pyrimidin-5-yl)methanone,
(4-((1-(cyclopropylsulfonyl)piperidin-4-yl)amino)-2-((4-(4-methylpiperazin-1-yl)phenyl)amino)-7H-pyrrolo[2,3-d]pyrimidin-5-yl)(4-fluorophenyl)methanone,
(4-fluorophenyl)(2-((4-(4-methylpiperazin-1-yl)phenyl)amino)-4-((1-(methylsulfonyl)piperidin-4-yl)amino)-7H-pyrrolo[2,3-d]pyrimidin-5-yl)methanone,
(S)-(4-fluorophenyl)(2-((4-(4-methylpiperazin-1-yl)phenyl)amino)-4-(tetrahydrofuran-3-yl)amino)-7H-pyrrolo[2,3-d]pyrimidin-5-yl)methanone,
(R)-(4-fluorophenyl)(2-((4-(4-methylpiperazin-1-yl)phenyl)amino)-4-(tetrahydrofuran-3-yl)a mino)-7H-pyrrolo[2,3-d]pyrimidin-5-yl)methanone,
(4-fluorophenyl)(4-((1-(hydroxymethyl)cyclopentyl)amino)-2-((4-(4-methylpiperazin-1-yl)ph enyl)amino)-7H-pyrrolo[2,3-d]pyrimidin-5-yl)methanone,
(4-fluorophenyl)(4-((1r,4r)-4-methoxycyclohexyl)amino)-2-((4-(4-methylpiperazin-1-yl)phen yl)amino)-7H-pyrrolo[2,3-d]pyrimidin-5-yl)methanone,
(4-fluorophenyl)(4-(((1r,4r)-4-(hydroxymethyl)cyclohexyl)amino)-2-((2-methoxy-4-(4-methy lpiperazin-1-yl)phenyl)amino)-7H-pyrrolo[2,3-d]pyrimidin-5-yl)methanone,
(2-((3-fluoro-4-morpholinophenyl)amino)-4-(((1r,4r)-4-(hydroxymethyl)cyclohexyl)amino)-7 H-pyrrolo[2,3-d]pyrimidin-5-yl)(4-fluorophenyl)methanone,
(4-fluorophenyl)(2-((4-(4-methylpiperazin-1-yl)phenyl)amino)-4-((tetrahydro-2H-pyran-4-yl) oxy)-7H-pyrrolo[2,3-d]pyrimidin-5-yl)methanone,
(4-fluorophenyl)(2-((4-(4-methylpiperazin-1-yl)phenyl)amino)-4-((1-methylpiperidin-4-yl)am ino)-7H-pyrrolo[2,3-d]pyrimidin-5-yl)methanone,
(4-fluorophenyl)(4-(((1r,4r)-4-(hydroxymethyl)cyclohexyl)amino)-2-((3-methoxy-1-methyl-1 H-pyrazol-4-yl)amino)-7H-pyrrolo[2,3-d]pyrimidin-5-yl)methanone,
((1r,4r)-4-((2-((1-methyl-1H-pyrazol-4-yl)amino)-7H-pyrrolo[2,3-d]pyrimidin-4-yl)amino)cy clohexyl)methanol,
(4-(((1r,4r)-4-(hydroxymethyl)cyclohexyl)amino)-2-((4-morpholinophenyl)amino)-7H-pyrrol o[2,3-d]pyrimidin-5-yl)(phenyl)methanone, and
(4-(((1r,4r)-4-(hydroxymethyl)cyclohexyl)amino)-2-((4-morpholinophenyl)amino)-7H-pyrrol o[2,3-d]pyrimidin-5-yl)(2,3,4-trifluorophenyl)methanone.

In some examples, the compound is selected from the following specific compounds:

(S)-(4-(1-(cyclopropylsulfonyl)piperidin-3-yl)amino)-2-((4-morpholinophenyl)amino )-7H-pyrrolo[2,3-d]pyrimidin-5-yl(4-fluorophenyl)methanone,
((1*r*,4*r*)-4-((2-((4-morpholinophenyl)amino)-7H-pyrrolo[2,3-d]pyrimidin-4-yl)amino)cyclohe xyl)methanol,
(4-fluorophenyl)(4-((1-isopropylpiperidin-4-yl)amino)-2-((4-(4-methylpiperazin-1-yl)phenyl) amino)-7H-pyrrolo[2,3-d]pyrimidin-5-yl)methanone,
(4-fluorophenyl)(4-((1-methylpiperidin-4-yl)amino)-2-((4-morpholinophenyl)amino)-7H-pyrr olo[2,3-d]pyrimidin-5-yl)methanone.

In some examples, the compound is selected from the following specific compounds:

(4-(((1*s*,4*s*)-4-aminocyclohexyl)amino)-2-((4-(4-methylpiperazin-1-yl)phenyl)amino)-7H-pyrr olo[2,3-d]pyrimidin-5-yl)(4-fluorophenyl)methanone,
(2-((4-(4-ethylpiperazin-1-yl)phenyl)amino)-4-(((1s,4s)-4-(hydroxymethyl)cyclohexyl)amino) -7H-pyrrolo[2,3-d]pyrimidin-5-yl)(4-fluorophenyl)methanone,
(4-fluorophenyl)(4-(((1s,4s)-4-(hydroxymethyl)cyclohexyl)amino)-2-((1-methyl-1H-pyrazol-4-yl)amino)-7H-pyrrolo[2,3-d]pyrimidin-5-yl)methanone,
(4-fluorophenyl)(4-(((1s,4s)-4-(hydroxymethyl)cyclohexyl)amino)-2-((1-(1-methylpiperidin-4 -yl)-1H-pyrazol-4-yl)amino)-7H-pyrrolo[2,3-d]pyrimidin-5-yl)methanone, and
(4-fluorophenyl)(4-(((1s,4s)-4-(hydroxymethyl)cyclohexyl)amino)-2-((4-(4-methylpiperazin-1-yl)phenyl)amino)-7H-pyrrolo[2,3-d]pyrimidin-5-yl)methanone.

In some examples, the compound is selected from the following specific compounds:

(R)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(4-fluorophenyl)methanone,
(S)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(4-fluorophenyl)methanone,
(R)-1-(3-((5-(4-fluorobenzoyl)-2-((4-(4-methylbenzoyl-1-yl)phenyl)amino)-7H-pyrrolo[2,3-d] pyrimidin-4-yl)amino)piperidin-1-yl)prop-2-en-1-one,
(S)-1-(3-((5-(4-fluorobenzoyl)-2-((4-(4-methylpiperazin-1-yl)phenyl)amino)-7H-pyrrolo[2,3-d]pyrimidin-4-yl)amino)piperidin-1-yl)prop-2-en-1-one,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1 -yl)prop-2-en-1 -one,

(S)-(4-(1-(cyclopropylsulfonyl)piperidin-3-yl)amino)-2-((3-fluoro-4-(4-methylpiperazin-1-yl) phenyl)amino)-7H-pyrrolo[2,3-d]pyrimidin-5-yl)(4-fluorophenyl)methanone,
(S)-(4-(1-(cyclopropylsulfonyl)piperidin-3-yl)amino)-2-((4-(4-ethylpiperazin-1-yl)phenyl)ami no)-7H-pyrrolo[2,3-d]pyrimidin-5-yl)(4-fluorophenyl)methanone,
(S)-(4-(1-(cyclopropylsulfonyl)pyrrolidin-3-yl)amino)-2-((4-morpholinophenyl)amino)-7H-p yrrolo[2,3-d]pyrimidin-5-yl)(4-fluorophenyl)methanone,
(S)-(4-(1-(cyclopropylsulfonyl)pyrrolidin-3-yl)amino)-2-((4-(4-methylpiperazin-1-phenyl)ami no)-7H-pyrrolo[2,3-d]pyrimidin-5-yl)(phenyl)methanone,
(S)-1-(3-((5-benzoyl-2-((4-(4-methylpiperazin-1-yl)phenyl)amino)-7H-pyrrolo[2,3-d]pyrimidi n-4-yl)amino)pyrrolidin-1-yl)prop-2-en-1-one,
(S)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(3-fluorophenyl)methanone,
(S)-1-(3-((5-(3-fluorobenzoyl)-2-((4-(4-methylpiperazin-1-yl)phenyl)amino)-7H-pyrrolo[2,3-d]pyrimidin-4-yl)amino)pyrrolidin-1 -yl)prop-2-en-1 -one,
(S)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(3,4-difluorophenyl)methanone,
(S)-1-(3-((5-(3,4-difluorobenzoyl)-2-((4-(4-methylpiperazin-1-yl)phenyl)amino)-7H-pyrrolo[ 2,3 -d]pyrimidin-4-yl)amino)pyrrolidin-1 -yl)prop-2-en-1 -one,
(4-fluorophenyl)(2-((4-(4-methylpiperazin-1-yl)phenyl)amino)-4-(morpholinoamino)-7H-pyrr olo[2,3-d]pyrimidin-5-yl)methanone,
(4-fluorophenyl)(2-((4-(4-methylpiperazin-1 -yl)phenyl)amino)-4-(piperidin-1 -ylamino)-7H-p yrrolo[2,3-d]pyrimidin-5-yl)methanone,
(S)-1-(3-((5-(2,4-difluorobenzoyl)-2-((4-(4-methylpiperazin-1-yl)phenyl)amino)-7H-pyrrolo[ 2,3 -d]pyrimidin-4-yl)amino)pyrrolidin-1 -yl)prop-2-en-1 -one,
(S)-(4-((1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(2-fluorophenyl)methanone,
(S)-1-(3-((5-(2-fluorobenzoyl)-2-((4-(4-methylpiperazin-1-yl)phenyl)amino)-7H-pyrrolo[2,3-d]pyrimidin-4-yl)amino)pyrrolidin-1 -yl)prop-2-en-1 -one,
(R)-1-(3-((5-(4-fluorobenzoyl)-2-((1-methyl-1H-pyrazol-4-yl)amino)-7H-pyrrolo[2,3-d]pyrim idin-4-yl)amino)pyrrolidin-1-yl)prop-2-en-1-one,
(4-((2-(dimethylphosphoryl)phenyl)amino)-2-((4-(4-methylpiperazin-1-yl)phenyl)amino)-7H-pyrrolo[2,3-d]pyrimidin-5-yl)(4-fluorophenyl)methanone,
(S)-(4-((1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(2,4-difluorophenyl)methanone,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1-yl)-3,3-dimethylbut-1-one,
(S)-(3-((5-(4-fluorobenzoyl)-2-(4-(4-methylpiperazin-1-yl)phenyl)amino)-7H-pyrrolo[2,3-d]p yrimidin-4-yl)amino)pyrrolidin-1-yl)(4-fluorophenyl)methanone,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1-yl)-2,2-dimethylprop-1-one,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1 -yl)ethyl-1 -one,
(4-fluorophenyl)(4-(methyl(tetrahydro-2H-pyran-4-yl)amino)-2-(4-((4-methylpiperazin-1-yl)p henyl)amino)-7H-pyrrolo[2,3-d]pyrimidin-5-yl)methanone,
1-(4-((5-(4-fluorobenzoyl)-2-((4-(4-methylpiperazin-1-yl)phenyl)amino)-7H-pyrrolo[2,3-d]py rimidin-4-yl)amino)piperidin-1 -yl)prop-2-en-1 -one,
1-(4-((5-(4-fluorobenzoyl)-2-((4-(4-methylpiperazin-1-yl)phenyl)amino)-7H-pyrrolo[2,3-d]py rimidin-4-yl)amino)piperidin-1-yl) -2, 2-dimethylprop-1-one,
1-(4-((5-(4-fluorobenzoyl)-2-((4-(4-methylpiperazin-1-yl)phenyl)amino)-7H-pyrrolo[2,3-d]py rimidin-4-yl)amino)piperidin-1-yl)-3,3-dimethylbut-1-one,
(4-fluorophenyl)(2-((4-(4-methylpiperazin-1-yl)phenyl)amino)-4-(oxetan-3-ylamino)-7H-pyrr olo[2,3-d]pyrimidin-5-yl)methanone,
(4-fluorophenyl)(4-((3-methyloxetan-3-yl)amino)-2-((4-(4-methylpiperazin-1-yl)phenyl)amin o)-7H-pyrrolo[2,3-d]pyrimidin-5-yl)methanone,
(S)-(4-fluorophenyl)(4-((1-hydroxy-4-methylpentan-2-yl)amino)-2-((4-(4-methylpiperazin-1-yl)phenyl)amino)-7H-pyrrolo[2,3-d]pyrimidin-5-yl)methanone,
(R)-(4-fluorophenyl)(4-((1-hydroxy-4-methylpentan-2-yl)amino)-2-((4-(4-methylpiperazin-1-yl)phenyl)amino)-7H-pyrrolo[2,3-d]pyrimidin-5-yl)methanone,

(R)-(4-fluorophenyl)(4-(1-hydroxy-3,3-dimethylbut-2-yl)amino)-2-((4-(4-methylpiperazin-1-yl)phenyl)amino)-7H-pyrrolo[2,3-d]pyrimidin-5-yl)methanone,
(S)-(4-fluorophenyl)(4-((1-hydroxy-3,3-dimethylbut-2-yl)amino)-2-((4-(4-methylpiperazin-1-yl)phenyl)amino)-7H-pyrrolo[2,3-d]pyrimidin-5-yl)methanone,
(4-fluorophenyl)(2-((4-(4-methylpiperazin-1-yl)phenyl)amino)-7H-pyrrolo[2,3-d]pyrimidin-5 -yl)methanone,
(4-fluorophenyl)(2-((4-(4-methylpiperazin-1-yl)phenyl)amino)-4-((tetrahydro-2H-pyran-3-yl) amino)-7H-pyrrolo[2,3-d]pyrimidin-5-yl)methanone,
(4-fluorophenyl)(2-((4-(4-methylpiperazin-1-yl)phenyl)amino)-4-((2-morpholinoethyl)amino) -7H-pyrrolo[2,3-d]pyrimidin-5-yl)methanone,
(4-((2-(diethylamino)ethyl)amino)-2-((4-(4-methylpiperazin-1-yl)phenyl)amino)-7H-pyrrolo[ 2,3-d]pyrimidin-5-yl)(4-fluorophenyl)methanone,
(4-fluorophenyl)(2-((4-(4-methylpiperazin-1-yl)phenyl)amino)-4-((2-(pyrrolidin-1-yl)ethyl)a mino)-7H-pyrrolo[2,3-d]pyrimidin-5-yl)methanone,
(4-fluorophenyl)(2-((4-(4-methylpiperazin-1 -yl)phenyl)amino)-4-((2-(piperidin-1 -yl)ethyl)am ino)-7H-pyrrolo[2,3-d]pyrimidin-5-yl)methanone,
(4-fluorophenyl)(4-((4-hydroxytetrahydro-2H-pyran-4-yl)methyl)amino)-2-((4-(4-methylpipe razin-1-yl)phenyl)amino)-7H-pyrrolo[2,3-d]pyrimidin-5-yl)methanone,
(4-fluorophenyl)(4-((3-(hydroxymethyl)phenyl)amino)-2-((4-(4-methylpiperazin-1-yl)phenyl) amino)-7H-pyrrolo[2,3-d]pyrimidin-5-yl)methanone,
(4-fluorophenyl)(4-((6-(hydroxymethyl)pyridin-3-yl)amino)-2-((4-(4-methylpiperazin-1-yl)ph enyl)amino)-7H-pyrrolo[2,3-d]pyrimidin-5-yl)methanone,
(4-(((1r,4r)-4-aminocyclohexyl)amino)-2-(4-(4-methylpiperazin-1-yl)phenyl)amino)-7H-pyrr olo[2,3-d]pyrimidin-5-yl)(4-fluorophenyl)methanone,
(4-fluorophenyl)(4-(((1r,4r)-4-(hydroxymethyl)cyclohexyl)amino)-2-((1-(1-methylpiperidin-4 -yl)-1H-pyrazol-4-yl)amino)-7H-pyrrolo[2,3-d]pyrimidin-5-yl)methanone,
(4-((2-((4-(4-methylpiperazin-1-yl)phenyl)amino)-7H-pyrrolo[2,3-d]pyrimidin-4-yl)amino)pi peri din-1 -yl)methanol,
(4-fluorophenyl)(4-(((1*r*,4r)-4-(hydroxymethyl)cyclohexyl)amino)-2-((3-methoxy-1-(1-methy lpiperidin-4-yl)-1H-pyrazol-4-yl)amino)-7H-pyrrolo[2,3-d]pyrimidin-5-yl)methanone, and
((1*r*,4*r*)-4-((2-((4-morpholinophenyl)amino)thieno[2,3-d]pyrimidin-4-yl)amino)cyclohexyl)m ethanol.

The present invention provides a method for preparing a compound of formula I, including an isomer thereof, a hydrate thereof, a solvate thereof, a pharmaceutically acceptable salt thereof, and a prodrug thereof, comprising the following steps:

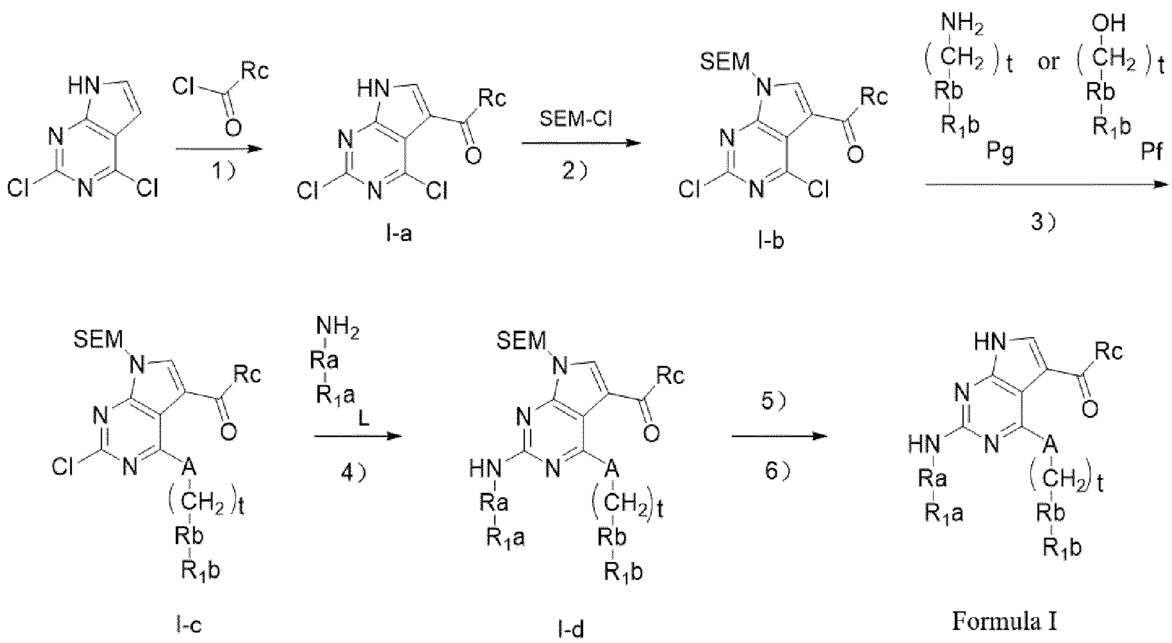

I-a
I-b
I-c
I-d
Formula I

1) reacting 2,4-dichloro-7H-pyrrolo[2,3-d]pyrimidine with

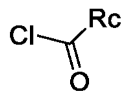

to obtain a compound of formula I-a, preferably, performing the reaction in an organic solvent, which is a polar solvent, preferably, dichloromethane or acetone;

2) reacting the compound I-a with 2-(trimethylsilyl)ethoxymethyl chloride (SEM-Cl) under the heating condition to obtain the compound I-b, preferably, at 30-60°C;

performing the reaction in an organic solvent: at least one of N,N-dimethylformamide (DMF), N,N-dimethylacetamide (DMA), N,N-diisopropylethylamine (DIPEA), N-methyl-2-pyrrolidone (NMP), ethylene glycol dimethyl ether, isopropanol, n-butanol, sec-butanol, and tert-butanol; and

performing the reaction under the alkaline condition, wherein the alkali is an inorganic alkali or an organic alkali, the inorganic alkali is selected from ammonia, potassium carbonate, sodium carbonate, and cesium carbonate, and the organic alkali is selected from at least one of sodium acetate, triethylamine, diisopropylethylamine, triethylene diamine, pyridine, 4-dimethylamino pyridine, 1,8-diazabicyclo undec-7-ene, and N-methylmorpholine;

3) reacting the compound I-b with the amine Pg and the alcohol Pf under the heating condition to obtain the compound I-c,

preferably, at 80-100°C; and

performing the reaction in an organic solvent and under the alkaline condition, wherein the alkali and the organic solvent are independently selected from the relevant limitations in step 2);

4) reacting the compound I-c with the amine L under the heating condition to obtain the compound I-d, preferably, at 100-130°C;

performing the reaction in an organic solvent and under the alkaline condition, wherein the alkali and the organic solvent are independently selected from the relevant limitations in step 2,

wherein the reaction is Buchwald-hartwig coupling using a palladium catalyst and an organic phosphine ligand, the used palladium catalyst is selected from palladium acetate, palladium dichloride, [1,1'-bis(diphenylphosphino)ferrocene]palladium dichloride, tris(dibenzylideneacetone)dipalladium, tetratriphenylphosphine palladium, and bistriphenylphosphine palladium dichloride, and the used organic phosphine ligand includes but is not limited to 2-dicyclohexylphosphine-2',4',6'-triisopropylbiphenyl, 1,1'-binaphthyl-2,2'-bisdiphenylphosphine, and 2-dicyclohexylphosphine-2 ', 6' -diisopropoxy-1,1' -biphenyl;

5) removing trimethylsilyl ethoxymethyl (an SEM group) from the compound I-d under the acid condition, wherein the acid is an inorganic acid or an organic acid, the inorganic acid is selected from at least one of hydrochloric acid, hydrobromic acid, hydrofluoric acid, hydroiodic acid, nitric acid, nitrous acid, and metaphosphoric acid, and the organic acid is selected from at least one of acetic acid, trichloroacetic acid, trifluoroacetic acid, glycolic acid, sulfamic acid, p-toluenesulfonic acid, ethylenediaminetetraacetic acid (EDTA), oxalic acid, and citric acid; and

6) neutralizing the product obtained in step 5 under the alkali condition to obtain the compound of formula I,

wherein the alkali is an inorganic alkali or an organic alkali, the inorganic alkali is selected from ammonia, potassium carbonate, sodium carbonate, and cesium carbonate, and the organic alkali is selected from at least one of sodium acetate, triethylamine, diisopropylethylamine, triethylene diamine, pyridine, 4-dimethylamino pyridine, 1,8-diazabicyclo undec-7-ene, and N-methylmorpholine; and

the Rc, the Ra, the Rb, the $R_1a$, and the $R_1b$ are as defined above, and the E is a protecting group selected from the SEM group, benzyloxycarbonyl, p-toluenesulfonyl, benzenesulfonyl, acetyl, trifluoroacetyl, fluorenylmethoxycarbonyl, and benzyl.

The present invention provides a method for preparing a compound of formula II, including an isomer thereof, a hydrate thereof, a solvate thereof, a pharmaceutically acceptable salt thereof, and a prodrug thereof, comprising the following steps:

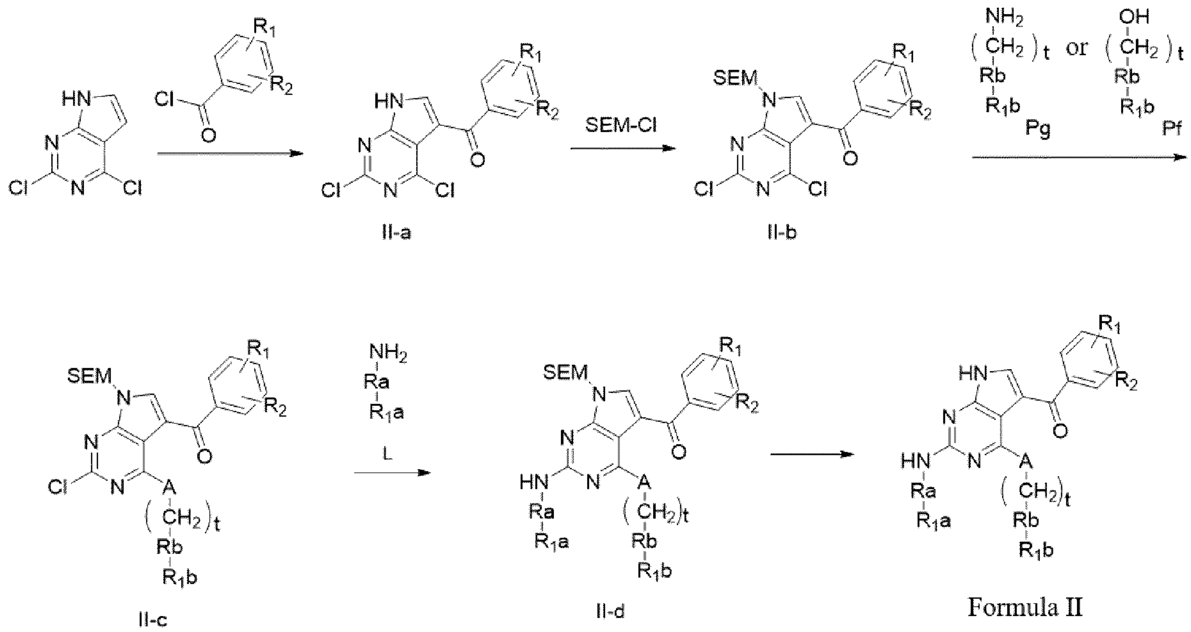


II-a

II-b

II-c

II-d

Formula II

reacting 2,4-dichloro-7H-pyrrolo[2,3-d]pyrimidine as a starting material with

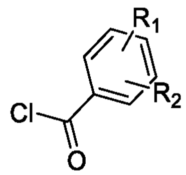

to obtain the compound II-a, wherein the substances in the reaction formula are used in the next steps, the reaction conditions are the same as those in the corresponding steps of formula I to prepare formula II; and the definition of each substituent in the reaction formula is the same as that of each substituent in a synthetic route in the general formula I or the same as that in formula II.

The present invention provides a method for preparing a compound of formula III, including an isomer thereof, a hydrate thereof, a solvate thereof, a pharmaceutically acceptable salt thereof, and a prodrug thereof, comprising the following steps:

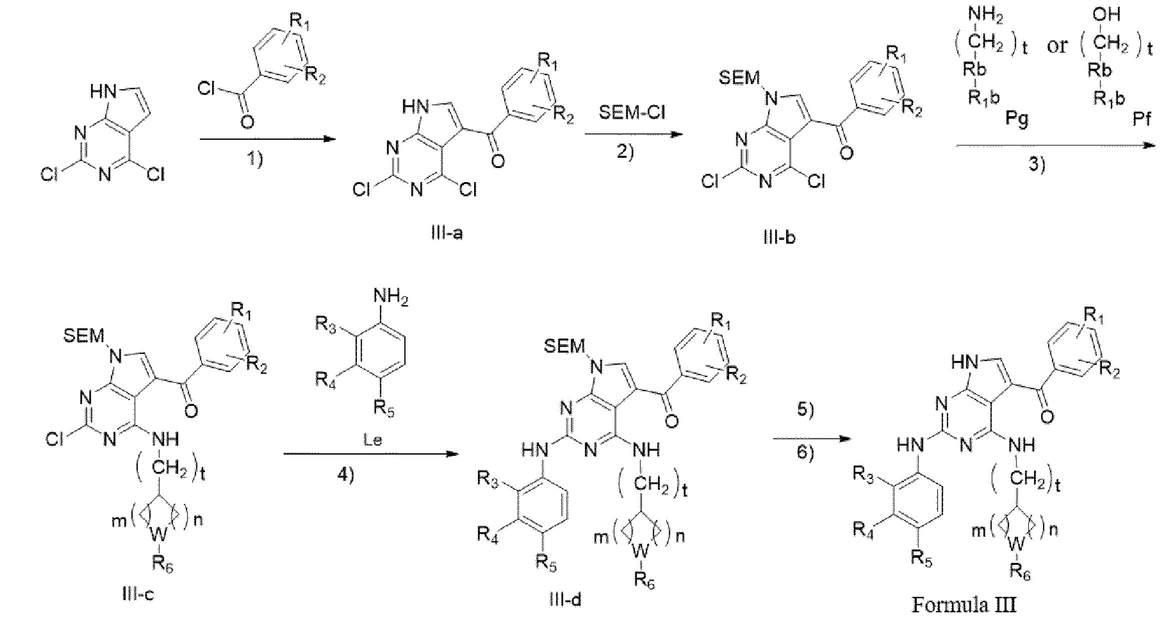


III-a

III-b

III-c

III-d

Formula III

1) reacting 2,4-dichloro-7H-pyrrolo[2,3-d] pyrimidine as a starting material with

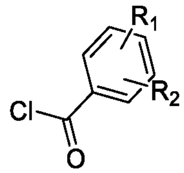

to obtain the compound III-a;

the substances in the reaction formula are used in the following steps 2) and 3), and the reaction conditions are the same as those in the corresponding steps of formula I to prepare formula III-c; and the definition of each substituent in the reaction formula is the same as that of each substituent in a synthetic route in the general formula I or the same as that in formula II; and

4) reacting the compound III-c reacts with

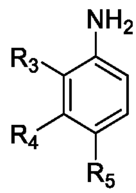

under the heating condition to obtain the compound III-d,

preferably, at 100-130°C;

performing the reaction in an organic solvent and under the alkaline condition, wherein the alkali and the organic solvent are independently selected from the relevant limitations in the synthetic step 2) of the compound of formula I, wherein the reaction is Buchwald-hartwig coupling using a palladium catalyst and an organic phosphine ligand, and the palladium catalyst and the organic phosphine ligand are independently selected from relevant limitations in the synthetic step 2) of the compound of formula I; and

in steps 5) and 6), the same preparation method of the corresponding steps of formula I is used to prepare formula III.

The present invention provides a method for preparing a compound of formula IV, including an isomer thereof, a hydrate thereof, a solvate thereof, a pharmaceutically acceptable salt thereof, and a prodrug thereof, comprising the following steps:

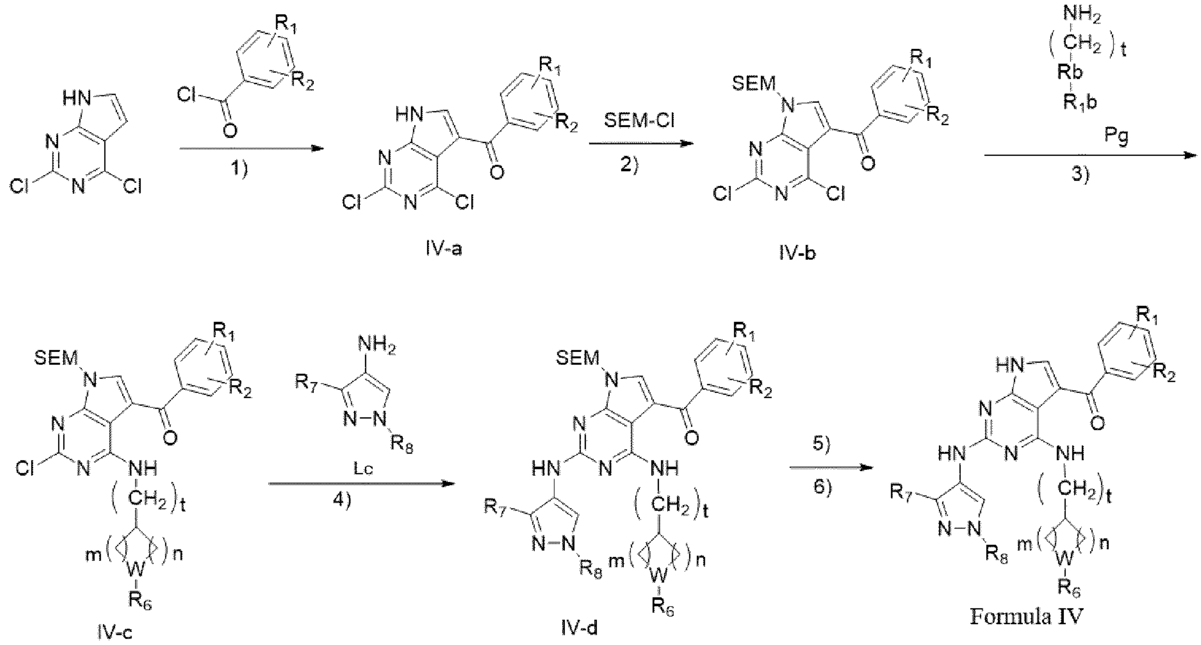

1) reacting 2,4-dichloro-7H-pyrrolo[2,3-d]pyrimidine with

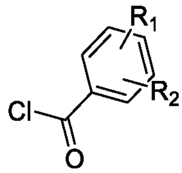

to obtain the compound IV-a,

wherein the substances in the reaction formula are used in the next steps 2) and 3), and the reaction conditions are the same as those in the corresponding steps of formula I to prepare formula IV-c; and the definition of each substituent in the reaction formula is the same as that of each substituent in a synthetic route in the general formula I or the same as that in formula II; and

4) reacting the compound IV-c with

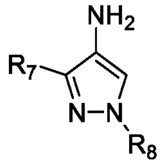

under the heating condition to obtain the compound IV-d,

preferably, at 100-130°C;

performing the reaction in an organic solvent and under the alkaline condition, wherein the alkali and the organic solvent are independently selected from the relevant limitations in the synthetic step 2) of the compound of formula I, wherein the reaction is Buchwald-hartwig coupling using a palladium catalyst and an organic phosphine ligand, and the palladium catalyst and the organic phosphine ligand are independently selected from relevant limitations in the synthetic step 2) of the compound of formula I; and

in steps 5) and 6), the same preparation method of the corresponding steps of formula I is used to prepare formula IV

The present invention further aims to provide a pharmaceutical composition containing an effective dose of the compound, the stereoisomer, or the pharmaceutically acceptable salt thereof of the present invention and one or more pharmaceutically acceptable pharmaceutical excipients. The compound of the present invention can be prepared independently or together with one or more medicinal carriers into different formulations such as a tablet, a pill, a powder, a capsule, a granule, a syrup, an emulsion, a microemulsion, or an injection. Specifically, the compound can be intravenously infused, subcutaneously infused, intramuscularly infused, intraperitoneally infused, transdermally infused, and directly infused into tissues for clinical oral administration, injection, or topical administration.

When the pharmaceutical composition of the present invention is prepared in the form of an oral formulation, an ingredient known in the art may be used as a pharmaceutically acceptable carrier without limitation so long as it does not interfere with the active expression of an active ingredient. The carrier may include, for example, an excipient, a diluent, a disintegrant, a binder, a glidant, a surfactant, an emulsifier, a suspending agent, a diluent, and the like, but is not limited thereto.

When the pharmaceutical composition of the present invention is prepared in the form of an injection, an ingredient known in the art may be used as a pharmaceutically acceptable carrier without limitation so long as it does not interfere with the active expression of an active ingredient. Specifically, the carrier may include, for example, water, saline, a dextrose aqueous solution, a pseudo-sugar aqueous solution, an alcohol, a glycol, an ether (e.g., polyethylene glycol 400), an oil, a fatty acid, a fatty acid ester, a glycerol ester, a surfactant, a suspending agent, an emulsifier, and the like, but is not limited thereto.

In these different formulations, the content of the compound of the present invention may be 0.1%-99.9%. The dose of the compound of the present invention may be 0.001-10,000 mg/kg/0.3 day and may be appropriately adjusted according to clinical needs.

The "pharmaceutically acceptable salt" of the present invention refers to a pharmaceutically acceptable acid or alkali addition salt, a solvate thereof, or a hydrate thereof.

Another objective of the present invention is to provide use of the compound, the stereoisomer thereof, the pharmaceutically acceptable salt thereof, and the pharmaceutical composition thereof in the preparation of an antitumor drug. The tumor is selected from lung cancer, liver cancer, colon cancer, pancreatic cancer, breast cancer, prostate

cancer, brain cancer, ovarian cancer, cervical cancer, testicular cancer, kidney cancer, head and neck cancer, lymphoma, melanoma, or leukemia. Further, the tumor is selected from lung cancer, liver cancer, or breast cancer, preferably, lung cancer. A series of tumor cell test results show that the compound of the present invention has broad-spectrum antitumor activity, the IC50 value of the compound is in nanomolar to micromolar level, the activity of the compound is equivalent to that of positive control drugs Brigatinib, Avitinib, and Osimetinib, and the activity of some compounds is superior to that of the positive drugs.

Another objective of the present invention is to provide use of the compound, the stereoisomer thereof, a prodrug thereof, a metabolite thereof, the pharmaceutically acceptable salt thereof, and the pharmaceutical composition thereof in the preparation of an antitumor drug. The tumor is selected from lung cancer, liver cancer, ascitic tumor, metastatic encephaloma, colon cancer, pancreatic cancer, breast cancer, prostate cancer, brain cancer, ovarian cancer, cervical cancer, testicular cancer, kidney cancer, head and neck cancer, lymphoma, melanoma, or leukemia, and preferably selected from lung cancer, liver cancer, breast cancer, ascitic tumor, pancreatic cancer, or metastatic encephaloma, wherein the lung cancer is selected from non-small cell lung cancer or small cell lung cancer, and more preferably selected from non-small cell lung cancer, liver cancer, breast cancer, and ascitic tumor, and the non-small cell lung cancer is lung adenocarcinoma. In a specific embodiment of the present invention, the lung cancer is selected from EGFR-mutant lung cancer, KRAS-mutant lung cancer, HER2-mutant lung cancer, PIK3CA-mutant lung cancer, BRAF-mutant lung cancer, *MET* gene-mutant lung cancer, *ALK* gene rearrangement lung cancer, *ROS1* gene rearrangement lung cancer, and *RET* gene rearrangement lung cancer. Further, in a specific embodiment of the present invention, the EGFR-mutant lung cancer is selected from EGFR-Del19-mutant lung cancer, EGFR-L858R-mutant lung cancer, EGFR-C797S-mutant lung cancer, EGFR-C797S/T790M-mutant lung cancer, EGFR-L858R/T790M-mutant lung cancer, EGFR-Del19/T790M-mutant lung cancer, EGFR-L858R/T790M/C797S-mutant lung cancer, EGFR-Del 19/T790MIC 797 S -mutant lung cancer, EGFR D770-N771 ins NPG-mutant lung cancer, or EGFR D770-N771 ins NPG/T790M-mutant lung cancer. In a specific embodiment of the present invention, the EGFR-mutant lung cancer is EGFR Del19/T790M/C797S-mutant lung cancer. In another specific embodiment of the present invention, the EGFR-mutant lung cancer is EGFR D770-N771 ins NPG-mutant lung cancer or EGFR D770-N771 ins NPG/T790M-mutant lung cancer.

Another objective of the present invention is to provide a method for treating a tumor, comprising administering the compound, the stereoisomer thereof, the pharmaceutically acceptable salt thereof, and the pharmaceutical composition thereof with the effective amount to a subject or a patient. The tumor is selected from lung cancer, liver cancer, ascitic tumor, metastatic encephaloma, colon cancer, pancreatic cancer, breast cancer, prostate cancer, brain cancer, ovarian cancer, cervical cancer, testicular cancer, kidney cancer, head and neck cancer, lymphoma, melanoma, or leukemia, preferably selected from lung cancer, liver cancer, breast cancer, ascitic tumor, pancreatic cancer, or metastatic encephaloma, and more preferably selected from non-small cell lung cancer, liver cancer, breast cancer, or ascitic tumor. A series of tumor cell tests show that: the present invention provides a novel-structured compound with an inhibitory activity of EGFR. Compared with the existing similar compounds, the compound of the present invention has a lower inhibition concentration on a tumor cell. The compound of the present invention has a significant inhibitory activity on EGFR D770-N771 ins NPG and EGFR D770-N771 ins NPG/T790M kinases, a very good inhibitory effect on an EGFR-Del19/T790M/C797S kinase, and has a very good inhibitory effect on a KC-0122:Ba/F3EGFR-L858R/T790M/C797S three-mutation cell line and a KC-0116:Ba/F3EGFR-Del19/T790M/C797S three-mutation cell line, and a weaker inhibitory effect on an EGFR wild-type cell line A549 with good selectivity. The compound provided by the present invention has an excellent inhibitory activity on mutant EGFR or EGFR-mutant cancer cells. The compound provided by the present invention has great industrialization and commercialization prospects and market value, and remarkable economic benefits. The compound provided by the present invention has great industrialization and commercialization prospects and market value, and remarkable economic benefits.

Although the preferred embodiments of the present invention have been shown and described herein, it would have been obvious to those skilled in the art that such embodiments are provided by way of examples only. At present, those skilled in the art will think of many variations, changes, and substitutions without departing from the present invention. It should be understood that various alternatives to the embodiments of the present invention described herein may be used in implementing the present invention. It is intended that the following claims define the scope of the present invention and cover the methods and structures, and equivalent forms thereof within the scope of these claims.

## Some chemical terms

Unless defined otherwise, all technical and scientific terms used herein have the same meaning as commonly understood by one of ordinary skills belonging to the art of the subject matter of the claims. All patents, patent applications, and publications cited herein are incorporated by reference in their entirety unless otherwise indicated.

It is to be understood that both the foregoing brief description and the following detailed description are exemplary and explanatory only and are not restrictive of the subject matter of the present application. In the present application, the use of the singular also includes the plural unless otherwise specifically indicated. It should also be noted that the

use of "or" means "and/or" unless otherwise indicated. Furthermore, the term "comprises" and other forms thereof, such as "includes", "including", and "contains" are intended to be non-limiting description.

Definitions for standardized chemical terms can be found in the reference (including Carey and Sundberg "ADVANCED ORGANIC CHEMISTRY 4TH ED", Vols. A(2000) and B(2001), Plenum Press, New York). Unless otherwise indicated, conventional methods within the technical range of the art, such as mass spectrometry, NMR, and pharmacological methods, are used. Unless specifically defined, the terms used herein in the related description of analytical chemistry, organic synthetic chemistry, and pharmaceuticals and pharmaceutical chemistry are known in the art. Standard techniques can be used in chemical synthesis, chemical analysis, pharmaceutical preparation, formulation and delivery, and treatment of patients. For example, the reaction and purification can be performed using instructions from the manufacturer for use of the kit, or in a manner known in the art, or using the description of the present application. The techniques and methods described above can generally be implemented according to conventional methods well known in the art, as described in various general and more specific references referred to and discussed in the description. In the present description, groups and substituents thereof may be selected by those skilled in the art to provide stable structural moieties and compounds.

When a substituent is described by a conventional chemical formula written from left to right, the substituent also includes a chemically equivalent substituent obtained when the formula is written from right to left.

For example, CH2O is equivalent to OCH2.

The term "compound" of the present application include all stereoisomers, geometric isomers, tautomers, and isotopes. The compound of the present application may be asymmetric, e.g., having one or more stereoisomers. Unless otherwise indicated, all the stereoisomers include, for example, enantiomers and diastereomers. The compound of the present application containing an asymmetrically substituted carbon atom can be isolated in the optically active pure form or in the racemic form. The optically active pure form can be resolved from a racemic mixture or synthesized by using a chiral raw material or a chiral reagent. The compound of the present application further includes the tautomeric form. The tautomeric form is derived from the exchange of one single bond with an adjacent double bond and the concomitant migration of one proton. The compound of the present application further includes all isotopic atoms, whether in an intermediate or a final compound. The isotopic atoms include atoms having the same number of atoms but different mass numbers. For example, the isotopes of hydrogen include tritium and deuterium. That is, the compound of the present application includes compounds in which some or all hydrogens (H) are substituted by tritium (T) and/or deuterium (D), and further includes compounds with some or all $^{12}C$ substituted by $^{13}C$ and/or $^{14}C$, and compounds with substitutions between other isotopes (e.g. N, O, P, and S), such as $^{14}N$ and $^{15}N$, $^{18}O$ and $^{17}O$, $^{31}P$ and $^{32}P$, $^{35}S$ and $^{36}S$, and the like. The compound described herein may have one or more stereoisomeric centers, and each stereoisomeric center may exist in the R or S configuration or a combination thereof. Similarly, the compound described herein may have one or more double bonds, and each double bond may be present in the E (trans) or Z (cis) configuration or a combination thereof. A particular stereoisomer, structural isomer, diastereoisomer, enantiomer or epimer is understood to encompass all possible isomers, such as a stereoisomer, a structural isomer, a diastereoisomer, an enantiomer, or an epimer, and a mixture thereof. Thus, the compound described herein includes all configurationally distinct stereoisomers, structural isomers, diastereomers, enantiomers, or epimers, and a corresponding mixture thereof. Techniques for converting a particular stereoisomer or keeping an original state of the particular stereoisomer, and resolving a mixture of the stereoisomer are well known in the art, and those skilled in the art will be able to select suitable methods for the particular situation.

The term "optional/random" or "optionally/randomly" means that the subsequently described event or circumstance may or may not occur. The description includes instances where the event or circumstance occurs and does not occur.

The $C_{1-3}$ or C1-3 used herein means that the moiety has 1-3 carbon atoms, i.e., the group contains 1 carbon atom, 2 carbon atoms, and 3 carbon atoms. Therefore, for example, "C1-3 alkyl" refers to alkyl having 1-3 carbon atoms, i.e., the alkyl is selected from methyl, ethyl, propyl, and isopropyl.

The term "alkyl" used herein, alone or in combination, refers to an optionally substituted straight-chain or optionally substituted branched-chain aliphatic hydrocarbon. The "alkyl" herein preferably may have from 1 to about 20 carbon atoms, for example, from 1 to about 10 carbon atoms, from 1 to about 8 carbon atoms, from 1 to about 6 carbon atoms, from 1 to about 4 carbon atoms, or from 1 to about 3 carbon atoms. Examples of the alkyl herein include, but are not limited to, methyl, ethyl, n-propyl, isopropyl, and the like.

The "alkyl" used in combination herein includes alkyl in combination with other groups, such as alkyl in alkoxy.

The term "halogen" used herein, alone or in combination, is selected from F, Cl, Br, and I.

As used herein, the term "treating" and other similar synonyms include alleviating, reducing, or ameliorating a symptom of a disease or disorder, and inhibiting a disease or disorder, e.g., arresting the development of a disease or disorder, ameliorating a disease or disorder, alleviating a symptom caused by a disease or disorder, or halting a symptom of a disease or disorder, preventing other symptoms, and relieving or preventing an underlying metabolic cause leading to the symptom. In addition, the term encompasses a prophylactic purpose. The term further comprises obtaining a

therapeutic effect and/or a prophylactic effect. The therapeutic effect refers to curing or relieving an underlying disease being treated. In addition, curing or relieving one or more physiological symptoms associated with an underlying disease is also a therapeutic effect, e.g., an improvement in a patient's condition is observed, although the patient may still be affected by the underlying disease. For the prophylactic effect, the composition may be administered to a patient at risk of developing a particular disease, or to a patient having one or more physiological symptoms of the disease, even if a diagnosis of the disease has not yet been made.

The term "effective amount", "therapeutically effective amount", or "pharmaceutically effective amount" used herein refers to the amount of at least one active substance (e.g., the compound of the present application) that is sufficient to alleviate one or more symptoms of the disease or disorder being treated to some extent after administration. The result may be a reduction and/or alleviation of signs, symptoms, or causes, or any other desired change in a biological system. For example, the "effective amount" for treatment is the amount of the composition containing the compound disclosed herein that is clinically necessary to provide an obvious effect for alleviating a disorder. The effective amount suitable in any individual case may be determined using techniques such as a dose escalation test.

The term "acceptable" used herein with respect to a formulation, a composition, or an ingredient means that there is no long-term deleterious effect on the general health of a subject being treated.

The term "pharmaceutically acceptable" used herein refers to a substance (e.g., a carrier or a diluent) that does not affect the biological activity or properties of the compound of the present application. Besides, the substance is relatively non-toxic, i.e., the substance can be administered to an individual without causing an undesirable biological response or interacting in an undesirable manner with any component contained in the composition.

The term "pharmaceutical composition" used herein refers to a mixture of the compound of the present application and at least one pharmaceutically acceptable substance. The pharmaceutically acceptable substance includes, but is not limited to, a carrier, a stabilizer, a diluent, a dispersing agent, a suspending agent, a thickening agent, and/or an excipient.

The term "carrier" used herein refers to a relatively non-toxic substance that facilitates the introduction of the compound of the present application into a cell or a tissue.

The term "pharmaceutically acceptable salt" used herein refers to a salt that retains the biological effectiveness of a free acid and a free alkali of the specified compound and has no adverse effects biologically or in other aspects. The compound of the present application further comprises a pharmaceutically acceptable salt. The pharmaceutically acceptable salt refers to the form in which an alkali group in a parent compound is converted to a salt. The pharmaceutically acceptable salt includes, but is not limited to, an inorganic acid or organic acid salt of the alkali group such as an amino group. The pharmaceutically acceptable salt of the present application may be synthesized from the parent compound by reacting the alkali group in the parent compound with 1-4 equivalents of an acid in a solvent system. Suitable salts are listed in Remingtong's Pharmaceutical Scicences, 17th ed., Mack Publishing Company, Easton, Pa., 1985, p. 1418 and Journal of Pharmaceutical Science, 66, 2(1977).

Unless otherwise indicated, the salt in the present application refer to an acid salt formed with an organic/inorganic acid, and an alkali salt formed with an organic/inorganic alkali.

The "EGFR" refers to the epidermal growth factor receptor.

For those skilled in the art, "EGFR mutation-negative" generally means that EGFR gene mutation is not detected according to gene detection methods usually for clinical diagnosis. An EGFR mutation status can be detected by various methods. A DNA mutation detection is the first method for detecting the EGFR status. Various DNA mutation detection analyses can be used for detecting the EGFR mutation status of tumor cells. The most common EGFR mutations of patients with non-small cell lung cancer are exon 19 deletion and exon 21 mutation. Direct DNA sequencing of exons 18-21 (or exons 19 and 21 only) is a reasonable choice.

The "advanced stage" means staging non-small cell lung cancer on the basis of the extent of the pathological changes and complications, e.g., stage III-IV non-small cell lung cancer according to the TNM classification method in the lung cancer staging system of the AJCC cancer staging manual. In some embodiments, advanced-stage non-small cell lung cancer is IIIB-IV non-small cell lung cancer.

As used herein, unless otherwise indicated, the doses and ranges provided herein are calculated on the basis of the molecular weight of the compound of the present application in its free alkali form.

**Brief Description of the Drawings**

Fig. 1 shows a tumor volume curve of mice in each group; and

Fig. 2 shows a tumor weight curve of mice in each group

## Detailed Description of Embodiments

In order to more clearly illustrate the present invention, a series of examples are listed herein. The following examples are illustrative and should not be construed as a limitation to the present invention.

The present invention provides methods for preparing the corresponding compounds. The compounds described herein can be prepared using various synthetic methods, including those referred to in the examples below. The compound, the pharmaceutically acceptable salt, the isomer, or the hydrate thereof of the present invention, may be synthesized by using the methods described below and the synthetic methods known in the art of organic chemical synthesis, or by variations of these methods as understood by those skilled in the art. The preferred method includes, but is not limited to, the methods described below.

Example 1

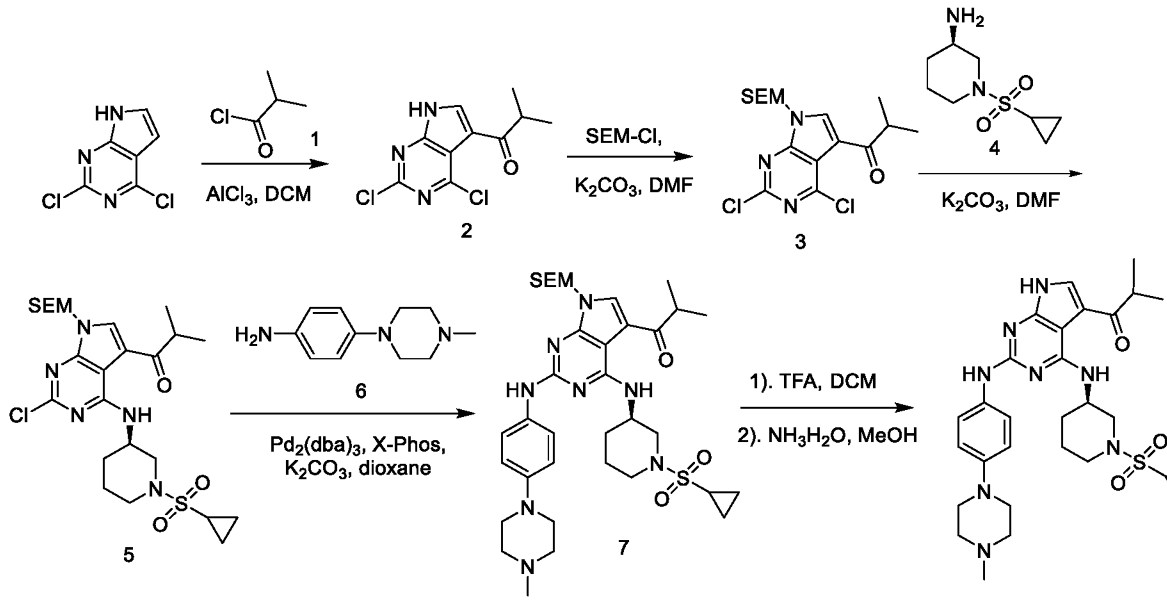


5.0 g of 2,4-dichloro-7H-pyrrolo[2,3-d] pyrimidine and 100 mL of dichloromethane were added into a 250-mL round-bottom flask, 14.15 g of aluminum trichloride was added under stirring in an ice-water bath, stirring was performed for 0.5 h, and 3.5 mL of isobutyryl chloride (compound 1) diluted with 50 mL of dichloromethane was added dropwise. After the addition, the mixture was heated and subjected to a reflux reaction for 12 h. An obtained system was poured into ice water and stirred for 1h. An obtained aqueous phase was extracted with dichloromethane and obtained organic phases were combined. The organic phase was washed with a saturated salt solution, dried over anhydrous sodium sulfate, and concentrated to dryness under reduced pressure. An obtained residue was subjected to silica gel column chromatography (petroleum ether: ethyl acetate = 10:1) to obtain 2,4-dichloro-5-(2-methylacetonyl)-7H-pyrrolo[2,3-d] pyrimidine (intermediate 2).

2 g of the intermediate 2, 30 mL of N,N-dimethylformamide, and 2.65 g of potassium carbonate were added to a 100-mL round bottom flask, 1.7 mL of 2-(trimethylsilyl)ethoxymethyl chloride diluted with 20 mL of N,N-dimethylformamide was added dropwise while the temperature of an oil bath was controlled at 40°C, and after the addition was completed, an obtained mixture was stirred for 4 h while the temperature was maintained at 40°C. An obtained system was poured into water, an obtained aqueous phase was extracted with ethyl acetate, and obtained organic phases were combined. The organic phase was washed with a saturated salt solution, dried over anhydrous sodium sulfate, and concentrated to dryness under reduced pressure. An obtained residue was subjected to silica gel column chromatography (petroleum ether: ethyl acetate = 15:1) to obtain 2,4-dichloro-5-(2-methylacetonyl)-7-(trimethylsilyl ethoxymethyl)-7H-pyrrolo[2,3-d]pyrimidine (intermediate 3).

200 mg of the intermediate 3, 10 mL of N,N-dimethylformamide, 186 mg of potassium carbonate, and (R)-1-cyclopropylsulfonyl-3-aminopiperidine (compound 4) were added into a 50-mL eggplant-shaped flask, and an obtained mixture was stirred in an oil bath at 90°C for 3 h. The N,N-dimethylformamide was evaporated under reduced pressure, 20 mL of water was added into an obtained residue, the residue was ultrasonically stirred for 10 min and subjected to

suction filtration, and a filter cake was dried to obtain (R)-2-chloro-4-(1-cyclopropylsulfonyl-3-piperidinamino)-5-(2-methylpropionyl)-7-(trimethyl silylethoxymethyl)-7H-pyrrolo[2,3-d]pyrimidine (intermediate 5).

150 mg of the intermediate 5, 45 mg of 4-(4-methyl-1-piperazin)-1-aniline (compound 6), 27 mg of $Pd_2(dba)_3$, 43 mg of X-Phos, 124 mg of potassium carbonate, and 10 mL of 1,4-dioxane were added into a 100-mL eggplant-shaped flask, and an obtained mixture was stirred in an oil bath at 110°C for 4 h under the nitrogen atmosphere. The mixture was concentrated to dryness under reduced pressure. An obtained residue was subjected to silica gel column chromatography (dichloromethane:methanol = 20:1) to obtain 2-(4-(4-methyl-1-piperazin)-1-phenylamino)-4-(tetrahydropyran-4-amino)-5-(2-methylpropan oyl)-7-(trimethylsilylethoxymethyl)-7H-pyrrolo[2,3-d]pyrimidine (intermediate 7).

110 mg of the intermediate 5, 5 mL of dichloromethane, and 2 mL of trifluoroacetic acid were added into a 100-mL eggplant-shaped flask, and an obtained mixture was stirred at room temperature for 2 h. The dichloromethane and the trifluoroacetic acid were evaporated under reduced pressure, 10 mL of methanol and 4 mL of ammonium hydroxide were added, and an obtained mixture was stirred at room temperature for 4 h. The methanol and the ammonium hydroxide were evaporated under reduced pressure. An obtained residue was subjected to silica gel column chromatography (dichloromethane:methanol = 20:1) to obtain (R)-2-(4-(4-methyl-1-piperazin)-1-phenylamino)-4-(1-cyclopropylsulfonyl-3-piperidinamino) -5-(2-methylpropanoyl)-7H-pyrrolo[2,3-d]pyrimidine. $^1H$ NMR (600 MHz, DMSO-$d_6$) $\delta$ 12.01 (s, 1H), 9.09 (d, $J$ = 7.8 Hz, 1H), 8.64 (s, 1H), 8.08 (s, 1H), 7.61 (d, $J$ = 9.0 Hz, 2H), 6.84 (d, $J$ = 9.1 Hz, 2H), 4.37-4.24 (m, 1H), 3.70-3.61 (m, 1H), 3.55-3.47 (m, 1H), 3.41-3.37 (m, 1H), 3.33-3.31 (m, 1H), 3.11-2.98 (m, 6H), 2.63-2.59 (m, 1H), 2.53-2.52 (m, 2H), 2.48-2.47 (m, 1H), 2.24 (s, 3H), 2.03-1.87 (m, 2H), 1.70-1.54 (m, 2H), 1.11 (d, $J$ = 6.8 Hz, 6H), 0.97-0.89 (m, 4H). MS/[M+H]+:581.36

Example 2

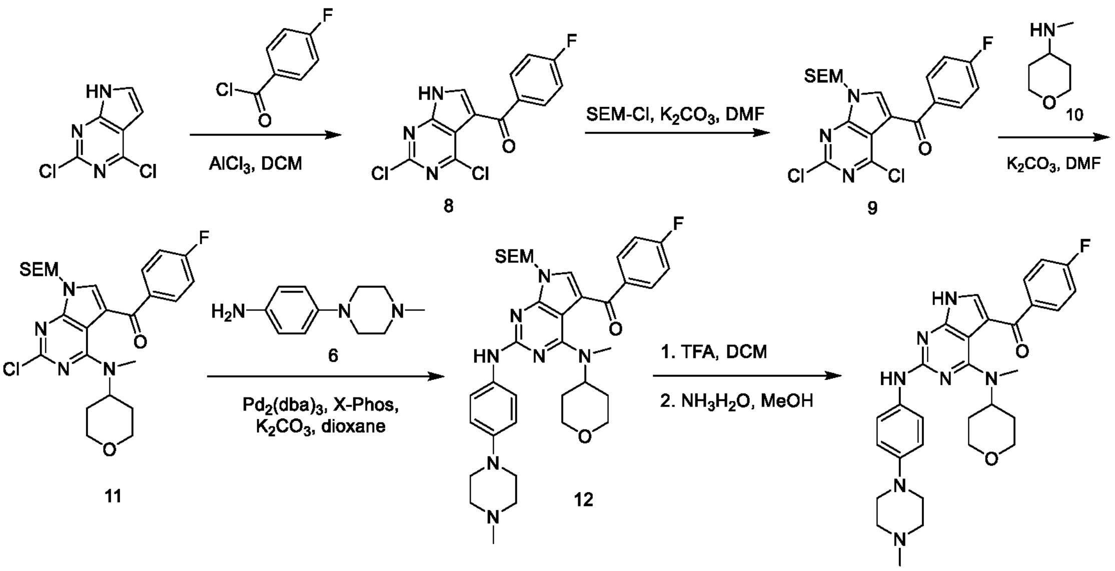


The preparation method used in example 2 was the same as that in example 1. $^1H$ NMR (600 MHz, DMSO-$d_6$) $\delta$ 11.88 (s, 1H), 8.69 (s, 1H), 7.96 (dd, $J$ = 8.5, 5.7 Hz, 2H), 7.62 (d, $J$ = 8.9 Hz, 2H), 7.37 (t, $J$ = 8.7 Hz, 2H), 7.32 (d, $J$ = 2.5 Hz, 1H), 6.86 (d, $J$ = 9.0 Hz, 2H), 4.41-4.27 (m, 1H), 3.93-3.78 (m, 2H), 3.21-3.11 (m, 2H), 3.10-3.00 (m, 4H), 2.79 (s, 3H), 2.49-2.45 (m, 4H), 2.23 (s, 3H), 1.76-1.63 (m, 2H), 1.56-1.43 (m, 2H). MS/$[M+H]^+$ :544.27

Example 3

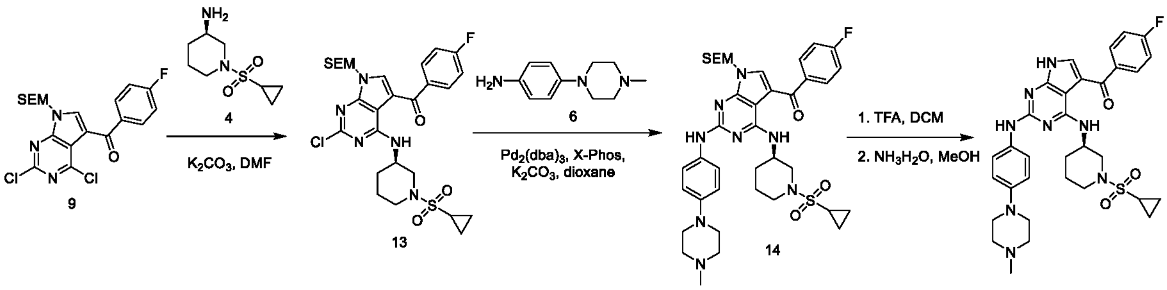


9 13 14

The preparation method used in example 3 was the same as that in example 1. $^1$H NMR (600 MHz, DMSO-$d_6$) δ 12.21 (s, 1H), 8.85 (d, *J* = 7.8 Hz, 1H), 8.74 (s, 1H), 7.84 (dd, *J* = 8.5, 5.6 Hz, 2H), 7.63 (d, *J* = 8.9 Hz, 2H), 7.48 (s, 1H), 7.36 (t, *J* = 8.8 Hz, 2H), 6.86 (d, *J* = 9.0 Hz, 2H), 4.47-4.27 (m, 1H), 3.70-3.56 (m, 1H), 3.33-3.31 (m, 1H), 3.17-3.07 (m, 2H), 3.07-3.00 (m, 4H), 2.62-2.58 (m, 1H), 2.55-2.52 (m, 4H), 2.26 (s, 3H), 2.04-1.88 (m, 2H), 1.73-1.58 (m, 2H), 0.99-0.88 (m, 4H). MS/[M+H]$^+$:633.31

**Example 4**

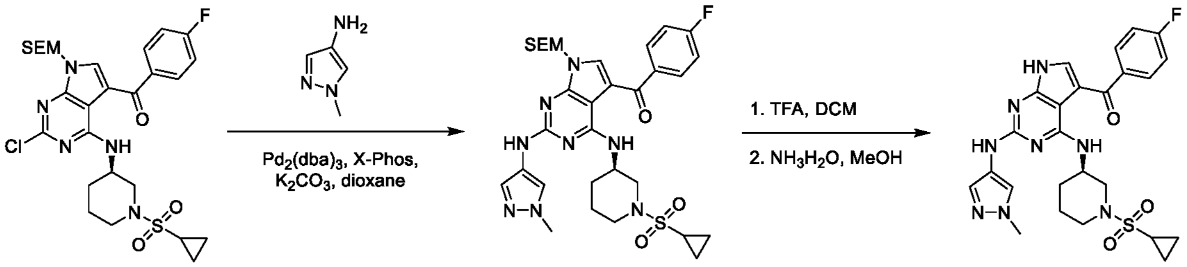


13 15

The preparation method used in example 4 was the same as that in example 1. $^1$H NMR (600 MHz, DMSO-$d_6$) δ 12.15 (s, 1H), 9.04-8.71 (m, 2H), 7.87 (s, 1H), 7.84 (dd, *J* = 8.1, 5.7 Hz, 2H), 7.54-7.39 (m, *J* = 2.3 Hz, 2H), 7.36 (t, *J* = 8.7 Hz, 2H), 4.42-4.20 (m, 1H), 3.78 (s, 3H), 3.47-3.38 (m, 1H), 3.34-3.29 (m, 1H), 3.13-3.04 (m, 1H), 3.03-2.94 (m, 1H), 2.63-2.59 (m, 1H), 2.09-1.88 (m, 2H), 1.74-1.58 (m, 2H), 0.99-0.87 (m, 4H).MS/[M+H]$^+$:539.24

The following compounds of examples 8-116 were synthesized with reference to the specific synthetic steps of example 1, guided by the synthetic route of general formula I.

| Exam ple | Structure | H-NMR | Mass spectrum/ actua l measurement [ M+H]$^+$ |
|---|---|---|---|
| 8 | 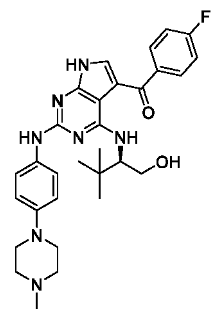 | $^1$H NMR (600 MHz, DMSO-$d_6$) δ 12.12 (s, 1H), 8.79 (d, *J* = 9.3 Hz, 1H), 8.62 (s, 1H), 7.84 (dd, *J* = 8.3, 5.7 Hz, 2H), 7.67 (d, *J* = 8.9 Hz, 2H), 7.43 (s, 1H), 7.35 (t, *J* = 8.7 Hz, 2H), 6.84 (d, *J* = 8.9 Hz, 2H), 4.58 (s, 1H), 4.33-4.22 (m, 1H), 3.80-3.70 (m, 1H), 3.58-3.49 (m, 1H), 3.15-2.97 (m, 4H), 2.55-2.52 (m, 4H), 2.26 (s, 3H), 104 (s, 9H). | 546.28 |

(continued)

| Example | Structure | H-NMR | Mass spectrum/ actua l measurement [ M+H]$^+$ |
|---|---|---|---|
| 9 | 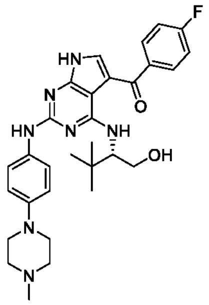 | $^1$H NMR (600 MHz, DMSO-$d_6$) δ 12.11 (s, 1H), 8.79 (d, *J* = 9.2 Hz, 1H), 8.62 (s, 1H), 7.91-7.78 (m, 2H), 7.67 (d, *J* = 8.5 Hz, 2H), 7.43 (s, 1H), 7.35 (t, *J* = 8.4 Hz, 2H), 6.84 (d, *J* = 8.5 Hz, 2H), 4.58 (s, 1H), 4.33-4.22 (m, 1H), 3.80-3.69 (m, 1H), 3.59-3.49 (m, 1H), 3.14-2.99 (m, 4H), 2.54-2.52 (m, 4H), 2.26 (s, 3H), 1.04 (s, 9H). | 546.27 |
| 10 | 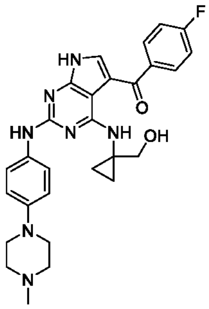 | $^1$H NMR (600 MHz, DMSO-$d_6$) δ 12.13 (s, 1H), 9.00 (s, 1H), 8.72 (s, 1H), 7.82 (dd, *J* = 8.5, 5.6 Hz, 2H), 7.77 (d, *J* = 8.9 Hz, 2H), 7.46 (s, 1H), 7.36 (t, *J* = 8.8 Hz, 2H), 6.86 (d, *J* = 9.0 Hz, 2H), 4.97-4.66 (m, 1H), 3.75-3.60 (m, 2H), 3.14-3.00 (m, 4H), 2.59-2.53 (m, 4H), 2.29 (s, 3H), 0.97-0.89 (m, 2H), 0.82-0.76 (m, 2H). | 516.22 |
| 11 | 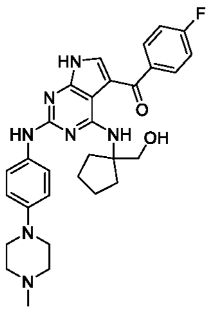 | $^1$H NMR (600 MHz, DMSO-$d_6$) δ 12.10 (s, 1H), 8.82 (s, 1H), 8.53 (s, 1H), 7.82 (dd, *J* = 8.6, 5.6 Hz, 2H), 7.61 (d, *J* = 9.0 Hz, 2H), 7.40 (s, 1H), 7.35 (t, *J* = 8.8 Hz, 2H), 6.85 (d, *J* = 9.0 Hz, 2H), 4.83 (t, *J* = 5.2 Hz, 1H), 3.82 (d, *J* = 5.2 Hz, 2H), 3.13-2.98 (m, 4H), 2.54-2.51 (m, 4H), 2.25 (s, 3H), 2.13-2.04 (m, 2H), 1.94-1.86 (m, 2H), 1.76-1.59 (m, 4H). | 544.26 |
| 12 | 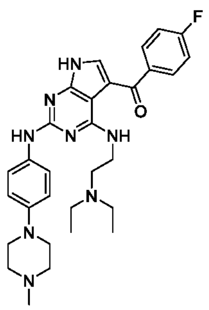 | $^1$H NMR (600 MHz, DMSO-$d_6$) δ 12.12 (s, 1H), 8.69 (t, *J* = 5.4 Hz, 1H), 8.63 (s, 1H), 7.86-7.79 (m, 2H), 7.64 (d, *J* = 9.0 Hz, 2H), 7.43 (s, 1H), 7.35 (t, *J* = 8.8 Hz, 2H), 6.85 (d, *J* = 9.1 Hz, 2H), 3.70-3.61 (m, 2H), 3.08-3.02 (m, 4H), 2.84-2.71 (m, 2H), 2.71-2.57 (m, 4H), 2.55-2.52 (m, 4H), 2.24 (s, 3H), 1.01 (t, *J* = 7.0 Hz, 6H). | 545.31 |

(continued)

| Example | Structure | H-NMR | Mass spectrum/ actua l measurement [ M+H]$^+$ |
|---|---|---|---|
| 13 | 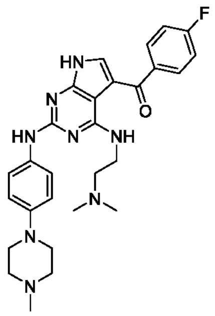 | ND | 517.28 |
| 14 | 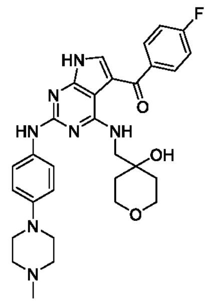 | $^1$H NMR (600 MHz, DMSO-$d_6$) δ 12.13 (s, 1H), 8.84 (t, *J* = 5.5 Hz, 1H), 8.73 (s, 1H), 7.86-7.82 (m, 2H), 7.76-7.69 (m, 2H), 7.45 (d, *J* = 2.6 Hz, 1H), 7.37-7.34 (m, 2H), 6.92 (d, *J* = 9.1 Hz, 2H), 4.73 (s, 1H), 3.70-3.49 (m, 10H), 2.81 (s, 3H), 2.52-2.52 (m, 4H), 1.65-1.61 (m, 2H), 1.54-1.50 (m, 2H). | 560.27 |
| 15 | 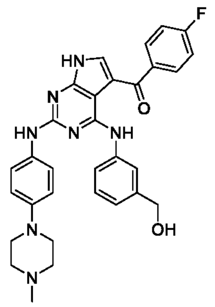 | $^1$H NMR (600 MHz, DMSO-$d_6$) δ 12.43 (s, 1H), 11.23 (s, 1H), 9.74 (s, 1H), 8.99 (s, 1H), 8.01 (d, *J* = 5.4 Hz, 1H), 7.95-7.86 (m, 2H), 7.68 (d, *J* = 8.7 Hz, 2H), 7.66-7.60 (m, 2H), 7.39 (t, *J* = 8.8 Hz, 2H), 7.31 (t, *J* = 7.8 Hz, 1H), 7.01 (d, *J* = 7.5 Hz, 1H), 6.97 (d, *J* = 9.0 Hz, 2H), 5.39-5.01 (m, 1H), 4.52 (s, 2H), 3.80-3.47 (m, 4H), 3.24-3.15 (m, 2H), 3.00-2.83 (m, 5H). | 552.25 |
| 16 | 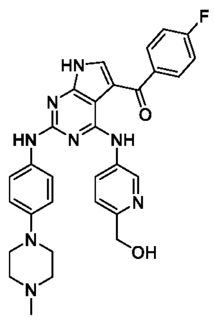 | $^1$H NMR (600 MHz, DMSO-$d_6$) δ 12.49 (s, 1H), 11.24 (s, 1H), 9.03 (s, 1H), 8.91 (s, 1H), 8.47 (dd, *J* = 8.5, 2.3 Hz, 1H), 7.93-7.89 (m, 2H), 7.65 (s, 1H), 7.62 (d, *J* = 8.5 Hz, 2H), 7.44 (d, *J* = 8.5 Hz, 1H), 7.41-7.37 (m, 2H), 6.89 (d, *J* = 9.1 Hz, 2H), 5.35 (t, *J* = 5.6 Hz, 1H), 4.56 (d, *J* = 5.2 Hz, 2H), 3.13-3.05 (m, 4H), 2.52-2.52 (m, 4H), 2.27 (s, 3H). | 553.24 |

(continued)

| Exam ple | Structure | H-NMR | Mass spectrum/ actua l measurement [ M+H]+ |
|---|---|---|---|
| 17 | 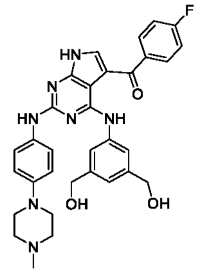 | ND | 582.26 |
| 18 | 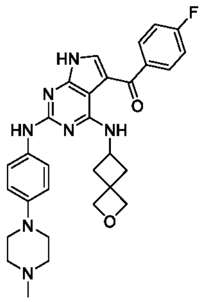 | $^1$H NMR (600 MHz, DMSO-$d_6$) δ 12.12 (s, 1H), 8.82 (d, *J* = 7.2 Hz, 1H), 8.68 (s, 1H), 7.91-7.80 (m, 2H), 7.70 (d, *J* = 9.0 Hz, 2H), 7.43 (d, *J* = 2.8 Hz, 1H), 7.38-7.31 (m, 2H), 6.87 (d, *J* = 9.1 Hz, 2H), 4.68-4.53 (m, 3H), 3.52-3.48 (m, 2H), 3.23-3.00 (m, 4H), 2.74 (s, 3H), 2.52-2.52 (m, 4H), 2.28-2.21 (m, 2H), 1.87-1.78 (m, 2H). | 542.26 |
| 19 | 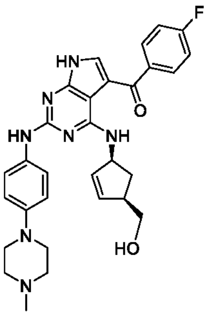 | $^1$H NMR (600 MHz, DMSO-$d_6$) δ 12.17 (s, 1H), 8.77 (s, 1H), 8.72 (d, *J* = 7.7 Hz, 1H), 7.86-7.81 (m, 2H), 7.77-7.70 (m, 2H), 7.45 (s, 1H), 7.35 (t, *J* = 8.8 Hz, 2H), 6.91 (d, *J* = 9.1 Hz, 2H), 6.03-5.97 (m, 1H), 5.97-5.89 (m, 1H), 5.30-5.23 (m, 1H), 4.72-4.68 (m, 2H), 3.47-3.38 (m, 5H), 3.25-3.18 (m, 4H), 2.78 (s, 3H), 2.57-2.51 (m, 2H), 1.49-1.37 (m, 1H). | 542.26 |
| 20 | 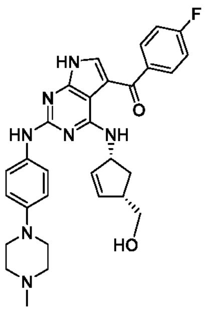 | $^1$H NMR (600 MHz, DMSO-$d_6$) δ 12.16 (s, 1H), 8.79 (s, 1H), 8.72 (d, *J* = 7.6 Hz, 1H), 7.82 (dd, *J* = 8.3, 5.7 Hz, 2H), 7.74 (d, *J* = 9.0 Hz, 2H), 7.45 (s, 1H), 7.35 (t, *J* = 8.8 Hz, 2H), 6.92 (d, *J* = 8.9 Hz, 2H), 6.05-5.96 (m, 1H), 5.96-5.87 (m, 1H), 5.35-5.20 (m, 1H), 4.99-4.49 (m, 2H), 3.49-3.37 (m, 4H), 3.32-3.15 (m, 4H), 2.85 (s, 3H), 2.82-2.75 (m, 1H), 2.58-2.51 (m, 2H), 1.48-1.37 (m, 1H). | 542.26 |

(continued)

| Example | Structure | H-NMR | Mass spectrum/ actua l measurement [ M+H]+ |
|---|---|---|---|
| 21 | 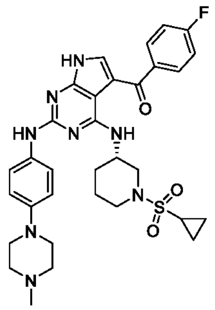 | $^1$H NMR (600 MHz, DMSO-$d_6$) δ 12.21 (s, 1H), 8.85 (d, $J$ = 7.9 Hz, 1H), 8.74 (s, 1H), 7.87-7.81 (m, 2H), 7.63 (d, $J$ = 9.0 Hz, 2H), 7.47 (s, 1H), 7.39-7.32 (m, 2H), 6.85 (d, $J$ = 9.1 Hz, 2H), 4.42-4.32 (m, 1H), 3.68-3.58 (m, 1H), 3.33-3.31 (m, 1H), 3.16-3.07 (m, 2H), 3.07-2.97 (m, 4H), 2.63-2.58 (m, 1H), 2.49-2.46 (m, 4H), 2.23 (s, 3H), 2.04-1.88 (m, 2H), 1.72-1.57 (m, 2H), 0.99-0.88 (m, 4H). | 633.31 |
| 22 | 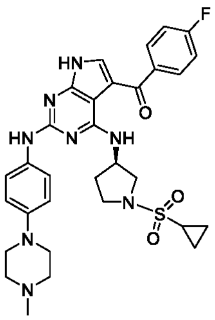 | $^1$H NMR (600 MHz, DMSO-$d_6$) δ 12.24 (s, 1H), 8.93 (d, $J$ = 6.1 Hz, 1H), 8.78 (s, 1H), 7.84 (dd, $J$ = 8.4, 5.7 Hz, 2H), 7.64 (d, $J$ = 8.9 Hz, 2H), 7.49 (s, 1H), 7.36 (t, $J$ = 8.8 Hz, 2H), 6.87 (d, $J$ = 9.0 Hz, 2H), 4.80-4.57 (m, 1H), 3.84-3.73 (m, 1H), 3.60-3.45 (m, 2H), 3.33-3.31 (m, 1H), 3.11-2.98 (m, 4H), 2.73-2.64 (m, 1H), 2.49-2.46 (m, 4H), 2.43-2.39 (m, 1H), 2.24 (s, 3H), 2.09-2.01 (m, 1H), 0.98-0.87 (m, 4H). | 619.29 |
| 23 | 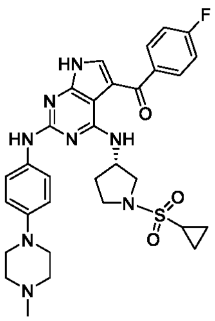 | $^1$H NMR (600 MHz, DMSO-$d_6$) δ 9.05 (s, 1H), 8.98 (d, $J$ = 6.2 Hz, 1H), 7.98-7.93 (m, 2H), 7.87 (s, 1H), 7.80 (d, $J$ = 8.9 Hz, 2H), 7.51-7.46 (m, 2H), 6.97 (d, $J$ = 9.1 Hz, 2H), 4.85-4.74 (m, 1H), 3.92-3.85 (m, 1H), 3.66-3.58 (m, 2H), 3.43-3.43 (m, 1H), 3.21-3.09 (m, 4H), 2.83-2.76 (m, 1H), 2.59-2.56 (m, 4H), 2.55-2.50 (m, 1H), 2.34 (s, 3H), 2.19-2.10 (m, 1H), 1.06-0.99 (m, 4H). | 618.73 |
| 24 | 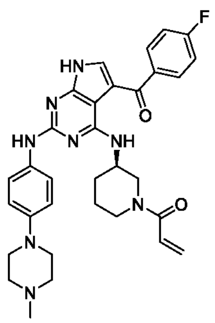 | $^1$H NMR (600 MHz, DMSO-$d_6$) δ 12.18 (s, 1H), 8.82 (d, $J$ = 6.8 Hz, 1H), 8.72 (s, 1H), 8.01-7.68 (m, 2H), 7.63 (d, $J$ = 8.3 Hz, 2H), 7.50-7.43 (m, 1H), 7.40-7.28 (m, 2H), 6.90-6.51 (m, 3H), 6.12-5.92 (m, 1H), 5.71-5.39 (m, 1H), 4.32-4.18 (m, 1H), 3.88-3.71 (m, 1H), 3.68-3.40 (m, 2H), 3.33-3.30 (m, 1H), 3.08-2.95 (m, 4H), 2.48-2.44 (m, 4H), 2.23 (s, 3H), 2.11-2.03 (m, 1H), 1.92-1.80 (m, 1H), 1.79-1.63 (m, 1H), 1.61-1.50 (m, 1H). | 583.32 |

(continued)

| Example | Structure | H-NMR | Mass spectrum/ actua l measurement [ M+H]+ |
|---|---|---|---|
| 25 | 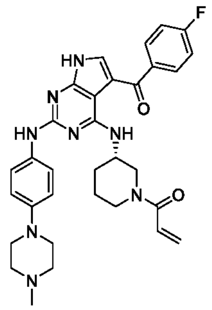 | $^{1}$H NMR (600 MHz, DMSO-$d_6$) δ 12.19 (s, 1H), 8.82 (d, *J* = 7.0 Hz, 1H), 8.72 (s, 1H), 7.88-7.75 (m, 2H), 7.63 (d, *J* = 8.1 Hz, 2H), 7.46 (s, 1H), 7.38-7.28 (m, 2H), 6.95-6.47 (m, 3H), 6.13-5.90 (m, 1H), 5.70-5.39 (m, 1H), 4.33-4.17 (m, 1H), 3.89-3.70 (m, 1H), 3.66-3.40 (m, 3H), 3.09-2.96 (m, 4H), 2.49-2.45 (m, 4H), 2.25 (s, 3H), 2.10-2.04 (m, 1H), 1.90-1.80 (m, 1H), 1.79-1.64 (m, 1H), 1.61-1.51 (m, 1H). | 583.37 |
| 26 | 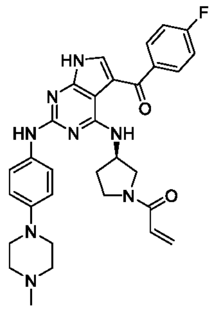 | $^{1}$H NMR (600 MHz, DMSO-$d_6$) δ 12.23 (s, 1H), 8.96-8.88 (m, 1H), 8.79 (s, 1H), 7.90-7.79 (m, 2H), 7.71-7.62 (m, 2H), 7.51-7.44 (m, 1H), 7.35 (t, *J* = 8.6 Hz, 2H), 6.95-6.82 (m, 2H), 6.69-6.55 (m, 1H), 6.19-6.12 (m, 1H), 5.71-5.63 (m, 1H), 4.74-4.60 (m, 1H), 4.11-3.74 (m, 2H), 3.67-3.42 (m, 3H), 3.33-3.32 (m, 1H), 3.13-2.97 (m, 4H), 2.53-2.52 (m, 4H), 2.39-2.21 (m, 4H), 2.13-1.97 (m, 1H). | 569.29 |
| 27 | 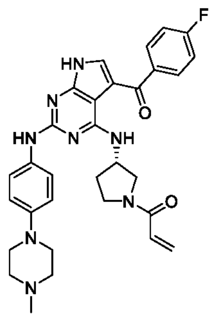 | $^{1}$H NMR (600 MHz, DMSO-$d_6$) δ 12.23 (s, 1H), 8.91 (dd, *J* = 15.1, 6.3 Hz, 1H), 8.79 (s, 1H), 7.86-7.78 (m, 2H), 7.66 (dd, *J* = 11.9, 9.0 Hz, 2H), 7.48 (s, 1H), 7.35 (t, *J* = 8.6 Hz, 2H), 6.86 (dd, *J* = 9.2, 2.6 Hz, 2H), 6.70-6.53 (m, 1H), 6.15 (ddd, *J* = 16.8, 5.3, 2.4 Hz, 1H), 5.68 (ddd, *J* = 19.4, 10.3, 2.3 Hz, 1H), 4.75-4.59 (m, 1H), 4.11-3.81 (m, 1H), 3.80-3.73 (m, 1H), 3.67-3.46 (m, 3H), 3.33-3.32 (m, 1H), 3.10-3.00 (m, 4H), 2.53-2.51 (m, 4H), 2.38-2.21 (m, 4H), 2.14-1.93 (m, 1H). | 569.32 |
| 28 | 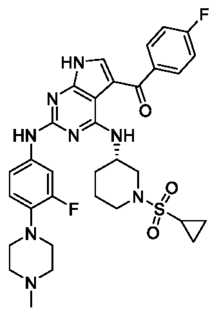 | $^{1}$H NMR (600 MHz, DMSO-$d_6$) δ 12.31 (s, 1H), 9.07 (s, 1H), 8.92 (d, *J* = 7.8 Hz, 1H), 7.94-7.79 (m, 3H), 7.53 (s, 1H), 7.41 (d, *J* = 8.0 Hz, 1H), 7.36 (t, *J* = 8.7 Hz, 2H), 6.94 (t, *J* = 9.4 Hz, 1H), 4.46-4.33 (m, 1H), 3.66-3.54 (m, *J* = 8.9 Hz, 1H), 3.21-3.10 (m, 2H), 3.07-2.86 (m, 4H), 2.64-2.57 (m, 2H), 2.56-2.52 (m, 4H), 2.26 (s, 3H), 2.05-1.89 (m, 2H), 1.73-1.59 (m, 2H), 0.98-0.88 (m, 4H). | 651.29 |

(continued)

| Example | Structure | H-NMR | Mass spectrum/ actua l measurement [ M+H]+ |
|---|---|---|---|
| 29 | 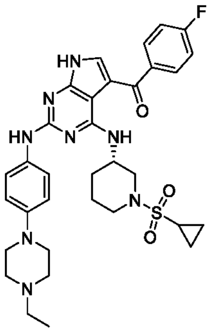 | $^1$H NMR (600 MHz, DMSO-$d_6$) δ 12.21 (s, 1H), 8.85 (d, *J* = 7.8 Hz, 1H), 8.74 (s, 1H), 7.84 (dd, *J* = 8.5, 5.6 Hz, 2H), 7.63 (d, *J* = 8.9 Hz, 2H), 7.47 (s, 1H), 7.36 (t, *J* = 8.8 Hz, 2H), 6.86 (d, *J* = 8.9 Hz, 2H), 4.43-4.31 (m, 1H), 3.69-3.59 (m, 1H), 3.18-2.97 (m, 6H), 2.64-2.58 (m, 2H), 2.57-2.52 (m, 4H), 2.46-2.36 (m, 2H), 2.05-1.88 (m, 2H), 1.74-1.58 (m, 2H), 1.05 (t, *J* = 7.0 Hz, 3H), 0.97-0.87 (m, 4H). | 647.33 |
| 30 | 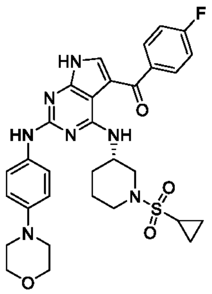 | $^1$H NMR (600 MHz, DMSO-$d_6$) δ 12.21 (s, 1H), 8.85 (d, *J* = 7.6 Hz, 1H), 8.76 (s, 1H), 7.98-7.74 (m, 2H), 7.65 (d, *J* = 8.6 Hz, 2H), 7.48 (s, 1H), 7.36 (t, *J* = 8.6 Hz, 2H), 6.86 (d, *J* = 8.6 Hz, 2H), 4.49-4.26 (m, 1H), 3.82-3.68 (m, 4H), 3.68-3.58 (m, 1H), 3.33-3.32 (m, 1H), 3.20-3.06 (m, 2H), 3.06-2.95 (m, 4H), 2.61-2.58 (m, 1H), 2.08-1.86 (m, 2H), 1.75-1.57 (m, 2H), 1.03-0.87 (m, 4H). | 620.28 |
| 31 | 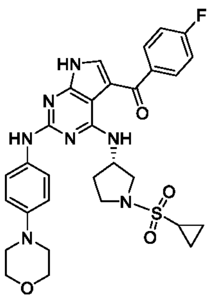 | $^1$H NMR (600 MHz, DMSO-$d_6$) δ 12.24 (s, 1H), 8.94 (d, *J* = 6.2 Hz, 1H), 8.80 (s, 1H), 7.90-7.77 (m, 2H), 7.67 (dd, *J* = 9.0, 3.7 Hz, 2H), 7.50 (d, *J* = 3.0 Hz, 1H), 7.41-7.31 (m, 2H), 6.88 (d, *J* = 9.1 Hz, 2H), 4.73-4.63 (m, 1H), 3.81-3.77 (m, 1H), 3.76-3.72 (m, 4H), 3.59-3.47 (m, 2H), 3.33-3.31 (m, 1H), 3.06-2.99 (m, 4H), 2.72-2.65 (m, 1H), 2.43-2.38 (m, 1H), 2.08-2.01 (m, 1H), 0.98-0.89 (m, 4H). | 606.26 |
| 32 | 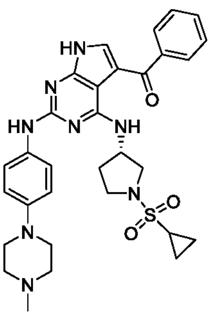 | $^1$H NMR (600 MHz, DMSO-$d_6$) δ 12.22 (s, 1H), 9.00 (d, *J* = 6.2 Hz, 1H), 8.78 (s, 1H), 7.78-7.73 (m, 2H), 7.67-7.60 (m, 3H), 7.54 (t, *J* = 7.6 Hz, 2H), 7.45 (s, 1H), 6.87 (d, *J* = 9.1 Hz, 2H), 4.74-4.63 (m, 1H), 3.83-3.76 (m, 1H), 3.58-3.48 (m, 2H), 3.33-3.31 (m, 1H), 3.14-3.00 (m, 4H), 2.73-2.66 (m, 1H), 2.54-2.51 (m, 4H), 2.44-2.39 (m, 1H), 2.25 (s, 3H), 2.10-2.02 (m, 1H), 0.97-0.89 (m, 4H). | 601.30 |

(continued)

| Example | Structure | H-NMR | Mass spectrum/ actua l measurement [ M+H]+ |
|---|---|---|---|
| 33 | 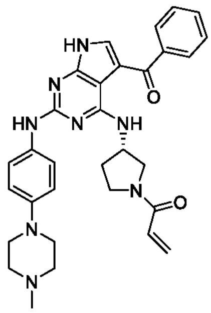 | $^1$H NMR (600 MHz, DMSO-$d_6$) δ 12.21 (s, 1H), 8.99 (dd, *J* = 15.2, 6.1 Hz, 1H), 8.79 (s, 1H), 7.74 (d, *J* = 7.4 Hz, 2H), 7.70-7.59 (m, 3H), 7.53 (t, *J* = 7.4 Hz, 2H), 7.43 (s, 1H), 6.86 (d, *J* = 6.6 Hz, 2H), 6.62 (ddd, *J* = 45.6, 16.7, 10.3 Hz, 1H), 6.16 (dd, *J* = 16.6, 2.5 Hz, 1H), 5.68 (dd, *J* = 18.6, 10.3 Hz, 1H), 4.79-4.56 (m, 1H), 4.12-3.75 (m, 2H), 3.64-3.44 (m, 2H), 3.14-2.99 (m, 4H), 2.53-2.52 (m, 4H), 2.40-2.17 (m, 4H), 2.15-1.95 (m, 1H). | 551.30 |
| 34 | 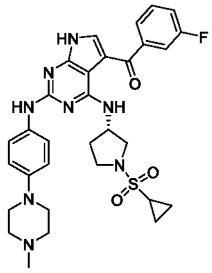 | $^1$H NMR (600 MHz, DMSO-$d_6$) δ 12.28 (s, 1H), 8.91 (d, *J* = 6.1 Hz, 1H), 8.80 (s, 1H), 7.64 (d, *J* = 8.9 Hz, 2H), 7.60-7.51 (m, 4H), 7.49-7.44 (m, 1H), 6.87 (d, *J* = 9.0 Hz, 2H), 4.75-4.61 (m, 1H), 3.84-3.74 (m, 1H), 3.59-3.48 (m, 2H), 3.33-3.31 (m, 1H), 3.12-3.00 (m, 4H), 2.73-2.66 (m, 1H), 2.53-2.52 (m, 4H), 2.44-2.39 (m, 1H), 2.26 (s, 3H), 2.14-1.93 (m, 1H), 0.97-0.89 (m, 4H). | 619.28 |
| 35 | 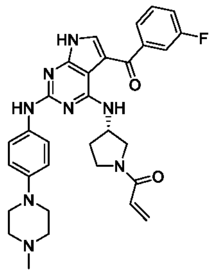 | $^1$H NMR (600 MHz, DMSO-$d_6$) δ 12.27 (s, 1H), 8.89 (dd, *J* = 14.0, 6.3 Hz, 1H), 8.80 (s, 1H), 7.66 (dd, *J* = 12.0, 9.0 Hz, 2H), 7.60-7.50 (m, 4H), 7.49-7.43 (m, 1H), 6.86 (dd, *J* = 9.2, 2.7 Hz, 2H), 6.62 (ddd, *J* = 46.8, 16.8, 10.3 Hz, 1H), 6.15 (ddd, *J* = 16.8, 5.3, 2.4 Hz, 1H), 5.68 (ddd, *J* = 19.3, 10.3, 2.4 Hz, 1H), 4.84-4.54 (m, 1H), 4.11-3.73 (m, 2H), 3.71-3.43 (m, 2H), 3.11-2.97 (m, 4H), 2.53-2.52 (m, 4H), 2.39-2.19 (m, 4H), 2.16-1.95 (m, 1H). | 569.30 |
| 36 | 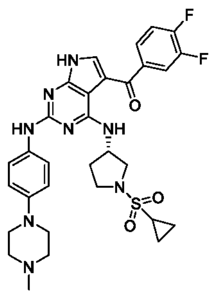 | $^1$H NMR (600 MHz, DMSO-$d_6$) δ 12.30 (s, 1H), 8.85 (d, *J* = 6.2 Hz, 1H), 8.80 (s, 1H), 7.86-7.81 (m, 1H), 7.68-7.56 (m, 5H), 6.87 (d, *J* = 9.1 Hz, 2H), 4.73-4.61 (m, 1H), 3.84-3.75 (m, 1H), 3.57-3.48 (m, 2H), 3.33-3.32 (m, 1H), 3.12-2.98 (m, 4H), 2.73-2.66 (m, 1H), 2.54-2.52 (m, 4H), 2.44-2.39 (m, 1H), 2.25 (s, 3H), 2.07-2.00 (m, 1H), 0.99-0.87 (m, 4H). | 637.28 |
| 37 | 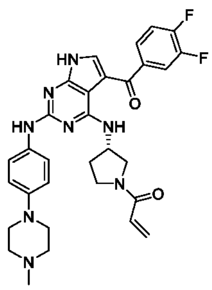 | $^1$H NMR (600 MHz, DMSO-$d_6$) δ 12.29 (s, 1H), 8.97-8.73 (m, 2H), 7.85-7.80 (m, 1H), 7.69-7.54 (m, 5H), 6.86 (dd, *J* = 9.0, 2.4 Hz, 2H), 6.62 (ddd, *J* = 47.6, 16.7, 10.3 Hz, 1H), 6.15 (ddd, *J* = 16.8, 5.4, 2.3 Hz, 1H), 5.68 (ddd, *J* = 20.0, 10.3, 2.2 Hz, 1H), 4.77-4.59 (m, 1H), 4.10-3.46 (m, 4H), 3.13-3.00 (m, 4H), 2.53-2.52 (m, 4H), 2.38-2.19 (m, 4H), 2.12-1.96 (m, 1H). | 587.26 |

(continued)

| Example | Structure | H-NMR | Mass spectrum/ actua l measurement [ M+H]+ |
|---|---|---|---|
| 38 | 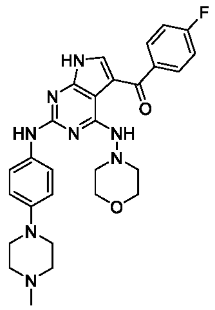 | $^1$H NMR (600 MHz, DMSO-$d_6$) δ 12.24 (s, 1H), 9.59 (s, 1H), 8.87 (s, 1H), 7.85 (dd, *J* = 8.6, 5.6 Hz, 2H), 7.80 (d, *J* = 8.4 Hz, 2H), 7.49 (s, 1H), 7.36 (t, *J* = 8.8 Hz, 2H), 6.90-6.82 (m, 2H), 3.86-3.69 (m, 4H), 3.14-3.01 (m, 4H), 3.01-2.88 (m, 4H), 2.58-2.53 (m, 4H), 2.29 (s, 3H). | 531.27 |
| 39 | 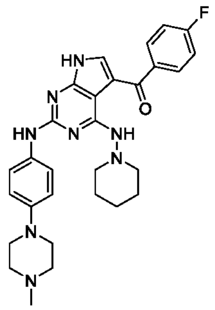 | $^1$H NMR (600 MHz, DMSO) δ 12.20 (s, 1H), 9.56 (s, 1H), 8.83 (s, 1H), 7.92-7.83 (m, 2H), 7.79 (d, *J* = 8.5 Hz, 2H), 7.46 (s, 1H), 7.40-7.30 (m, 2H), 6.84 (d, *J* = 9.1 Hz, 2H), 3.11-2.99 (m, 4H), 2.97-2.76 (m, 4H), 2.54-2.52 (m, 4H), 2.26 (s, 3H), 1.75-1.65 (m, 4H), 1.52-1.43 (m, 2H). | 529.23 |
| 40 | 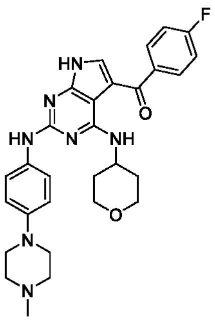 | $^1$H NMR (600 MHz, DMSO-$d_6$) δ 12.16 (s, 1H), 8.77 (d, *J* = 7.2 Hz, 1H), 8.71 (s, 1H), 7.90-7.77 (m, 2H), 7.69-7.62 (m, 2H), 7.46 (s, 1H), 7.39-7.32 (m, 2H), 6.88-6.83 (m, 2H), 4.32-4.21 (m, 1H), 3.96-3.89 (m, 2H), 3.56-3.48 (m, 2H), 3.10-2.99 (m, 4H), 2.49-2.45 (m, 4H), 2.24 (s, 3H), 2.13-2.00 (m, 2H), 1.59-1.50 (m, 2H). | 530.23 |
| 41 | 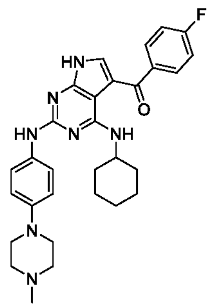 | $^1$H NMR (600 MHz, DMSO-$d_6$) δ 12.12 (s, 1H), 8.74 (d, *J* = 7.6 Hz, 1H), 8.67 (s, 1H), 7.87-7.81 (m, 2H), 7.71-7.65 (m, 2H), 7.43 (s, 1H), 7.38-7.33 (m, 2H), 6.84 (d, *J* = 9.1 Hz, 2H), 4.18-4.03 (m, 1H), 3.10-2.98 (m, 4H), 2.49-2.46 (m, 4H), 2.24 (s, 3H), 2.09-1.95 (m, 2H), 1.82-1.73 (m, 2H), 1.63-1.30 (m, 6H). | 528.25 |

(continued)

| Example | Structure | H-NMR | Mass spectrum/ actual measurement [M+H]$^+$ |
|---|---|---|---|
| 42 | 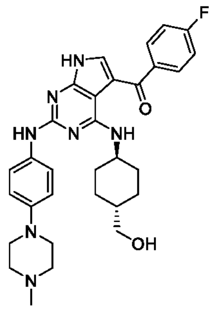 | $^1$H NMR (600 MHz, DMSO-$d_6$) δ 12.13 (s, 1H), 8.86-8.49 (m, 2H), 7.88-7.79 (m, 2H), 7.67 (dd, *J* = 9.8, 2.7 Hz, 2H), 7.43 (s, 1H), 7.39-7.31 (m, 2H), 6.84 (d, *J* = 9.1 Hz, 2H), 4.47 (t, *J* = 5.3 Hz, 1H), 4.08-3.91 (m, 1H), 3.32-3.26 (m, 2H), 3.11-2.97 (m, 4H), 2.49-2.46 (m, 4H), 2.24 (s, 3H), 2.19-2.10 (m, 2H), 1.88-1.79 (m, 2H), 1.48-1.38 (m, 1H), 1.32-1.24 (m, 2H), 1.14-1.02 (m, 2H). | 558.27 |
| 43 | 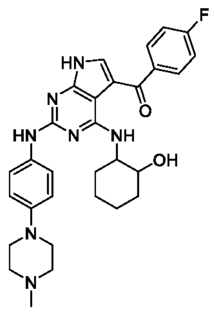 | $^1$H NMR (600 MHz, DMSO-$d_6$) δ 12.10 (s, 1H), 8.76 (d, *J* = 7.4 Hz, 1H), 8.65 (s, 1H), 7.88-7.79 (m, 2H), 7.71-7.64 (m, 2H), 7.42 (d, *J* = 2.4 Hz, 1H), 7.39-7.30 (m, 2H), 6.84 (d, *J* = 9.1 Hz, 2H), 4.80 (d, *J* = 4.2 Hz, 1H), 4.03-3.93 (m, 1H), 3.53-3.42 (m, 1H), 3.12-2.97 (m, 4H), 2.54-2.51 (m, 4H), 2.24 (s, 3H), 2.21-2.14 (m, 1H), 1.98-1.89 (m, 1H), 1.75-1.62 (m, 2H), 1.40-1.25 (m, 4H). | 544.30 |
| 44 | 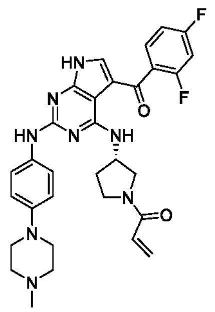 | $^1$H NMR (600 MHz, DMSO-$d_6$) δ 12.29 (s, 1H), 9.06-8.63 (m, 2H), 7.74-7.37 (m, 5H), 7.21 (t, *J* = 7.4 Hz, 1H), 6.86 (dd, *J* = 9.2, 2.8 Hz, 2H), 6.62 (ddd, *J* = 44.7, 16.8, 10.3 Hz, 1H), 6.15 (ddd, *J* = 16.8, 4.6, 2.4 Hz, 1H), 5.68 (ddd, *J* = 18.8, 10.3, 2.3 Hz, 1H), 4.81-4.54 (m, 1H), 4.11-3.74 (m, 2H), 3.65-3.45 (m, 2H), 3.11-2.98 (m, 4H), 2.53-2.52 (m, 4H), 2.38-2.28 (m, 1H), 2.25 (s, 3H), 2.15-1.97 (m, 1H). | 587.27 |
| 45 | 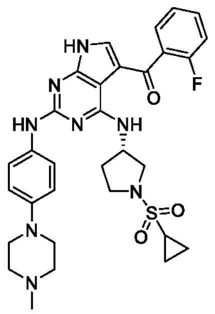 | $^1$H NMR (600 MHz, DMSO-$d_6$) δ 12.29 (s, 1H), 8.87 (d, *J* = 6.1 Hz, 1H), 8.81 (s, 1H), 7.67-7.57 (m, 4H), 7.42-7.29 (m, 3H), 6.87 (d, *J* = 9.1 Hz, 2H), 4.76-4.63 (m, 1H), 3.84-3.72 (m, 1H), 3.61-3.46 (m, 2H), 3.36-3.35 (m, 1H), 3.10-3.02 (m, 4H), 2.70-2.64 (m, 1H), 2.50-2.45 (m, 4H), 2.45-2.37 (m, 1H), 2.24 (s, 3H), 2.10-2.01 (m, 1H), 0.97-0.87 (m, 4H). | 619.26 |

(continued)

| Example | Structure | H-NMR | Mass spectrum/ actual measurement [ M+H]+ |
|---|---|---|---|
| 46 | 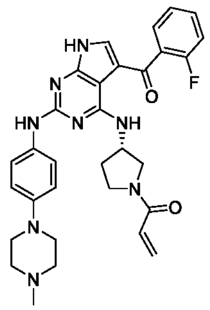 | $^1$H NMR (600 MHz, DMSO-$d_6$) δ 12.27 (s, 1H), 8.98-8.68 (m, 2H), 7.72-7.55 (m, 4H), 7.41-7.28 (m, 3H), 6.86 (dd, *J* = 9.2, 2.7 Hz, 2H), 6.62 (ddd, *J* = 43.7, 16.7, 10.3 Hz, 1H), 6.16 (ddd, *J* = 16.8, 4.6, 2.4 Hz, 1H), 5.68 (ddd, *J* = 18.2, 10.3, 2.3 Hz, 1H), 4.80-4.58 (m, 1H), 4.14-3.81 (m, 1H), 3.81-3.72 (m, 1H), 3.63-3.47 (m, 2H), 3.10-3.00 (m, 4H), 2.50-2.44 (m, 4H), 2.42-2.29 (m, 1H), 2.25 (s, 3H), 2.16-1.97 (m, 1H). | 569.28 |
| 47 | 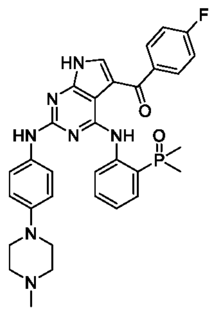 | $^1$H NMR (600 MHz, DMSO-$d_6$) δ 12.41 (s, 1H), 10.32 (s, 1H), 8.77 (s, 1H), 7.95-7.85 (m, 3H), 7.73-7.63 (m, 3H), 7.57-7.41 (m, 3H), 7.41-7.34 (m, 2H), 6.71 (d, *J* = 8.0 Hz, 2H), 3.09-2.92 (m, 4H), 2.55-2.51 (m, 4H), 2.26 (s, 3H), 1.68 (d, *J* = 13.4 Hz, 6H). | 598.26 |
| 48 | 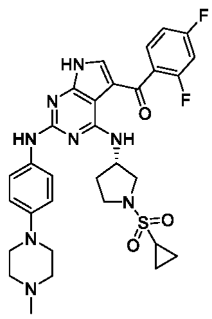 | $^1$H NMR (600 MHz, DMSO-$d_6$) δ 12.31 (s, 1H), 8.82 (s, 1H), 8.81 (s, 1H), 7.72-7.61 (m, 3H), 7.45 (s, 1H), 7.42 (dd, *J* = 9.8, 2.3 Hz, 1H), 7.25-7.19 (m, *J* = 8.4, 2.1 Hz, 1H), 6.87 (d, *J* = 9.1 Hz, 2H), 4.79-4.64 (m, 1H), 3.85-3.72 (m, 1H), 3.58-3.47 (m, 2H), 3.33-3.28 (m, 1H), 3.09-3.03 (m, 4H), 2.70-2.64 (m, 1H), 2.49-2.46 (m, 4H), 2.44-2.38 (m, 1H), 2.24 (s, 3H), 2.09-2.01 (m, 1H), 0.96-0.89 (m, 4H). | 637.05 |
| 49 | 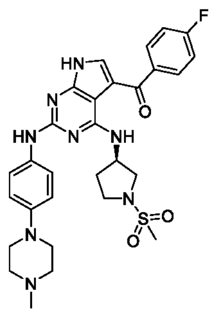 | $^1$H NMR (600 MHz, DMSO-$d_6$) δ 12.24 (s, 1H), 8.92 (d, *J* = 6.2 Hz, 1H), 8.78 (s, 1H), 7.89-7.80 (m, 2H), 7.68-7.61 (m, 2H), 7.50 (s, 1H), 7.39-7.33 (m, 2H), 6.87 (d, *J* = 9.1 Hz, 2H), 4.71-4.60 (m, 1H), 3.79-3.70 (m, 1H), 3.54-3.41 (m, 2H), 3.27-3.20 (m, 1H), 3.11-2.98 (m, 4H), 2.93 (s, 3H), 2.49-2.45 (m, 4H), 2.41-2.35 (m, 1H), 2.24 (s, 3H), 2.09-1.99 (m, 1H). | 593.18 |

(continued)

| Example | Structure | H-NMR | Mass spectrum/ actual measurement [ M+H]+ |
|---|---|---|---|
| 50 | 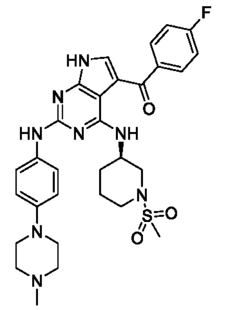 | $^1$H NMR (600 MHz, DMSO-$d_6$) δ 12.21 (s, 1H), 8.85 (d, *J* = 7.9 Hz, 1H), 8.75 (s, 1H), 7.89-7.78 (m, 2H), 7.66-7.59 (m, 2H), 7.47 (s, 1H), 7.35 (dd, *J* = 12.3, 5.4 Hz, 2H), 6.86 (d, *J* = 9.1 Hz, 2H), 4.45-4.32 (m, 1H), 3.61-3.54 (m, 1H), 3.31-3.27 (m, 1H), 3.10-2.97 (m, 6H), 2.89 (s, 3H), 2.49-2.46 (m, 4H), 2.24 (s, 3H), 2.05-1.86 (m, 2H), 1.74-1.54 (m, 2H). | 607.22 |
| 51 | 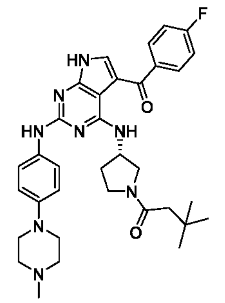 | $^1$H NMR (600 MHz, DMSO-$d_6$) δ 12.21 (s, 1H), 9.00-8.84 (m, 1H), 8.78 (d, *J* = 6.1 Hz, 1H), 7.88-7.73 (m, 2H), 7.71-7.61 (m, 2H), 7.48 (s, 1H), 7.41-7.31 (m, 2H), 6.86 (d, *J* = 7.7 Hz, 2H), 4.75-4.56 (m, 1H), 3.96-3.39 (m, 4H), 3.14-2.98 (m, 4H), 2.53-2.52 (m, 4H), 2.37-2.25 (m, 4H), 2.21-2.10 (m, 2H), 2.08-1.91 (m, 1H), 0.98 (d, *J* = 16.9 Hz, 9H). | 613.31 |
| 52 | 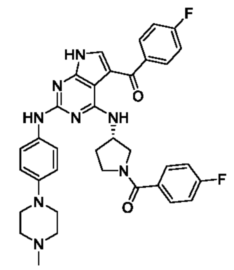 | $^1$H NMR (600 MHz, DMSO-$d_6$) δ 12.23 (s, 1H), 8.95 (dd, *J* = 39.8, 6.0 Hz, 1H), 8.75 (d, *J* = 53.9 Hz, 1H), 7.88-7.81 (m, 2H), 7.71-7.61 (m, 3H), 7.55 (d, *J* = 8.5 Hz, 1H), 7.49 (d, *J* = 13.7 Hz, 1H), 7.41-7.33 (m, 2H), 7.32-7.20 (m, 2H), 6.87 (d, *J* = 8.8 Hz, 1H), 6.78 (d, *J* = 8.7 Hz, 1H), 4.76-4.58 (m, 1H), 4.00-3.89 (m, 1H), 3.77-3.44 (m, 3H), 3.08-2.99 (m, 4H), 2.48-2.44 (m, 4H), 2.39-2.28 (m, 1H), 2.23 (s, 3H), 2.13-1.97 (m, 1H). | 637.23 |
| 53 | 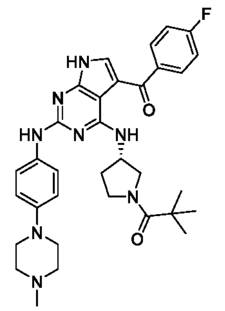 | $^1$H NMR (600 MHz, DMSO-$d_6$) δ 12.21 (s, 1H), 8.90 (d, *J* = 6.4 Hz, 1H), 8.78 (s, 1H), 7.82 (dd, *J* = 8.5, 5.6 Hz, 2H), 7.66 (d, *J* = 8.8 Hz, 2H), 7.48 (s, 1H), 7.35 (t, *J* = 8.8 Hz, 2H), 6.86 (d, *J* = 9.0 Hz, 2H), 4.82-4.48 (m, 1H), 4.00-3.44 (m, 4H), 3.15-2.98 (m, 4H), 2.54-2.52 (m, 4H), 2.35-2.18 (m, 4H), 2.10-1.86 (m, 1H), 1.17 (s, 9H). | 599.27 |
| 54 | 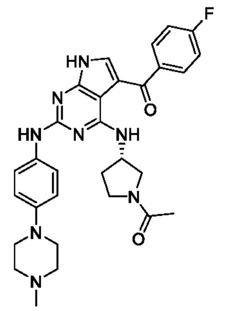 | $^1$H NMR (600 MHz, DMSO-$d_6$) δ 12.23 (s, 1H), 8.91 (dd, *J* = 21.6, 6.4 Hz, 1H), 8.77 (s, 1H), 7.87-7.80 (m, 2H), 7.69-7.62 (m, 2H), 7.48 (d, *J* = 3.2 Hz, 1H), 7.35 (t, *J* = 8.8 Hz, 2H), 6.86 (dd, *J* = 9.1, 2.2 Hz, 2H), 4.75-4.57 (m, 1H), 3.96-3.42 (m, 4H), 3.11-3.00 (m, 4H), 2.50-2.46 (m, 4H), 2.40-2.27 (m, 1H), 2.24 (s, 3H), 2.11-1.93 (m, 4H). | 557.21 |

(continued)

| Example | Structure | H-NMR | Mass spectrum/ actual measurement [M+H]$^+$ |
|---|---|---|---|
| 55 | 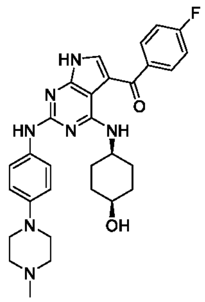 | $^1$H NMR (600 MHz, DMSO-$d_6$) δ 12.10 (s, 1H), 8.82 (d, *J* = 7.5 Hz, 1H), 8.63 (s, 1H), 7.89-7.81 (m, 2H), 7.71-7.62 (m, 2H), 7.42 (s, 1H), 7.38-7.32 (m, 2H), 6.83 (d, *J* = 9.1 Hz, 2H), 4.52 (d, *J* = 3.4 Hz, 1H), 4.24-4.13 (m, 1H), 3.73-3.65 (m, 1H), 3.07-3.01 (m, 4H), 2.47-2.43 (m, 4H), 2.22 (s, 3H), 1.83-1.70 (m, 4H), 1.70-1.62 (m, 4H). | 544.23 |
| 56 | 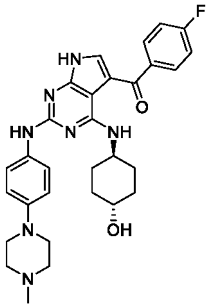 | $^1$H NMR (600 MHz, DMSO-$d_6$) δ 12.10 (s, 1H), 8.69-8.60 (m, *J* = 8.5 Hz, 2H), 7.90-7.77 (m, 2H), 7.71-7.62 (m, 2H), 7.42 (s, 1H), 7.39-7.30 (m, 2H), 6.84 (d, *J* = 9.1 Hz, 2H), 4.60 (d, *J* = 4.4 Hz, 1H), 4.08-3.95 (m, 1H), 3.60-3.51 (m, 1H), 3.09-3.00 (m, 4H), 2.49-2.41 (m, 4H), 2.23 (s, 3H), 2.15-2.07 (m, 2H), 1.95-1.87 (m, 2H), 1.40-1.31 (m, 4H). | 544.24 |
| 57 | 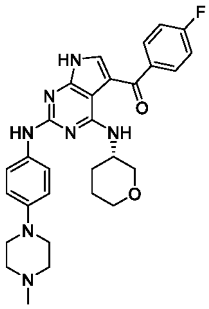 | $^1$H NMR (600 MHz, DMSO-$d_6$) δ 12.18 (s, 1H), 8.81 (d, *J* = 7.7 Hz, 1H), 8.72 (s, 1H), 7.88-7.81 (m, 2H), 7.70-7.60 (m, 2H), 7.45 (d, *J* = 2.1 Hz, 1H), 7.35 (t, *J* = 8.8 Hz, 2H), 6.84 (d, *J* = 9.1 Hz, 2H), 4.28-4.22 (m, 1H), 3.98-3.90 (m, 1H), 3.73-3.66 (m, 1H), 3.60-3.52 (m, 1H), 3.42-3.39 (m, 1H), 3.08-3.02 (m, 4H), 2.54-2.52 (m, 4H), 2.25 (s, 3H), 2.09-2.02 (m, 1H), 1.86-1.79 (m, 1H), 1.73-1.57 (m, 2H). | 530.23 |
| 58 | 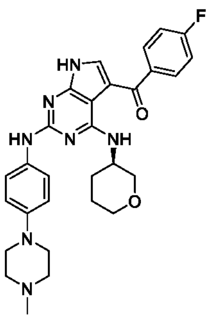 | $^1$H NMR (600 MHz, DMSO-$d_6$) δ 12.20 (s, 1H), 8.83 (d, *J* = 7.7 Hz, 1H), 8.81 (s, 1H), 7.89-7.80 (m, 2H), 7.71 (d, *J* = 9.1 Hz, 2H), 7.47 (d, *J* = 2.8 Hz, 1H), 7.41-7.32 (m, 2H), 6.91 (d, *J* = 9.1 Hz, 2H), 4.29-4.21 (m, 1H), 3.98-3.90 (m, 1H), 3.74-3.65 (m, 1H), 3.60-3.53 (m, 1H), 3.42-3.39 (m, 1H), 3.30-3.17 (m, 4H), 2.81 (s, 3H), 2.54-2.52 (m, 4H), 2.10-2.02 (m, 1H), 1.88-1.79 (m, 1H), 1.74-1.56 (m, 2H). | 530.22 |

(continued)

| Example | Structure | H-NMR | Mass spectrum/ actua l measurement [ M+H]+ |
|---|---|---|---|
| 59 | 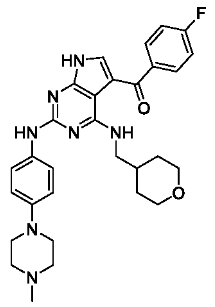 | $^1$H NMR (600 MHz, DMSO-$d_6$) δ 12.17 (s, 1H), 8.78 (t, *J* = 5.6 Hz, 1H), 8.70 (s, 1H), 7.86-7.80 (m, 2H), 7.67 (d, *J* = 9.1 Hz, 2H), 7.45 (s, 1H), 7.39-7.32 (m, 2H), 6.85 (d, *J* = 9.1 Hz, 2H), 3.94-3.85 (m, 2H), 3.51-3.43 (m, 2H), 3.32-3.28 (m, 2H), 3.12-2.99 (m, 4H), 2.55-2.51 (m, 4H), 2.25 (s, 3H), 1.97-1.84 (m, 1H), 1.74-1.67 (m, 2H), 1.40-1.28 (m, 2H). | 544.24 |
| 60 | 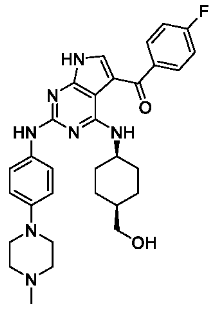 | $^1$H NMR (600 MHz, DMSO-$d_6$) δ 12.13 (s, 1H), 8.96 (d, *J* = 7.7 Hz, 1H), 8.66 (s, 1H), 7.89-7.80 (m, 2H), 7.66 (d, *J* = 9.1 Hz, 2H), 7.43 (d, *J* = 2.2 Hz, 1H), 7.35 (t, *J* = 8.8 Hz, 2H), 6.84 (d, *J* = 9.1 Hz, 2H), 4.47 (t, *J* = 5.3 Hz, 1H), 4.45-4.38 (m, 1H), 3.31-3.27 (m, 2H), 3.11-2.99 (m, 4H), 2.54-2.51 (m, 4H), 2.24 (s, 3H), 1.89-1.80 (m, 2H), 1.71-1.62 (m, 4H), 1.58-1.48 (m, 1H), 1.43-1.33 (m, 2H). | 558.27 |
| 61 | 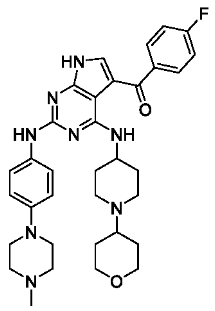 | $^1$H NMR (600 MHz, DMSO-$d_6$) δ 12.15 (s, 1H), 8.74 (d, *J* = 5.7 Hz, 1H), 8.68 (s, 1H), 7.94-7.75 (m, 2H), 7.65 (d, *J* = 8.4 Hz, 2H), 7.44 (s, 1H), 7.36 (t, *J* = 8.4 Hz, 2H), 6.85 (d, *J* = 8.5 Hz, 2H), 4.14-4.04 (m, 1H), 3.97-3.84 (m, 2H), 3.30-3.27 (m, 2H), 3.12-2.99 (m, 4H), 2.98-2.85 (m, 2H), 2.55-2.52 (m, 6H), 2.43-2.41 (m, 1H), 2.24 (s, 3H), 2.12-2.01 (m, 2H), 1.78-1.67 (m, 2H), 1.60-1.42 (m, 4H). | 613.31 |
| 62 | 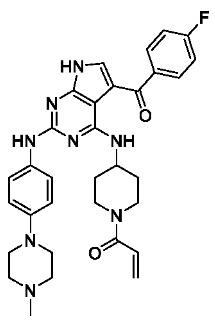 | $^1$H NMR (600 MHz, DMSO-$d_6$) δ 12.20 (s, 1H), 8.91-8.76 (m, 2H), 7.88-7.79 (m, 2H), 7.72 (d, *J* = 9.1 Hz, 2H), 7.48 (d, *J* = 2.9 Hz, 1H), 7.39-7.33 (m, 2H), 6.93 (d, *J* = 9.1 Hz, 2H), 6.87 (dd, *J* = 16.7, 10.5 Hz, 1H), 6.13 (dd, *J* = 16.7, 2.4 Hz, 1H), 5.69 (dd, *J* = 10.5, 2.4 Hz, 1H), 4.37-4.27 (m, 1H), 4.27-4.17 (m, 1H), 4.08-3.96 (m, 1H), 3.33-3.18 (m, 4H), 3.18-3.08 (m, 2H), 2.82 (s, 3H), 2.53-2.52 (m, 4H), 2.17-2.06 (m, 2H), 1.59-1.40 (m, 2H). | 583.27 |

(continued)

| Example | Structure | H-NMR | Mass spectrum/ actua l measurement [ M+H]+ |
|---|---|---|---|
| 63 | 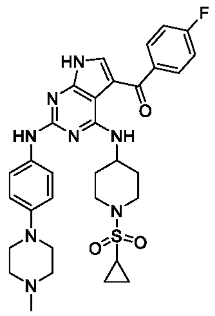 | 1H NMR (600 MHz, DMSO-$d_6$) δ 12.19 (s, 1H), 8.81 (d, *J* = 7.1 Hz, 1H), 8.72 (s, 1H), 7.89-7.79 (m, 2H), 7.65 (d, *J* = 9.0 Hz, 2H), 7.47 (s, 1H), 7.35 (t, *J* = 8.8 Hz, 2H), 6.86 (d, *J* = 9.1 Hz, 2H), 4.26-4.13 (m, 1H), 3.68-3.57 (m, 2H), 3.18-3.11 (m, 2H), 3.09-3.01 (m, 4H), 2.72-2.65 (m, 1H), 2.54-2.52 (m, 4H), 2.24 (s, 3H), 2.19-2.13 (m, 2H), 1.69-1.58 (m, 2H), 1.05-0.95 (m, 4H). | 633.25 |
| 64 | 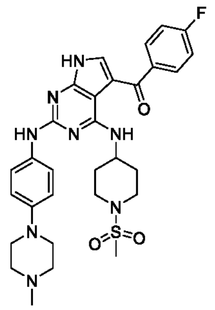 | 1H NMR (600 MHz, DMSO-$d_6$) δ 12.19 (s, 1H), 8.81 (d, *J* = 7.1 Hz, 1H), 8.72 (s, 1H), 7.84 (dd, *J* = 8.5, 5.6 Hz, 2H), 7.64 (d, *J* = 9.0 Hz, 2H), 7.47 (s, 1H), 7.36 (t, *J* = 8.8 Hz, 2H), 6.92-6.83 (m, 2H), 4.24-4.14 (m, 1H), 3.61-3.52 (m, 2H), 3.10-2.99 (m, 6H), 2.95 (s, 3H), 2.55-2.51 (m, 4H), 2.25 (s, 3H), 2.20-2.12 (m, 2H), 1.70-1.58 (m, 2H). | 607.25 |
| 65 | 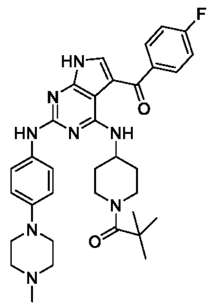 | 1H NMR (600 MHz, DMSO-$d_6$) δ 12.17 (s, 1H), 8.80 (d, *J* = 7.3 Hz, 1H), 8.73 (s, 1H), 7.83 (dd, *J* = 8.4, 5.7 Hz, 2H), 7.66 (d, *J* = 8.9 Hz, 2H), 7.46 (s, 1H), 7.35 (t, *J* = 8.8 Hz, 2H), 6.87 (d, *J* = 9.0 Hz, 2H), 4.38-4.26 (m, 1H), 4.15-4.06 (m, 2H), 3.26-3.15 (m, 2H), 3.11-2.99 (m, 4H), 2.58-2.46 (m, 4H), 2.24 (s, 3H), 2.19-2.01 (m, 2H), 1.53-1.29 (m, 2H), 1.23 (s, 9H). | 613.32 |
| 66 | 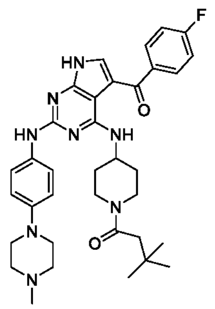 | 1H NMR (600 MHz, DMSO-$d_6$) δ 12.20 (s, 1H), 8.86-8.77 (m, 2H), 7.84 (dd, *J* = 8.6, 5.6 Hz, 2H), 7.72 (d, *J* = 9.0 Hz, 2H), 7.48 (d, *J* = 2.6 Hz, 1H), 7.36 (t, *J* = 8.8 Hz, 2H), 6.93 (d, *J* = 9.1 Hz, 2H), 4.33-4.21 (m, 2H), 4.00-3.92 (m, 1H), 3.28-2.99 (m, 6H), 2.80 (s, 3H), 2.55-2.51 (m, 4H), 2.28 (d, *J* = 1.7 Hz, 2H), 2.15-2.03 (m, 2H), 1.55-1.34 (m, 2H), 1.01 (s, 9H) | 627.34 |

(continued)

| Example | Structure | H-NMR | Mass spectrum/ actua l measurement [ M+H]$^+$ |
|---|---|---|---|
| 67 | 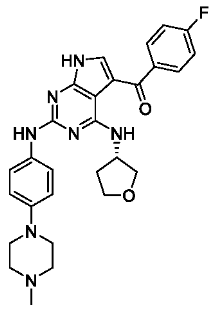 | $^1$H NMR (600 MHz, DMSO-$d_6$) δ 12.20 (s, 1H), 8.90 (d, $J$ = 6.5 Hz, 1H), 8.74 (s, 1H), 7.89-7.81 (m, 2H), 7.66 (d, $J$ = 9.0 Hz, 2H), 7.47 (s, 1H), 7.35 (t, $J$ = 8.8 Hz, 2H), 6.86 (d, $J$ = 9.1 Hz, 2H), 4.73-4.65 (m, 1H), 4.02-3.97 (m, 1H), 3.96-3.89 (m, 1H), 3.85-3.78 (m, 1H), 3.70-3.63 (m, 1H), 3.11-3.00 (m, 4H), 2.53-2.51 (m, 4H), 2.38-2.30 (m, 1H), 2.25 (s, 3H), 1.95-1.85 (m, 1H). | 516.23 |
| 68 | 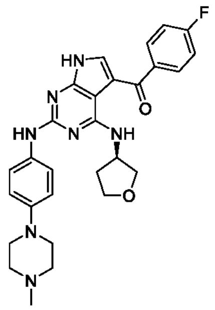 | $^1$H NMR (600 MHz, DMSO-$d_6$) δ 12.20 (s, 1H), 8.90 (d, $J$ = 6.5 Hz, 1H), 8.74 (s, 1H), 7.88-7.79 (m, 2H), 7.66 (d, $J$ = 9.0 Hz, 2H), 7.47 (s, 1H), 7.35 (t, $J$ = 8.8 Hz, 2H), 6.86 (d, $J$ = 9.1 Hz, 2H), 4.73-4.64 (m, 1H), 4.02-3.96 (m, 1H), 3.96-3.88 (m, 1H), 3.85-3.76 (m, 1H), 3.70-3.62 (m, 1H), 3.12-3.00 (m, 4H), 2.54-2.51 (m, 4H), 2.38-2.31 (m, 1H), 2.25 (s, 3H), 1.95-1.86 (m, 1H). | 516.23 |
| 69 | 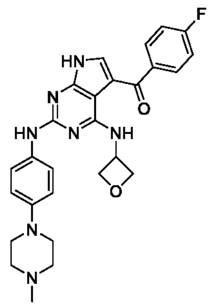 | $^1$H NMR (600 MHz, DMSO-$d_6$) δ 12.88 (s, 1H), 9.34 (s, 1H), 9.11 (s, 1H), 7.97-7.91 (m, 2H), 7.85 (s, 1H), 7.44-7.36 (m, 4H), 7.01 (d, $J$ = 9.0 Hz, 2H), 4.56-4.48 (m, 2H), 4.44-4.38 (m, 1H), 3.78-3.62 (m, 2H), 3.30-3.09 (m, 4H), 2.76 (s, 3H), 2.54-2.51(m, 4H). | 502.21 |
| 70 | 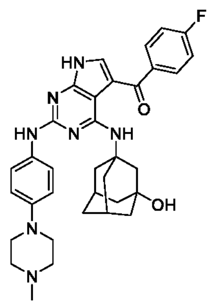 | $^1$H NMR (600 MHz, DMSO-$d_6$) δ 12.07 (s, 1H), 8.83 (s, 1H), 8.49 (s, 1H), 7.84-7.78 (m, 2H), 7.57 (d, $J$ = 8.9 Hz, 2H), 7.38 (s, 1H), 7.36-7.32 (m, 2H), 6.86 (d, $J$ = 9.1 Hz, 2H), 4.52 (s, 1H), 3.14-2.98 (m, 4H), 2.53-2.52 (m, 4H), 2.26 (s, 3H), 2.23-1.98 (m, 9H), 1.68-1.46 (m, 6H). (84-2) | 596.29 |

(continued)

| Example | Structure | H-NMR | Mass spectrum/ actua l measurement [ M+H]+ |
|---|---|---|---|
| 71 | 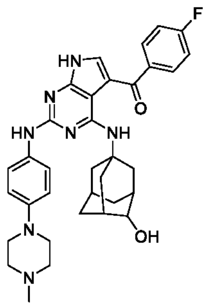 | $^1$H NMR (600 MHz, DMSO-$d_6$) δ 12.13 (s, 1H), 9.08 (d, *J* = 7.8 Hz, 1H), 8.69 (s, 1H), 7.88-7.82 (m, 2H), 7.67 (d, *J* = 9.1 Hz, 2H), 7.45 (d, *J* = 2.2 Hz, 1H), 7.35 (t, *J* = 8.8 Hz, 2H), 6.85 (d, *J* = 9.1 Hz, 2H), 4.55 (s, 1H), 4.37-4.27 (m, 1H), 3.13-3.01 (m, 4H), 2.53-2.51 (m, 4H), 2.32 (s, 3H), 2.23-2.17 (m, 2H), 2.14-2.09 (m, 1H), 2.04-1.98 (m, 2H), 1.87-1.81 (m, 2H), 1.75-1.70 (m, 2H), 1.69-1.66 (m, 2H), 1.52-1.43 (m, 2H). | 596.30 |
| 72 | 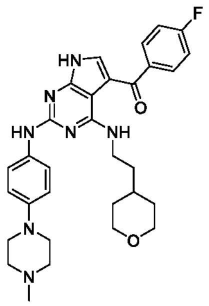 | $^1$H NMR (600 MHz, DMSO-$d_6$) δ 12.15 (s, 1H), 8.76-8.59 (m, 2H), 7.87-7.80 (m, 2H), 7.70-7.62 (m, 2H), 7.44 (s, 1H), 7.35 (t, *J* = 8.8 Hz, 2H), 6.84 (d, *J* = 9.1 Hz, 2H), 3.88-3.80 (m, 2H), 3.63-3.53 (m, 2H), 3.32-3.25 (m, 2H), 3.09-3.01 (m, 4H), 2.53-2.51 (m, 4H), 2.24 (s, 3H), 1.71-1.58 (m, 5H), 1.25-1.22 (m, 2H). | 558.27 |
| 73 | 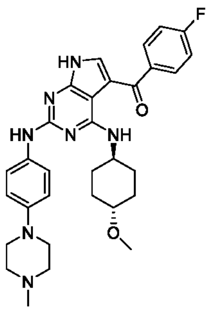 | $^1$H NMR (600 MHz, DMSO-$d_6$) δ 12.14 (s, 1H), 8.70 (d, *J* = 7.4 Hz, 1H), 8.69 (s, 1H), 7.87-7.80 (m, 2H), 7.67 (d, *J* = 9.0 Hz, 2H), 7.44 (s, 1H), 7.35 (t, *J* = 8.8 Hz, 2H), 6.84 (d, *J* = 9.1 Hz, 2H), 4.09-4.01 (m, 1H), 3.27 (s, 3H), 3.27-3.23 (m, 1H), 3.09-3.00 (m, 4H), 2.49-2.45 (m, 4H), 2.24 (s, 3H), 2.16-2.02 (m, 4H), 1.42-1.31 (m, 4H). | 558.27 |
| 74 | 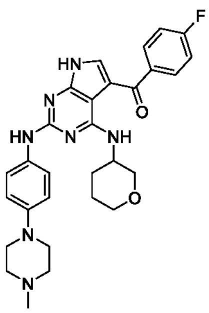 | $^1$H NMR (600 MHz, DMSO-$d_6$) δ 12.17 (s, 1H), 8.82 (d, *J* = 7.7 Hz, 1H), 8.78 (s, 1H), 7.92-7.78 (m, 2H), 7.74-7.68 (m, 2H), 7.46 (s, *J* = 2.8 Hz, 1H), 7.39-7.33 (m, 2H), 6.92 (d, *J* = 8.8 Hz, 2H), 4.32-4.20 (m, 1H), 3.99-3.90 (m, 1H), 3.73-3.66 (m, 1H), 3.58-3.54 (m, 1H), 3.42-3.39 (m, 1H), 3.32-3.28 (m, 4H), 2.82 (s, 3H), 2.52-2.52 (m, 4H), 2.13-1.94 (m, 1H), 1.91-1.76 (m, 1H), 1.73-1.56 (m, 2H). | 530.26 |

(continued)

| Example | Structure | H-NMR | Mass spectrum/ actua l measurement [ M+H]+ |
|---|---|---|---|
| 75 | 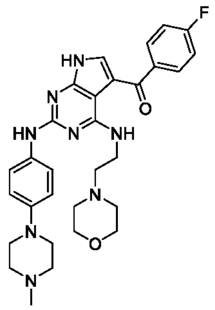 | $^{1}H$ NMR (600 MHz, DMSO-$d_6$) δ 12.11 (s, 1H), 8.76 (t, *J* = 5.3 Hz, 1H), 8.65 (s, 1H), 7.89-7.79 (m, 2H), 7.72-7.63 (m, 2H), 7.43 (s, 1H), 7.35 (t, *J* = 8.8 Hz, 2H), 6.85 (d, *J* = 9.0 Hz, 2H), 3.71-3.64 (m, 2H), 3.64-3.58 (m, 4H), 3.12-3.02 (m, 4H), 2.63-2.53 (m, 6H), 2.49-2.42 (m, 4H), 2.30 (s, 3H). | 559.29 |
| 76 | 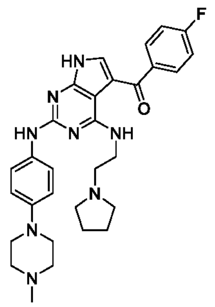 | $^{1}H$ NMR (600 MHz, DMSO-$d_6$) δ 12.30 (s, 1H), 8.88-8.71 (m, 2H), 7.92-7.77 (m, 2H), 7.66 (d, *J* = 8.8 Hz, 2H), 7.55 (s, 1H), 7.38 (t, *J* = 8.8 Hz, 2H), 6.95 (d, *J* = 8.9 Hz, 2H), 3.92-3.89 (m, 2H), 3.73-3.61 (m, 4H), 3.56-3.51 (m, 2H), 3.51-3.45 (m, 2H), 3.22-3.02 (m, 4H), 2.95-2.83 (m, 5H), 2.07-1.77 (m, 4H). | 543.30 |
| 77 | 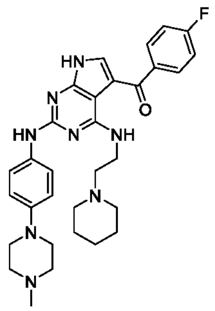 | **ND** | 557.31 |
| 78 | 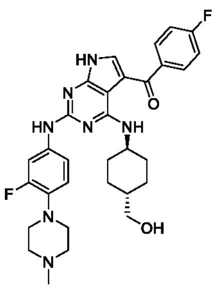 | $^{1}H$ NMR (600 MHz, DMSO-$d_6$) δ 12.18 (s, 1H), 8.97 (s, 1H), 8.71 (d, *J* = 7.5 Hz, 1H), 8.00 (dd, *J* = 16.0, 2.3 Hz, 1H), 7.88-7.79 (m, 2H), 7.47 (d, *J* = 2.5 Hz, 1H), 7.35 (t, *J* = 8.8 Hz, 2H), 7.31 (dd, *J* = 8.7, 1.8 Hz, 1H), 7.02-6.83 (m, 1H), 4.45 (t, *J* = 5.3 Hz, 1H), 4.03-3.94 (m, 1H), 3.29-3.26 (m, 2H), 3.02-2.88 (m, 4H), 2.52-2.52 (m, 4H), 2.27 (s, 3H), 2.21-2.12 (m, 2H), 1.89-1.81 (m, 2H), 1.48-1.39 (m, 1H), 1.35-1.25 (m, 2H), 1.15-1.04 (m, 2H). | 576.29 |
| 79 | 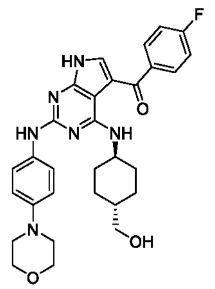 | $^{1}H$ NMR (600 MHz, DMSO-$d_6$) δ 12.11 (s, 1H), 8.65 (s, 1H), 8.63 (d, *J* = 7.7 Hz, 1H), 7.88-7.79 (m, 2H), 7.75-7.66 (m, 2H), 7.42 (d, *J* = 2.8 Hz, 1H), 7.35 (t, *J* = 8.8 Hz, 2H), 6.84 (d, *J* = 9.1 Hz, 2H), 4.44 (t, *J* = 5.3 Hz, 1H), 4.07-3.91 (m, 1H), 3.78-3.71 (m, 4H), 3.30-3.28 (m, 2H), 3.06-2.97 (m, 4H), 2.20-2.10 (m, 2H), 1.89-1.80 (m, 2H), 1.47-1.39 (m, 1H), 1.31-1.24 (m, 2H), 1.13-1.04 (m, 2H). | 545.26 |

(continued)

| Example | Structure | H-NMR | Mass spectrum/ actual measurement [ M+H]$^+$ |
|---|---|---|---|
| 80 | 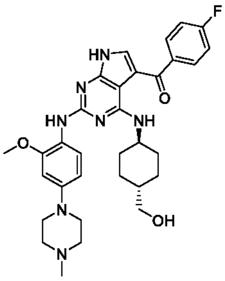 | $^1$H NMR (600 MHz, DMSO-$d_6$) δ 12.15 (s, 1H), 8.66 (d, *J* = 7.5 Hz, 1H), 8.15 (d, *J* = 8.8 Hz, 1H), 7.83 (dd, *J* = 8.6, 5.6 Hz, 2H), 7.43 (s, 1H), 7.34 (t, *J* = 8.8 Hz, 2H), 7.19 (s, 1H), 6.64 (d, *J* = 2.3 Hz, 1H), 6.45 (dd, *J* = 8.8, 2.3 Hz, 1H), 4.44 (t, *J* = 4.8 Hz, 1H), 4.04-3.89 (m, 1H), 3.86 (s, 3H), 3.29-3.27 (m, 2H), 3.17-3.05 (m, 4H), 2.28 (s, 3H), 2.18-2.08 (m, 2H), 1.89-1.76 (m, 2H), 1.48-1.37 (m, 1H), 1.32-1.22 (m, 2H), 1.14-1.02 (m, 2H). | 588.31 |
| 81 | 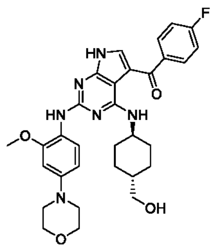 | $^1$H NMR (600 MHz, DMSO-$d_6$) δ 12.16 (s, 1H), 8.67 (d, *J* = 7.4 Hz, 1H), 8.17 (d, *J* = 8.9 Hz, 1H), 7.88-7.79 (m, 2H), 7.44 (s, 1H), 7.34 (t, *J* = 8.8 Hz, 2H), 7.20 (s, 1H), 6.65 (d, *J* = 2.4 Hz, 1H), 6.46 (dd, *J* = 8.8, 2.4 Hz, 1H), 4.44 (t, *J* = 5.3 Hz, 1H), 3.98-3.89 (m, 1H), 3.86 (s, 3H), 3.79-3.72 (m, 4H), 3.29-3.25 (m, 2H), 3.11-3.03 (m, 4H), 2.22-2.06 (m, 2H), 1.91-1.76 (m, 2H), 1.46-1.38 (m, 1H), 1.31-1.22 (m, 2H), 1.13-1.04 (m, 2H). | 575.27 |
| 82 | 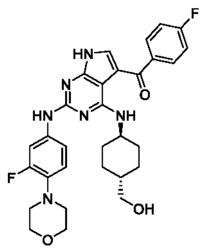 | $^1$H NMR (600 MHz, DMSO-$d_6$) δ 12.19 (s, 1H), 8.98 (s, 1H), 8.72 (d, *J* = 7.5 Hz, 1H), 8.02 (dd, *J* = 16.0, 2.3 Hz, 1H), 7.89-7.76 (m, 2H), 7.47 (d, *J* = 3.0 Hz, 1H), 7.40-7.28 (m, 3H), 6.96-6.90 (m, 1H), 4.65-4.33 (m, 1H), 4.01-3.95 (m, 1H), 3.77-3.70 (m, 4H), 3.29-3.27 (m, 2H), 2.96-2.89 (m, 4H), 2.21-2.14 (m, 2H), 1.89-1.82 (m, 2H), 1.48-1.39 (m, 1H), 1.35-1.24 (m, 2H), 1.15-1.05 (m, 2H). | 563.25 |
| 83 | 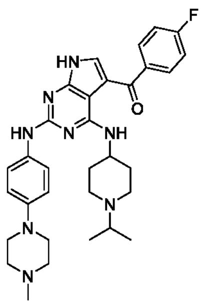 | $^1$H NMR (600 MHz, DMSO-$d_6$) δ 12.14 (s, 1H), 8.73 (s, 1H), 8.65 (s, 1H), 7.88-7.78 (m, 2H), 7.70-7.60 (m, 2H), 7.44 (s, 1H), 7.35 (t, *J* = 8.8 Hz, 2H), 6.84 (d, *J* = 9.0 Hz, 2H), 4.18-4.01 (m, 1H), 3.31-3.29 (m, 2H), 3.09-3.02 (m, 4H), 3.01-2.64 (m, 3H), 2.48-2.43 (m, 4H), 2.23 (s, 3H), 2.16-1.99 (m, 2H), 1.68-1.43 (m, 2H), 1.15-0.93 (m, 6H). | 571.33 |
| 84 | 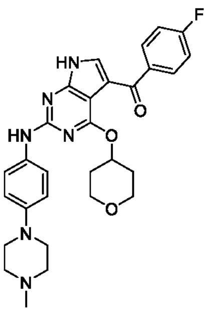 | $^1$H NMR (600 MHz, DMSO-$d_6$) δ 12.15 (s, 1H), 9.02 (s, 1H), 7.89-7.79 (m, 2H), 7.68-7.56 (m, 2H), 7.53 (s, 1H), 7.32 (t, *J* = 8.8 Hz, 2H), 6.88 (d, *J* = 9.0 Hz, 2H), 5.35-5.21 (m, 1H), 3.77-3.68 (m, 2H), 3.46-3.38 (m, 2H), 3.12-3.00 (m, 4H), 2.49-2.41 (m, 4H), 2.23 (s, 3H), 1.90-1.80 (m, 2H), 1.39-1.30 (m, 2H). | 531.25 |

(continued)

| Example | Structure | H-NMR | Mass spectrum/ actual measurement [M+H]$^+$ |
|---|---|---|---|
| 85 | 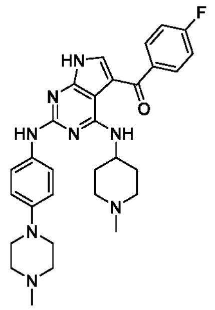 | $^1$H NMR (600 MHz, DMSO-$d_6$) δ 12.15 (s, 1H), 8.72 (d, $J$ = 6.8 Hz, 1H), 8.66 (s, 1H), 7.88-7.79 (m, 2H), 7.69-7.62 (m, 2H), 7.44 (s, 1H), 7.39-7.32 (m, 2H), 6.85 (d, $J$ = 9.1 Hz, 2H), 4.14-4.03 (m, 1H), 3.09-3.00 (m, 4H), 2.93-2.79 (m, 2H), 2.54-2.52 (m, 4H), 2.38-2.26 (m, 5H), 2.24 (s, 3H), 2.13-2.04 (m, 2H), 1.68-1.56 (m, 2H). | 543.30 |
| 86 | 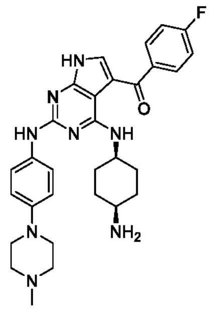 | $^1$H NMR (600 MHz, DMSO-$d_6$) δ 12.17 (s, 1H), 8.90 (d, $J$ = 6.5 Hz, 1H), 8.67 (s, 1H), 8.20-7.89 (m, 2H), 7.88-7.82 (m, 2H), 7.71-7.63 (m, 2H), 7.46 (s, 1H), 7.39-7.33 (m, 2H), 6.85 (d, $J$ = 9.1 Hz, 2H), 4.31-4.19 (m, 1H), 3.22-3.15 (m, 1H), 3.11-3.00 (m, 4H), 2.53-2.52 (m, 4H), 2.27 (s, 3H), 2.06-1.95 (m, 2H), 1.90-1.69 (m, 6H). | 543.30 |
| 87 | 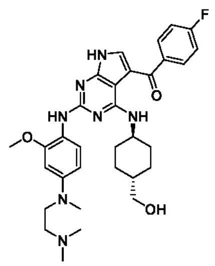 | ND | 590.32 |
| 88 | 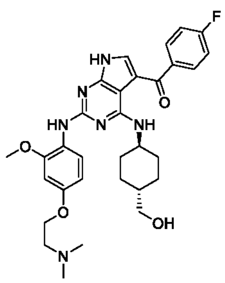 | ND | 577.29 |
| 89 | 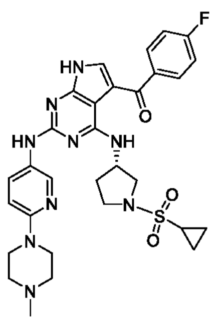 | $^1$H NMR (600 MHz, DMSO-$d_6$) δ 12.26 (s, 1H), 8.99 (s, 1H), 8.91 (d, $J$ = 6.2 Hz, 1H), 8.58 (s, 1H), 8.01 (dd, $J$ = 9.0, 2.4 Hz, 1H), 7.84 (dd, $J$ = 8.7, 5.6 Hz, 2H), 7.78 (s, 1H), 7.39 (t, $J$ = 8.8 Hz, 2H), 6.83 (d, $J$ = 9.1 Hz, 1H), 4.72-4.64 (m, 1H), 3.79-3.73 (m, 1H), 3.52-3.39 (m, 5H), 3.33-3.32 (m, 2H), 2.71-2.67 (m, 1H), 2.59-2.53 (m, 4H), 2.43-2.39 (m, 1H), 2.33 (s, 3H), 2.09-2.00 (m, 1H), 0.96-0.90 (m, 4H). | 620.25 |

(continued)

| Example | Structure | H-NMR | Mass spectrum/ actual measurement [M+H]$^+$ |
|---|---|---|---|
| 90 | 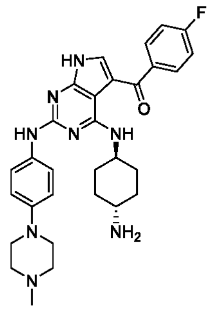 | ND | 543.30 |
| 91 | 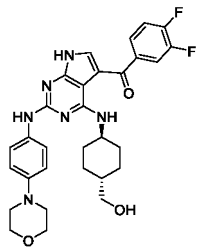 | $^1$H NMR (600 MHz, DMSO-$d_6$) δ 12.20 (s, 1H), 8.74 (s, 1H), 8.63 (s, 1H), 7.90-7.78 (m, 1H), 7.69 (d, $J$ = 7.5 Hz, 2H), 7.65-7.49 (m, 3H), 7.00-6.75 (m, 2H), 4.40 (s, 1H), 4.02-3.94 (m, 1H), 3.81-3.71 (m, 4H), 3.29 (d, $J$ = 6.2 Hz, 2H), 3.10-2.98 (m, 4H), 2.25-2.04 (m, 2H), 1.92-1.76 (m, 2H), 1.48-1.38 (m, 1H), 1.35-1.22 (m, 2H), 1.14-1.01 (m, 2H). | 563.25 |
| 92 | 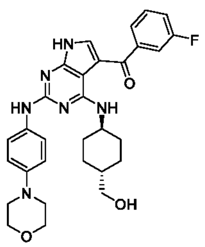 | $^1$H NMR (600 MHz, DMSO-$d_6$) δ 12.17 (s, 1H), 8.73 (s, 1H), 8.62 (s, 1H), 7.69 (d, $J$ = 8.6 Hz, 2H), 7.65-7.55 (m, 2H), 7.40-7.26 (m, 3H), 6.87 (d, $J$ = 6.7 Hz, 2H), 4.41 (s, 1H), 4.05-3.94 (m, 1H), 3.81-3.71 (m, 4H), 3.29 (d, $J$ = 6.2 Hz, 2H), 3.11-2.95 (m, 4H), 2.24-2.09 (m, 2H), 1.93-1.77 (m, 2H), 1.48-1.37 (m, 1H), 1.34-1.24 (m, 2H), 1.15-1.01 (m, 2H). | 545.26 |
| 93 | 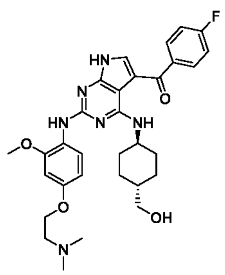 | ND | 576.67 |
| 94 | 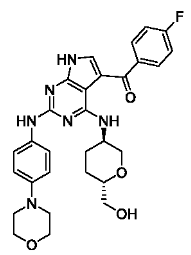 | $^1$H NMR (600 MHz, DMSO-$d_6$) δ 12.19 (s, 1H), 8.76 (s, 1H), 8.57 (d, $J$ = 7.3 Hz, 1H), 7.86-7.80 (m, 2H), 7.69 (d, $J$ = 8.9 Hz, 2H), 7.45 (s, 1H), 7.38-7.29 (m, 2H), 6.86 (d, $J$ = 8.3 Hz, 2H), 4.65 (s, 1H), 4.26-4.13 (m, 2H), 3.79-3.70 (m, 4H), 3.46-3.42 (m, 1H), 3.38-3.35 (m, 2H), 3.15-3.07 (m, 1H), 3.07-2.94 (m, 4H), 2.27-2.11 (m, 1H), 1.85-1.73 (m, 1H), 1.64-1.47 (m, 1H), 1.47-1.29 (m, 1H). | 546.60 |

(continued)

| Example | Structure | H-NMR | Mass spectrum/ actual measurement [ M+H]+ |
|---|---|---|---|
| 95 | 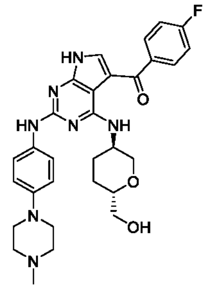 | $^1$H NMR (600 MHz, DMSO-$d_6$) δ 12.21 (s, 1H), 8.80 (s, 1H), 8.56 (d, *J* = 7.5 Hz, 1H), 7.89-7.80 (m, 2H), 7.79-7.68 (m, 2H), 7.46 (d, *J* = 2.7 Hz, 1H), 7.35 (t, *J* = 8.8 Hz, 2H), 6.89 (d, *J* = 9.1 Hz, 2H), 4.65 (s, 1H), 4.25-4.11 (m, 2H), 3.48-3.41 (m, 1H), 3.29-3.15 (m, 4H), 3.15-3.06 (m, 4H), 2.69 (s, 3H), 2.24-2.13 (m, 1H), 1.86-1.75 (m, 1H), 1.61-1.47 (m, 1H), 1.47-1.32 (m, 1H). | 559.65 |
| 96 | 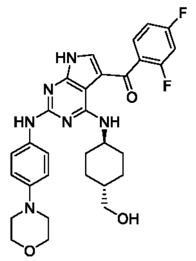 | $^1$H NMR (600 MHz, DMSO-$d_6$) δ 12.18 (s, 1H), 8.75 (s, 1H), 8.63 (s, 1H), 7.69 (d, *J* = 8.5 Hz, 2H), 7.63-7.55 (m, 2H), 7.40-7.25 (m, 3H), 6.88 (d, *J* = 5.8 Hz, 2H), 4.42 (s, 1H), 4.05-3.94 (m, 1H), 3.78-3.72 (m, 4H), 3.29 (d, *J* = 6.2 Hz, 2H), 3.10-2.98 (m, 4H), 2.24-2.09 (m, 2H), 1.92-1.77 (m, 2H), 1.49-1.38 (m, 1H), 1.36-1.25 (m, 2H), 1.14-1.04 (m, 2H). | 562.62 |
| 97 | 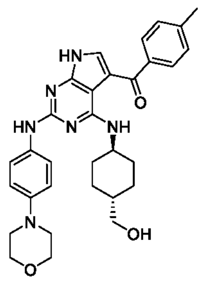 | $^1$H NMR (600 MHz, DMSO-$d_6$) δ 12.15 (s, 1H), 9.39-8.51 (m, 2H), 7.78-7.60 (m, 4H), 7.43 (s, 1H), 7.34 (d, *J* = 7.9 Hz, 2H), 6.92 (d, *J* = 6.1 Hz, 2H), 4.44 (s, 1H), 4.03-3.93 (m, 1H), 3.80-3.73 (m, 4H), 3.29 (d, *J* = 6.2 Hz, 2H), 3.12-3.02 (m, 4H), 2.41 (s, 3H), 2.20-2.09 (m, 2H), 1.89-1.80 (m, 2H), 1.51-1.39 (m, 1H), 1.35-1.25 (m, 2H), 1.13-1.03 (m, 2H). | 541.32 |
| 98 | 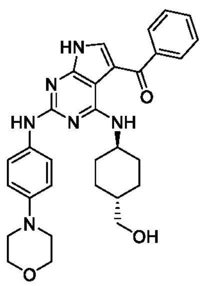 | $^1$H NMR (600 MHz, DMSO-$d_6$) δ 12.20 (s, 1H), 9.28-8.55 (m, 2H), 7.79-7.74 (m, 2H), 7.69 (d, *J* = 7.9 Hz, 2H), 7.65-7.60 (m, 1H), 7.54 (t, *J* = 7.6 Hz, 2H), 7.43 (s, 1H), 6.93 (d, *J* = 6.0 Hz, 2H), 4.24 (s, 1H), 4.01-3.95 (m, 1H), 3.79-3.74 (m, 4H), 3.29 (d, *J* = 6.2 Hz, 2H), 3.16-3.02 (m, 4H), 2.24-2.09 (m, 2H), 1.93-1.77 (m, 2H), 1.49-1.39 (m, 1H), 1.36-1.26 (m, 2H), 1.14-1.04 (m, 2H). | 527.33 |
| 99 | 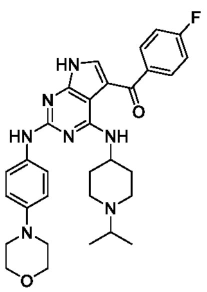 | $^1$H NMR (600 MHz, DMSO-$d_6$) δ 12.28 (s, 1H), 9.19 (s, 1H), 8.81 (d, *J* = 8.6 Hz, 1H), 7.85-7.82 (m, 2H), 7.66 (d, *J* = 8.8 Hz, 2H), 7.51 (s, 1H), 7.38-7.35 (m, 2H), 6.94 (d, *J* = 8.9 Hz, 2H), 4.25-4.16 (m, 1H), 3.77-3.75 (m, 4H), 3.55-3.52 (m, 2H), 3.27-3.18 (m, 1H), 3.18-3.10 (m, 2H), 3.08-3.05 (m, 4H), 2.47-2.40 (m, 2H), 1.87-1.72 (m, 2H), 1.30 (d, *J* = 6.6 Hz, 6H). | 558.33 |

(continued)

| Example | Structure | H-NMR | Mass spectrum/ actua l measurement [ M+H]+ |
|---|---|---|---|
| 100 | 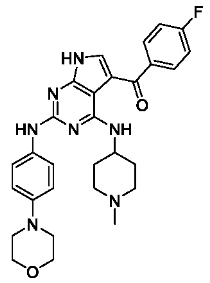 | ND | 530.29 |
| 101 | 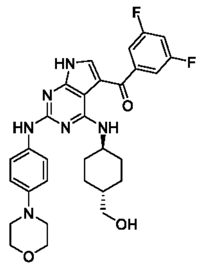 | $^1$H NMR (600 MHz, DMSO-$d_6$) δ 12.20 (s, 1H), 8.68 (s, 1H), 8.54 (d, $J$ = 7.5 Hz, 1H), 7.68 (d, $J$ = 8.8 Hz, 2H), 7.56 (s, 1H), 7.50 (t, $J$ = 9.2 Hz, 1H), 7.44 (d, $J$ = 5.6 Hz, 2H), 6.85 (d, $J$ = 8.9 Hz, 2H), 4.44 (t, $J$ = 5.2 Hz, 1H), 4.07-3.94 (m, 1H), 3.78-3.69 (m, 4H), 3.30-3.27 (m, 2H), 3.08-2.96 (m, 4H), 2.21-2.09 (m, 2H), 1.89-1.78 (m, 2H), 1.49-1.37 (m, 1H), 1.33-1.21 (m, 2H), 1.14-1.03 (m, 2H). | 563.30 |
| 102 | 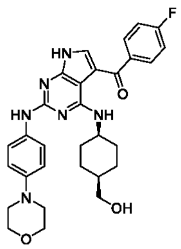 | $^1$H NMR (600 MHz, DMSO-$d_6$) δ 12.17 (s, 1H), 9.06 (s, 1H), 8.76 (s, 1H), 7.92-7.78 (m, 2H), 7.69 (d, $J$ = 7.9 Hz, 2H), 7.45 (s, 1H), 7.35 (t, $J$ = 8.7 Hz, 2H), 6.90 (d, $J$ = 6.2 Hz, 2H), 4.40 (s, 1H), 3.81-3.71 (m, 4H), 3.30 (d, $J$ = 6.6 Hz, 2H), 3.12-2.97 (m, 4H), 1.89-1.79 (m, 2H), 1.73-1.62 (m, 4H), 1.59-1.49 (m, 1H), 1.44-1.33 (m, 2H). | 545.32 |
| 103 | 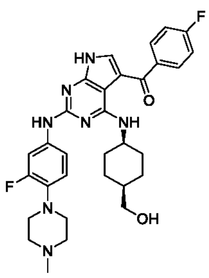 | $^1$H NMR (600 MHz, DMSO-$d_6$) δ 12.26 (s, 1H), 9.11 (s, 1H), 9.08 (d, $J$ = 7.4 Hz, 1H), 8.05-7.96 (m, 1H), 7.90-7.80 (m, 2H), 7.51 (s, 1H), 7.46-7.32 (m, 3H), 7.01 (t, $J$ = 9.4 Hz, 1H), 4.44-4.36 (m, 1H), 3.54-3.49 (m, 1H), 3.44-3.37 (m, 2H), 3.31 (d, $J$ = 6.7 Hz, 2H), 3.26-3.19 (m, 2H), 3.03-2.95 (m, 2H), 2.88 (s, 3H), 1.91-1.81 (m, 2H), 1.74-1.64 (m, 4H), 1.57-1.49 (m, 1H), 1.46-1.34 (m, 2H). | 576.33 |
| 104 | 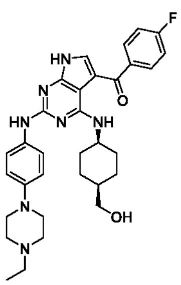 | $^1$H NMR (600 MHz, DMSO-$d_6$) δ 12.11 (s, 1H), 8.96 (d, $J$ = 7.5 Hz, 1H), 8.65 (s, 1H), 7.90-7.77 (m, 2H), 7.68 (d, $J$ = 8.5 Hz, 2H), 7.43 (s, 1H), 7.35 (t, $J$ = 8.7 Hz, 2H), 6.86 (d, $J$ = 8.4 Hz, 2H), 4.45 (t, $J$ = 5.1 Hz, 1H), 4.43-4.36 (m, 1H), 3.31-3.23 (m, 4H), 3.19-2.94 (m, 4H), 2.84-2.54 (m, 4H), 1.90-1.80 (m, 2H), 1.72-1.61 (m, 4H), 1.58-1.48 (m, 1H), 1.44-1.34 (m, 2H), 1.15-1.01 (m, 3H). | 572.35 |

(continued)

| Example | Structure | H-NMR | Mass spectrum/ actua l measurement [ M+H]$^+$ |
|---|---|---|---|
| 105 | 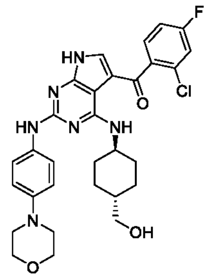 | $^1$H NMR (600 MHz, DMSO-$d_6$) δ 12.35 (s, 1H), 8.84 (s, 1H), 7.68-7.62 (m, 2H), 7.28-7.20 (m, 1H), 6.93-6.77 (m, 4H), 5.54-5.38 (m, 1H), 4.05-3.89 (m, 1H), 3.78-3.70 (m, 4H), 3.30-3.26 (m, 2H), 3.05-2.98 (m, 4H), 2.17-2.07 (m, 2H), 1.88-1.78 (m, 2H), 1.47-1.38 (m, 1H), 1.33-1.20 (m, 2H), 1.14-1.02 (m, 2H). | 579.22815 |
| 106 | 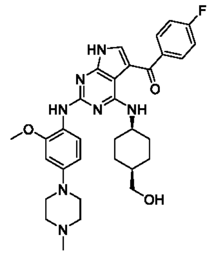 | $^1$H NMR (600 MHz, DMSO-$d_6$) δ 12.17 (s, 1H), 8.99 (d, *J* = 7.2 Hz, 1H), 8.19 (d, *J* = 8.5 Hz, 1H), 7.93-7.77 (m, 2H), 7.45 (s, 1H), 7.35 (t, *J* = 8.6 Hz, 2H), 7.19 (s, 1H), 6.64 (s, 1H), 6.48 (d, *J* = 8.5 Hz, 1H), 4.56-4.40 (m, 1H), 4.41-4.30 (m, 1H), 3.86 (s, 3H), 3.30-3.28 (m, 2H), 3.20-3.02 (m, 4H), 2.56-2.52 (m, 4H), 2.28 (s, 3H), 1.89-1.80 (m, 2H), 1.72-1.60 (m, 4H), 1.58-1.47 (m, 1H), 1.43-1.32 (m, 2H). | 588.30927 |
| 107 | 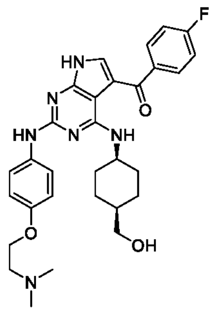 | $^1$H NMR (600 MHz, DMSO-$d_6$) δ 12.13 (s, 1H), 8.98 (d, *J* = 7.6 Hz, 1H), 8.78 (s, 1H), 7.84 (dd, *J* = 8.3, 5.7 Hz, 2H), 7.76 (d, *J* = 9.0 Hz, 2H), 7.45 (s, 1H), 7.35 (t, *J* = 8.7 Hz, 2H), 6.91 (d, *J* = 8.9 Hz, 2H), 4.46 (t, *J* = 5.2 Hz, 1H), 4.42-4.35 (m, 1H), 4.29-4.18 (m, 2H), 3.40-3.37 (m, 2H), 3.30-3.28 (m, 2H), 2.79 (s, 6H), 1.90-1.80 (m, 2H), 1.72-1.60 (m, 4H), 1.58-1.50 (m, 1H), 1.45-1.33 (m, 2H). | 547.28 |
| 108 | 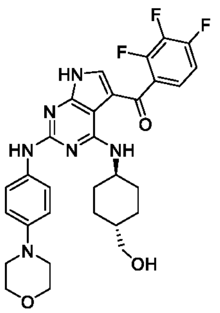 | $^1$H NMR (600 MHz, DMSO-$d_6$) δ 12.25 (s, 1H), 8.70 (s, 1H), 8.43 (d, *J* = 7.5 Hz, 1H), 7.68 (dd, *J* = 9.1, 3.4 Hz, 2H), 7.54 (s, 1H), 7.51-7.40 (m, 2H), 6.85 (d, *J* = 9.1 Hz, 2H), 4.45 (t, *J* = 5.3 Hz, 1H), 4.02-3.96 (m, 1H), 3.76-3.72 (m, 4H), 3.29 (t, *J* = 5.7 Hz, 2H), 3.03-2.99 (m, 4H), 2.20-2.11 (m, 2H), 1.88-1.81 (m, 2H), 1.47-1.39 (m, 1H), 1.32-1.23 (m, 2H), 1.13-1.05 (m, 2H). | 581.30 |
| 109 | 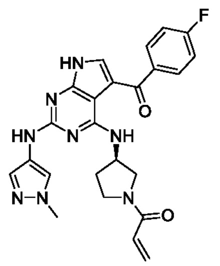 | $^1$H NMR (600 MHz, DMSO-$d_6$) δ 12.18 (s, 1H), 8.93 (d, *J* = 9.8 Hz, 1H), 8.86 (s, 1H), 7.93-7.71 (m, 3H), 7.52 (s, 1H), 7.48 (d, *J* = 2.8 Hz, 1H), 7.35 (t, *J* = 8.8 Hz, 2H), 6.62 (ddd, *J* = 42.2, 16.7, 10.3 Hz, 1H), 6.15 (ddd, *J* = 16.8, 5.8, 2.4 Hz, 1H), 5.68 (ddd, *J* = 17.9, 10.3, 2.4 Hz, 1H), 4.82-4.57 (m, 1H), 4.08-3.76 (m, 5H), 3.68-3.45 (m, 2H), 2.44-2.26 (m, 1H), 2.17-1.93 (m, 1H). | 475.22 |

(continued)

| Example | Structure | H-NMR | Mass spectrum/ actual measurement [ M+H]+ |
|---|---|---|---|
| 110 | 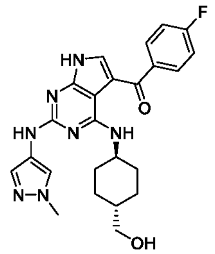 | $^1$H NMR (600 MHz, DMSO-$d_6$) δ 12.05 (s, 1H), 8.72 (s, 1H), 8.67 (d, *J* = 4.6 Hz, 1H), 7.93-7.76 (m, 3H), 7.51 (s, 1H), 7.41 (d, *J* = 2.7 Hz, 1H), 7.37-7.32 (m, 2H), 4.72-4.24 (m, 1H), 4.01-3.92 (m, 1H), 3.79 (s, 3H), 3.30-3.26 (m, 2H), 2.25-2.08 (m, 2H), 1.91-1.79 (m, 2H), 1.50-1.38 (m, 1H), 1.33-1.22 (m, 2H), 1.17-1.04 (m, 2H). | 464.22 |
| 111 | 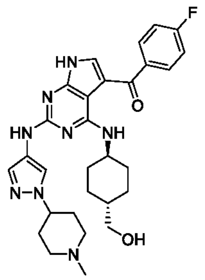 | ND | 547.29 |
| 112 | 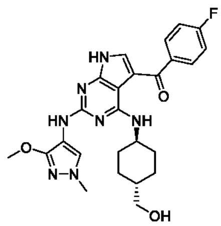 | $^1$H NMR (600 MHz, DMSO-$d_6$) δ 12.10 (s, 1H), 8.59 (d, *J* = 6.3 Hz, 1H), 7.85-7.78 (m, 2H), 7.66 (s, 1H), 7.43 (s, 1H), 7.37 (s, 1H), 7.33 (t, *J* = 8.8 Hz, 2H), 4.43 (s, 1H), 3.99-3.84 (m, 1H), 3.79 (s, 3H), 3.66 (s, 3H), 3.29-3.24 (m, 2H), 2.20-2.05 (m, 2H), 1.87-1.78 (m, 2H), 1.48-1.35 (m, 1H), 1.29-1.21 (m, 2H), 1.11-1.00 (m, 2H). | 494.26 |
| 113 | 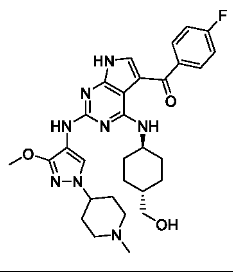 | ND | 577.36 |
| 114 | 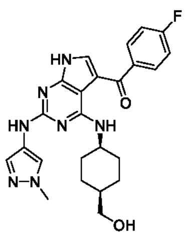 | $^1$H NMR (600 MHz, DMSO-$d_6$) δ 12.19 (s, 1H), 9.21 (s, 1H), 8.93 (s, 1H), 7.95-7.77 (m, 3H), 7.53 (s, 1H), 7.47 (s, 1H), 7.36 (t, *J* = 8.6 Hz, 2H), 4.43-4.33 (m, 1H), 3.80 (s, 3H), 3.30 (d, *J* = 6.4 Hz, 2H), 1.93-1.81 (m, 2H), 1.74-1.61 (m, 4H), 1.59-1.49 (m, 1H), 1.44-1.30 (m, 2H). | 464.22 |

(continued)

| Example | Structure | H-NMR | Mass spectrum/ actual measurement [ M+H]$^+$ |
|---|---|---|---|
| 115 | 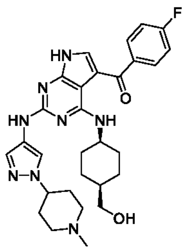 | ND | 547.28259 |
| 116 | 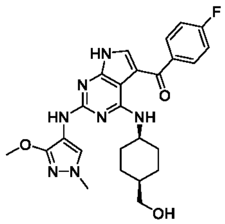 | $^1$H NMR (600 MHz, DMSO-$d_6$) δ 12.11 (s, 1H), 8.90 (d, *J* = 7.6 Hz, 1H), 7.82 (dd, *J* = 8.6, 5.6 Hz, 2H), 7.64 (s, 1H), 7.43-7.29 (m, 4H), 4.43 (t, *J* = 5.3 Hz, 1H), 4.39-4.27 (m, 1H), 3.78 (s, 3H), 3.66 (s, 3H), 3.30-3.24 (m, 2H), 1.85-1.77 (m, 2H), 1.70-1.57 (m, 4H), 1.56-1.46 (m, 1H), 1.42-1.31 (m, 2H). | 494.23 |

**Example 117 *In-vitro* enzyme experiment** 1

The compound of the present invention had a good inhibitory activity on an EGFR kinase, and the IC50 values were all less than 10 μM. The specific experiment was as follows:

1. Preparation of 1x concentration kinase buffer

1 volume of 5 times concentration of an enzyme buffer diluted with 4 volumes of distilled water; 5 mM magnesium chloride; 1 mM DTT; and 1 mM $MnCl_2$.

2. Test method of activity of compound

a) a compound diluent was transferred to each well of an assay plate (784075, Greiner) using Echo 550;
b) the assay plate was sealed and centrifuged at 1,000 g for 1 min;
c) 2X EGFR (D770-N771 ins NPG T790M) was prepared in a 1x kinase buffer;
d) 5 μl of the 2X EGFR (D770-N771 ins NPG T790M) was added to a 384-well assay plate (784075, Greiner);
e) the assay plate was centrifuged at 1,000 g for 30 s and incubated at room temperature for 10 min;
f) 2x TK-substrate-biotin (2 μM) and ATP were added to the 1x kinase buffer and mixed;
g) the reaction was started after adding 5 μl of the TK-substrate-biotin (2 μM) and ATP mixture;
h) the assay plate was centrifuged at 1,000 g for 30 s; and the assay plate was sealed and incubated at room temperature for 40 min;
i) 4X Sa-XI, 665 was prepared in an HTRF detection buffer;
j) 5 μl of the Sa-XL 665 and 5 μl of a TK-antibody-cryptate were added to each well of the assay plate;
k) the assay plate was centrifuged at 1,000 g for 30 s and incubated at room temperature for 1 h; and
l) fluorescence signals at 615 nm (cryptate) and 665 nm (XL665) were read on an Envision 2104 reader.

3. Data analysis

3.1 Calculation of ratio (665/615 nm) of each well

3.2 Calculation of inhibition rate

$$\text{Inhibition rate} = 100\text{-(signalmpd-SignalAve_PC)/(SignalAve_VC-SignalAve_PC)} \times 100.$$

3.3 Calculation of $IC_{50}$ value of compound and drawing dose-effect curve of compound

$IC_{50}$ values were calculated using GraphPad 6.0 by applying a non-linear regression curve (dose-effect relationship-variable slope) of the logarithm of the compound concentration versus the inhibition rate.

$$Y = \text{lowest value+(highest value-lowest value)/(1+10\^{}((LogIC}_{50}\text{-X)} \times \text{slope))}$$

X: logarithm of inhibitor concentration; and Y: inhibition rate.

3.4 Double test report

3.4.1 After one tester completed a report, another tester again checked the report to ensure that data analysis was correct.
3.4.2 The data was exported from the software and manually analyzed.
3.4.2.1 The rate was converted into the inhibition rate. All the $IC_{50}$ values were first calculated with the inhibition rate using Prism GraphPad 6.0.
3.4.2.2 Then the $IC_{50}$ value was recalculated using the ratio to check the accuracy of the analysis.
3.4.3 The number of all the compounds was ensured to be correct.

The activity of the compound of the present invention on a D770-N771 ins NPG kinase was determined by using the method.
The results of activity of some compounds were shown in Table 1.
Table 1 Some compounds of the present invention on activity of D770-N771 ins NPG/T790M kinase and D770-N771 ins NPG kinase and BaF3-EGFR D770-N771 ins NPG cells

| **Example No.** | **D770-N771 ins NPG/T790M kinase** | **D770-N771 ins NPG kinase** | **KC-1362 BaF3-EGFR D770-N771 ins NPG cells** |
|---|---|---|---|
| | **$IC_{50}$ (nM)** | **$IC_{50}$ (nM)** | **$IC_{50}$ (μM)** |
| **60** | 8.80 | 2.82 | 0.070 |
| **114** | 18.21 | 4.12 | 0.16 |
| **115** | 8.16 | 2.46 | 0.56 |
| **71** | 9.27 | 4.54 | 0.070 |
| **104** | 11.39 | 2.72 | 0.16 |
| **18** | ND | 28.20 | 0.15 |
| **64** | 93.64 | 24.25 | 0.20 |
| **109** | 7.27 | 3.00 | 0.12 |
| **26** | 6.31 | 3.52 | 0.15 |

(continued)

| Example No. | D770-N771 ins NPG/T790M kinase | D770-N771 ins NPG kinase | KC-1362 BaF3-EGFR D770-N771 ins NPG cells |
|---|---|---|---|
| | $IC_{50}$ (nM) | $IC_{50}$ (nM) | $IC_{50}$ (μM) |
| **70** | ND | 6.55 | 0.054 |
| **42** | ND | 4.44 | 0.035 |
| ND indicated not detected | | | |

The compound of the present invention had significant inhibitory activity and anti-proliferative activity on EGFR D770-N771 ins NPG and EGFR D770-N771 ins NPG/T790M kinases and BaF3-EGFR D770-N771 ins NPG cells carrying an insertion mutation. It was suggested that the compound of the present invention can inhibit EGFR D770-N771 ins NPG mutation and EGFR D770-N771 ins NPG/T790M mutation.

**Example 118 *In-vitro* enzyme experiment 2**

The preparation of the kinase buffer, the test method of the activity of the compound, and the data analysis referred to the relevant definitions in example 117, wherein step c) of the test method of the activity of the compound was replaced by preparing 2X EGFR Del19 T790M C797S in the 1x kinase buffer.

The results of activity of some compounds were shown in Table 2.

Table 2 Results of enzyme inhibition $IC_{50}$ (nM)

| Target compound correspondin g to example No. | EGFR-De119/T790M/C797 S | Target compound correspondin g to example No. | EGFR-Del19/T790M/C797 S |
|---|---|---|---|
| **41** | C | **110** | B |
| **43** | C | **84** | C |
| **49** | C | **85** | C |
| **50** | C | **86** | A |
| **55** | B | **Osimertinib** | C |
| **56** | B | **94** | B |
| **57** | C | **95** | B |
| **58** | C | **96** | B |
| **59** | C | **112** | C |
| **60** | A | **18** | B |
| **63** | C | **98** | C |
| **64** | C | **102** | B |
| **67** | C | **103** | B |
| **68** | C | **104** | A |
| **70** | B | **114** | A |
| **71** | B | **106** | B |
| **10** | B | **115** | A |
| **11** | C | **107** | B |
| **73** | C | **116** | B |
| **78** | B | **19** | B |

(continued)

| Target compound correspondin g to example No. | EGFR-De119/T790M/C797 S | Target compound correspondin g to example No. | EGFR-Del19/T790M/C797 S |
|---|---|---|---|
| **80** | C | **20** | B |
| **82** | C | **108** | C |
| A: < 10 nM; B: 10-100 nM; and C: 100-600 nM. | | | |

The compound of the present invention had a very good inhibition effect on an EGFR-Del19/T790M/C797S kinase.

**Example 119 Cell proliferation inhibition experiment**

1. Experimental grouping

Experimental group (As): cell+medium+drug+cck-8; control group ($A_0$): cell+medium+cck-8, and no drug; and blank group (Ac): medium+cck-8, and no cell and no drug.

2. Experimental cell lines

Human lung cancer cells:
HCC827 $^{EGFR-De119}$;
PC-9 $^{EGFR-Del19}$;
H1975 $^{EGFR-E858R/T790M}$;
A549 $^{EGFR-WT}$;
KC-0122:BaF3 $^{EGER-E858R/T790M/C797S}$;
KC-0116:BaF3 $^{EGFR-Del19/T790M/C797S}$;
KC-1474:PC-9 $^{EGFR-Del19/T790M/C797S}$; and
KC-0178:NCI-1975 $^{EGFR-L858R/T790M/C797S}$.

3. Cell culture

3.1. Cell recovery

Cryopreservation tubes of cells were taken out from liquid nitrogen, immediately put into warm water at 37°C, and quickly shaken until cryopreserved liquid was completely dissolved; the cell cryopreserved liquid was transferred into a centrifuge tube, about 3 ml of a culture solution was added, an obtained mixture was gently pipetted, uniformly mixed, and centrifuged at 1,000 r/min for 5 min; and a supernatant was discarded, and an appropriate amount of the culture solution was added, and the cells were transferred to a culture flask for culture.

3.2 Cell passage

When the cell covering rate in the culture flask reached 80-90%, the original culture solution was sucked and a proper amount of pancreatin was added; after the cells became round, the pancreatin was discarded, an appropriate amount of the culture solution was added, and an obtained mixture was pipetted and uniformly mixed; and an appropriate amount of a cell suspension was discarded, an equal amount of the culture solution was added, and the cells were placed into an incubator for continuous culture.

4. Effect of drug on cell line

The cells in the logarithmic growth phase were taken, the density of the cells was adjusted to be 2-3 $\times$ $10_4$ cells/ml by using the culture solution, the cells were inoculated into a 96-well plate with 100 μl per well, and 200 μl of normal saline per well was added on the outermost periphery. After the cells were cultured in the incubator for 24 h,

different concentrations of compounds were added, 6 replicate wells were set per concentration, and 100 μl of the compounds was administered per well. The cells were cultured for 72 h and then a CCK-8 solution was added at 15 μl/well. After the cells were continuously cultured for 2 h, the absorbance value was detected at the wavelength of 450 nm using a microplate reader. Inhibition rate% = [(A0-AS)/(A0-AC)] × 100%. The $IC_{50}$ values of the compounds on the cells were calculated using an SPSS software.

5. Test results

Table 3 Results of cell proliferation inhibition experiment $IC_{50}$ (μM)

| Target compound corresponding to example No. | KC-1474 | KC-0178 | HCC827 | PC-9 | H1975 | A549 |
|---|---|---|---|---|---|---|
| 40 | * | ND | ** | ** | ND | ** |
| 42 | * | *** | ** | * | ** | *** |
| 43 | ND | ND | ** | ** | ** | ND |
| 46 | ND | ND | ** | * | * | ND |
| 109 | ND | ND | ** | * | ** | ** |
| 55 | * | ** | ND | * | ** | ** |
| 56 | * | *** | ND | * | ** | ** |
| 60 | * | *** | ND | * | ** | ** |
| 78 | * | *** | ND | * | ** | ND |
| 79 | ND | * | * | ** | ** | ** |
| 110 | ** | * | * | * | ND | ** |
| 86 | * | *** | ND | * | ** | ND |
| 92 | ND | ND | * | ** | * | ND |
| 95 | ** | * | ND | ND | ND | ND |
| 97 | ** | ** | ND | ND | ND | ND |
| 102 | ** | ** | ND | * | ** | ** |
| 103 | * | *** | ND | * | ** | ** |
| 104 | * | ** | ND | * | * | ND |
| 114 | * | ** | ND | * | * | ** |
| 106 | ** | *** | ND | ND | ND | ND |
| 115 | ** | *** | ND | ** | ** | *** |
| 107 | ** | *** | ND | * | ** | ND |
| 116 | ** | *** | ND | * | ** | *** |
| 19 | ** | *** | ND | * | ** | ** |
| 20 | ** | *** | ND | * | ** | ** |
| Avitinib | ** | *** | ND | ND | ND | ND |
| Osimertinib | *** | *** | ND | ND | ND | ND |
| *: < 0.1 μM; **: 0.1-1.0 μM; ***: 1.0-2.0 μM; and ND represented not detected. | | | | | | |

The compound of the present invention has a very good inhibitory effect on the cell proliferation of $HCC827^{EGFR\text{-}Del19}$ and $PC\text{-}9^{EGFR\text{-}Del19}$ mutant cell lines, and an $H1975^{EGFR\text{-}L858R/T790M}$ double mutant cell line.

The compound of the present invention has a very good inhibitory effect on the cell proliferation of a KC-1474:PC-9$^{EGFR-Del19/T790M/C797S}$ three-mutation cell line and a KC-0178:NCI-1975$^{EGFR-L858R/T790M/C797S}$ three-mutation cell line.

Table 4 Results of cell proliferation inhibition experiment $IC_{50}$ ($\mu$M)

| Target compound corresponding to example No. | KC-0122 | KC-0116 | Target compound corresponding to example No. | KC-0122 | KC-0116 |
|---|---|---|---|---|---|
| 22 | *** | ** | 72 | ** | ** |
| 23 | ** | ** | 10 | ** | ** |
| 24 | ** | ** | 74 | ** | ** |
| 25 | ** | ** | 75 | ** | ** |
| 27 | ** | ** | 12 | ** | ** |
| 28 | *** | ND | 13 | * | * |
| 30 | ** | * | 76 | ** | ** |
| 31 | ** | ND | 77 | ** | ** |
| 32 | ND | ** | 14 | ** | ** |
| 33 | ND | ** | 78 | * | ** |
| 34 | ND | ** | 79 | * | * |
| 35 | ND | ** | 80 | ND | * |
| 36 | ND | ** | 81 | ND | * |
| 37 | ND | ** | 82 | ND | * |
| 38 | ND | ** | 110 | ** | * |
| 39 | ND | ** | 86 | ** | * |
| 40 | * | * | 15 | ND | ** |
| 42 | * | * | 16 | ND | ** |
| 43 | ** | ** | 89 | ND | * |
| 44 | ** | ** | 90 | ND | ** |
| 45 | ** | ** | 111 | ND | ** |
| 46 | ** | ** | 91 | * | * |
| 109 | *** | *** | 92 | * | * |
| 47 | ND | *** | 95 | ** | ** |
| 48 | ** | ** | 96 | ND | * |
| 51 | ** | ** | 113 | ND | ** |
| 52 | ** | ** | 97 | * | * |
| 53 | ** | ** | 98 | * | * |
| 54 | ND | ** | 99 | ND | * |
| 55 | * | * | 100 | ND | * |
| 56 | * | * | 101 | * | * |
| 59 | * | * | 102 | * | ** |
| 60 | * | * | 103 | ** | ** |
| 2 | *** | ** | 104 | * | * |
| 62 | ** | ** | 14 | * | ** |

(continued)

| Target compound corresponding to example No. | KC-0122 | KC-0116 | Target compound corresponding to example No. | KC-0122 | KC-0116 |
|---|---|---|---|---|---|
| 64 | * | * | 105 | ND | * |
| 65 | ** | ** | 106 | * | ** |
| 66 | ** | ** | 115 | ** | ** |
| 69 | ** | ** | 107 | ** | ** |
| 5 | ** | ** | 116 | ** | ** |
| 70 | * | * | 19 | ** | ** |
| 71 | ** | * | 20 | ** | ** |
| 6 | ** | ** | Brigatinib | ** | ** |
| 7 | ** | ** | Avitinib | *** | ** |
| 8 | ** | ** | Osimertinib | *** | *** |
| 9 | ** | ** | | | |
| *: < 0.1 μM; **: 0.1-1.0 μM; ***: 1.0-2.0 μM; and ND represented not detected. | | | | | |

The compound of the present invention has a very good inhibitory effect on the cell proliferation of a KC-0122:Ba/F3$^{EGFR-L858R/T790M/C797S}$ three-mutation cell line and a KC-0116:Ba/F3$^{EGFR-Del19/T790M/C797S}$ three-mutation cell line.

Table 5 Results of cell proliferation inhibition experiment $IC_{50}$ (μM)

| **Target compound correspondin g to example No.** | **A54 9** | **KC-012 2** | **KC-011 6** | **Target compound correspondin g to example No.** | **A54 9** | **KC-012 2** | **KC-011 6** |
|---|---|---|---|---|---|---|---|
| **3** | 2.67 | 0.14 | 0.15 | **91** | 0.45 | 0.016 | 0.025 |
| **4** | 6.53 | 0.45 | 0.31 | **92** | 0.07 0 | 0.0063 | 0.0075 |
| **1** | 7.00 | 0.36 | 0.65 | **99** | 0.76 | ND | 0.076 |
| **21** | 0.61 | 0.27 | 0.15 | **101** | 0.46 | 0.04 | 0.043 |
| **26** | 0.52 | 0.68 | 0.17 | **105** | 0.29 | ND | 0.085 |
| **40** | 0.23 | 0.051 | 0.060 | **Brigatini** | 6.13 | 0.49 | 0.43 |
| **61** | 0.28 | 0.054 | 0.040 | **Avitinib** | 1.06 | 1.50 | 0.97 |
| **60** | 0.23 | 0.088 | 0.038 | **Osimertinib** | 3.01 | 2.95 | 1.74 |
| **81** | 0.92 | ND | 0.077 | | | | |

The compound of the present invention has a very good inhibitory effect on the cell proliferation of a KC-0122:Ba/F3$^{EGFR-L858R/T790M/C797S}$ three-mutation cell line and a KC-0116:Ba/F3$^{EGFR-Del19/T790M/C797S}$ three-mutation cell line, and a weaker inhibitory effect on an EGFR wild-type cell line A549 with good selectivity.

**Example 120 *In-vivo* drug efficacy study**

1 Materials

1.1 Animals

Female SPF NPG mice weighed 19-22 g were used. The experimental animals were provided by Beijing

Vitalstar Biotechnology Co., Ltd. with the experimental animal production license number SCXK (Jing) 2019-0002 and the experimental animal quality certification number of No.110341211100087928.

1.2 Main reagents and instruments

Name of reagent:

RPMI1640, manufacturer: gibco, and lot number: 2120614; and
FBS, manufacturer: gibco, and lot number: 2094468CP;

Name of instrument:

carbon dioxide incubator, instrument manufacturer: Thermo, and model: BB15;
clean bench, instrument manufacturer: Suzhou Antai Airtech Co., Ltd., and model: SW-CJ-2FD;
centrifuge, instrument manufacturer: Shanghai Anting Scientific Instrument Factory, and model: TDL-5A;
inverted microscope, instrument manufacturer: OLYMPUS, and model: CKX31;
balance, instrument manufacturer: Mettler, and model: PL2002; and
vernier caliper, instrument manufacturer: SATA, and model: Shida 91512.

2 Experimental procedures

2.1 Cell culture and inoculation

KC-1474 cells used in the experiment were cultured in a 5% $CO_2$ 37°C incubator with an RPMI1640 medium supplemented with 10% FBS and 0.5 $\mu$g/mL of puromycin. Before the cells were continuously cultured for ten passages, the KC-1474 cells were adjusted to a concentration of $1\times10^8$/mL, and mixed with Matrigel at a volume ratio of 1:1, and the mixture was inoculated subcutaneously into the right lateral thorax of the NPG mice at 0.1 mL/mouse with $5\times10^6$ cells per mouse.

2.2 Grouping and administration

When the mean tumor volume of the mice reached about 100-130mm$^3$, according to the tumor volume and weight, all the mice were randomly divided into six group respectively: a model group (V), an example 60 compound oral administration group (po), an example 60 compound intravenous injection low-dose group (L), and an example 60 compound intravenous injection medium-dose group (M). The mice in all the groups were administrated once daily. The administration started on the day of the grouping and continued for 39 days. The mice in the model group were intravenously injected with the same volume of a solvent. The solvent was prepared by adding 2 g of polyethylene glycol-15 hydroxystearate (HS15) into 4 mL of dimethyl sulfoxide (DMSO), then adding 40 mL of purified water, and filtering an obtained mixture by using a 0.22-$\mu$m sterilizing filter. At the end of the experiment or the humane endpoint, the animals were euthanized using sodium pentobarbital for anesthesia.

Specific grouping and administration regimens were shown in the following table:

| Code of group | Test substance | Administration dose (mg/kg) | Administration frequency | Administration route |
|---|---|---|---|---|
| V | Solvent | - | qd | iv |
| po | Example 60 | 20 | qd | po |
| L | Example 60 | 3 | qd | iv |
| M | Example 60 | 10 | qd | iv |

2.3 Detection indicators

2.3.1 Tumor volume and tumor volume inhibition rate ($TGI_{TV}$)

Tumor volume was measured once during the grouping, once per week after the grouping using a vernier caliper, and before the euthanasia. A method for measuring the tumor volume was measuring the long diameter and the short diameter of a tumor using the vernier caliper.

The tumor volume was calculated by the formula: tumor volume = 0.5×long diameter×short diameter$^2$.

The tumor volume inhibition rate was calculated by the formula: $TGI_{TV}$ (%) = [1-(Ti-T0)/(Vi-V0)]×100%

(Ti: mean tumor volume of mice in the treatment groups on day i of the administration and T0: mean tumor volume of the mice in the treatment group on day 0 of the administration; and Vi: mean tumor volume of the mice in the model group on day i of the administration and V0: mean tumor volume of the mice in the model group on day 0 of the administration).

2.3.2 Tumor weight and tumor weight inhibition rate ($TGI_{TW}$)

At the end of the experiment, the tumor tissue was stripped after the animals were euthanized, the tumor weight was weighed, and the tumor weight inhibition rate was calculated according to the following formula:

$$TGI_{TW}\ (\%) = (W_{model\ group}-W_{treatment\ group})/W_{model\ group}\times 100\%,$$

wherein the W means tumor weight.

3 Results and conclusions

Original data was analyzed and the results were expressed as mean±standard deviation (mean±SD). At the same time, the tumor volume and the tumor weight were subjected to statistical analysis. AP < 0.05 was considered to be a significant difference.

All the animals were well mobile and fed during the administration and observation with no diarrhea, vomiting, or rash. As shown in Fig. 1, at the end of the experiment, the mean tumor volume of the mice in the model group was 1,082±225 mm$^3$, and the tumor volume inhibition rates of the mice in the po, L, and M groups were 28%, 31%, and 57%, respectively. As shown in Fig. 2, the mean tumor weight of the mice in the model group was 1.1792±0.0989 g, and the tumor weight inhibition rates of the mice in the po, L, and M groups were 21%, 22%, and 61%, respectively.

In conclusion, in the mouse model of KC-1474 cell subcutaneous transplantation tumor, compared with the model group, the growth of tumor was inhibited in the po, L, and M groups, wherein the difference between the M group and the model group was extremely significant (*P* < 0.001). Besides, the tumor volume was reduced in the po, L, and M groups, showing a good antitumor drug efficacy.

**Claims**

1. A compound shown in formula I, a stereoisomer thereof, or a pharmaceutically acceptable salt thereof,

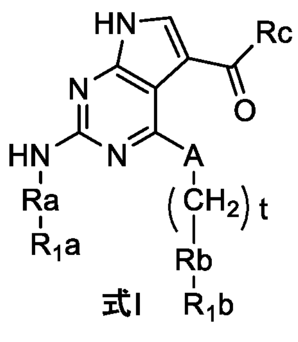

式I ,

wherein the Rc is

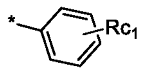 or 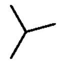 ;

the number of the $Rc_1$ is 0, 1, 2, or 3; each $Rc_1$ is independently selected from at least one of halogen and $C_{1-3}$ alkyl; the Ra is a substituted or unsubstituted phenyl, or a substituted or unsubstituted

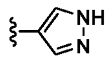 ;

the substituents are each independently selected from halogen, $C_{1-3}$ alkyl, and $C_{1-3}$ alkoxy; the number of the respective substituents of the phenyl or the

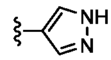

is 0, 1, or 2; the halogen is F, Cl, Br, and I;
the $R_1a$ is selected from H,

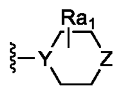 , 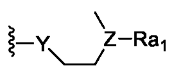 ;

or $C_{1-3}$ alkyl; the Y and the Z are each independently selected from N, O, and C, wherein the N and the C are substituted by 0 or 1 $C_{1-3}$ alkyl and the insufficient valence bonds are complemented by H; and the $Ra_1$ is H or $C_{1-3}$ alkyl;
the A is O or N, wherein the N is substituted by at least one of H and $C_{1-3}$ alkyl;
the t is selected from 0, 1, 2, or 3;
the Rb is selected from groups in one or more groups of the following 1)-5):

1) a substituted or unsubstituted 5- to 8-membered spiroheterocycle, wherein a heteroatom in the spiroheterocycle is O, S, or N; the substituent of the spiroheterocycle is one or more independent substituted or unsubstituted amino, $C_{1-3}$ alkyl, $C_{1-3}$ alkoxy, and OH; and the substituent of the amino is H or one or two $C_{1-3}$ alkyls;
2) a substituted or unsubstituted $C_8$-$C_{11}$ bridge ring, wherein the substituent of the bridge ring is one or more independent substituted or unsubstituted amino, $C_{1-3}$ alkyl, $C_{1-3}$ alkoxy, and OH; and the substituent of the amino is H or one or two $C_{1-3}$ alkyls;
3) a substituted or unsubstituted amino, wherein the substituent of the amino is H or one or two $C_{1-3}$ alkyls, or $-(CH_2)_a-E-(CH_2)_b-$, and N in the amino is linked with both ends of the $-(CH_2)_a-E-(CH_2)_b-$ to form a ring; the E is C or O, and the a is 1, 2, 3, or 4; and the b is 1, 2, 3, or 4;
4)

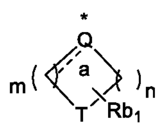 ,

wherein the number of the double bonds a is 0, 1, 2, or 3; the position of the a is any position with a reasonable valence bond in the ring; the insufficient valence bonds are complemented by H; the m is 0, 1, 2, or 3; the n is 0, 1, 2, or 3; the Q and the T are each independently N, O, S, or C; and the insufficient valence bonds are complemented by H; and
5)

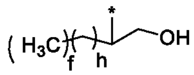,

wherein the f is 1, 2, or 3; and the h is 0, 1, or 2;

the number of the Rbi is 0, 1, or 2; the Rbi is amino, hydroxy, $C_{1-3}$ alkyl, $C_{1-3}$ alkoxy, $C_{1-3}$ alkyl substituted by hydroxy, $SO_2R_2b$,

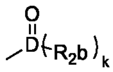, or 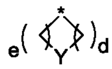;

the $R_2b$ is a substituted or unsubstituted $C_{1-3}$ alkyl, a substituted or unsubstituted phenyl, $C_{3-6}$ cycloalkyl, $C_{2-5}$ alkenyl, or

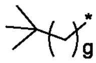;

the substituent of the $C_{1-3}$ alkyl or the phenyl is halogen or hydroxyl; the D is selected from C, P, or S; the g is 1, 2, or 3; the e and the d are each independently selected from 0, 1, 2, or 3; and the Y is selected from C, O, or N;
the $R_1b$ is H, $NH_2$, hydroxyl, $C_{1-3}$ alkoxy, $C_{1-3}$ alkyl substituted by hydroxyl, $SO_2R_2b$, $C_{1-3}$ alkyl,

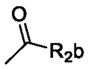, 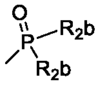, or 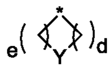;

the $R_2b$ is independently a substituted or unsubstituted $C_{1-3}$ alkyl, a substituted or unsubstituted phenyl, $C_{3-6}$ cycloalkyl, $C_{2-5}$ alkenyl, or

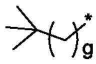;

the substituent of the $C_{1-3}$ alkyl or the phenyl is halogen or hydroxyl; the g is 1, 2, or 3; the e and the d are each independently selected from 0, 1, 2, or 3; and the Y is selected from C, O, or N; and
the $C_{1-3}$ alkyl is methyl, ethyl, propyl, or isopropyl; the $C_{1-3}$ alkoxy is methoxy, ethoxy, propoxy, or isopropoxy; and the halogen is F, Cl, Br, or I.

**2.** The compound, the stereoisomer thereof, or the pharmaceutically acceptable salt thereof according to claim 1, wherein the Rc is

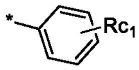 or 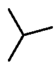,

the number of the $Rc_1$ is 0, 1, 2, or 3; each $Rc_1$ is independently selected from at least one of F, Cl, Br, I, and C1-3 alkyl;

the Ra is a substituted or unsubstituted phenyl, or a substituted or unsubstituted

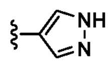;

; the substituents are each independently selected from F, C1-3 alkyl, and C1-3 alkoxy; the number of the respective substituents of the phenyl or the

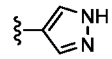

is independently 0, 1, or 2;
the $R_1a$ is selected from H,

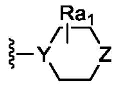, 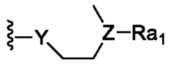,

or $C_{1-3}$ alkyl; the Y is C or N; the Z is N or O, wherein the N and the C are substituted by 0 or 1 $C_{1-3}$ alkyl; and the $Ra_1$ is H or $C_{1-3}$ alkyl;
the Rb is selected from groups in one or more groups of the following 1)-5):

1) a substituted or unsubstituted 5- to 8-membered spiroheterocycle, wherein a heteroatom in the spiroheterocycle is O;
2) a substituted or unsubstituted C10 bridge ring, wherein the substituent of the bridge ring is one or more independent amino, C1-3 alkyl, C1-3 alkoxy, and OH;
3) a substituted or unsubstituted amino, wherein the substituent of the amino is one or two $C_{1-3}$ alkyls, or $-(CH_2)_a$-E-$(CH_2)_b$-, and N in the amino is linked with both ends of the $-(CH_2)_a$-E-$(CH_2)_b$- to form a ring; the E is C or O, and the a is 1, 2, or 3; and the b is 1, 2, or 3;
4)

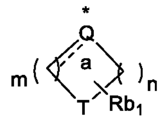,

wherein the number of the double bonds a is 0, 1, 2, or 3; the position of the a is any position with a reasonable valence bond in the ring; the m is 0, 1, 2, or 3; the n is 0, 1, 2, or 3; the Q and the T are each independently N, O, S, or C; and the insufficient valence bonds are complemented by H; and
5)

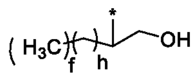,

wherein the f is 1, 2, or 3; and the h is 0 or 1;

the number of the Rbi is 0, 1, or 2; the Rbi is amino, hydroxy, $C_{1-3}$ alkyl, $C_{1-3}$ alkoxy or $C_{1-3}$ alkyl substituted by hydroxy, $SO_2R_2b$,

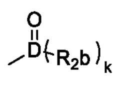, or 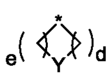;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

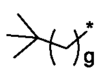;

the substituent of the $C_{1-3}$ alkyl or the phenyl is halogen or hydroxyl;
the D is selected from C, P, or S; the k is 1 or 2; the g is 1, 2, or 3; the e and the d are each independently selected from 0, 1, 2, or 3; and Y is selected from C, O, or N; and

the $R_1b$ is H, $NH_2$, hydroxyl, $C_{1-3}$ alkoxy, $C_{1-3}$ alkyl substituted by hydroxyl, $SO_2R_2b$, $C_{1-3}$ alkyl,

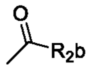, 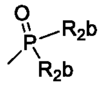, or 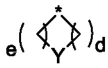;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

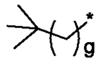;

the substituent of the $C_{1-3}$ alkyl or the phenyl is halogen or hydroxyl; the g is 1, 2, or 3; the e and the d are each independently selected from 0, 1, 2, or 3; and the Y is selected from C, O, or N.

**3.** The compound, the stereoisomer thereof, or the pharmaceutically acceptable salt thereof according to claim 1, wherein the Rc is

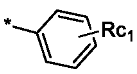 or 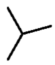,

the number of the $Rc_1$ is 0, 1, 2, or 3; each $Rc_1$ is independently selected from at least one of F, Cl, Br, I, and $C_{1-3}$ alkyl;

the Ra is a substituted or unsubstituted phenyl, or a substituted or unsubstituted

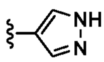;

; the substituents are each independently selected from F, C1-3 alkyl, and C1-3 alkoxy; the number of the respective substituents of the phenyl or the

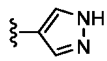

is independently 0, 1, or 2;
the $R_1a$ is selected from H,

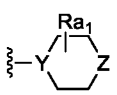, 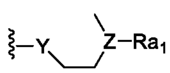;

or $C_{1-3}$ alkyl; the Y is C or N; the Z is N or O and the $Ra_1$ is H or $C_{1-3}$ alkyl;
the Rb is selected from groups in one or more groups of the following 1)-5):

1) a substituted or unsubstituted 5- to 8-membered spiroheterocycle, wherein a heteroatom in the spiroheterocycle is O;
2) a substituted or unsubstituted C10 bridge ring, wherein the substituent of the bridge ring is one or more independent amino, $C_{1-3}$ alkyl, $C_{1-3}$ alkoxy, and OH;
3) a substituted or unsubstituted amino, wherein the substituent of the amino is one or two $C_{1-3}$ alkyls, or $-(CH_2)_a-E-(CH_2)_b-$, and N in the amino is linked with both ends of the $-(CH_2)_a-E-(CH_2)_b-$ to form a ring; the E is C or O, and the a is 1, 2, or 3; and the b is 1, 2, or 3;
4)

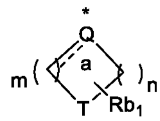,

wherein the number of the double bonds a is 0, 1, 2, or 3; the position of the a is any position with a reasonable valence bond in the ring; the insufficient valence bonds are complemented by H; the m is 0, 1, 2, or 3; the n is 0, 1, 2, or 3; the Q and the T are each independently N, O, S, or C; and the insufficient valence bonds are complemented by H; and

5)

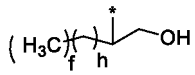,

wherein the f is 1, 2, or 3; and the h is 0 or 1;

the number of the Rbi is 0, 1, or 2; the Rbi is amino, hydroxy, $C_{1-3}$ alkyl, $C_{1-3}$ alkoxy or $C_{1-3}$ alkyl substituted by hydroxy, $SO_2R_2b$,

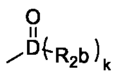, or 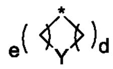;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

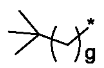;

the substituent of the $C_{1-3}$ alkyl or the phenyl is halogen or hydroxyl;

the D is selected from C, P, or S; the k is 1 or 2; the g is 1, 2, or 3; the e and the d are each independently selected from 0, 1, 2, or 3; and Y is selected from C, O, or N; and

the $R_1b$ is H, $NH_2$, hydroxyl, $C_{1-3}$ alkoxy, $C_{1-3}$ alkyl substituted by hydroxyl, $SO_2R_2b$, $C_{1-3}$ alkyl,

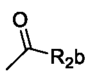, 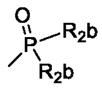, or 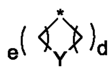;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

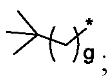;

the substituent of the $C_{1-3}$ alkyl or the phenyl is halogen or hydroxyl; the g is 1, 2, or 3; the e and the d are each independently selected from 0, 1, 2, or 3; and the Y is selected from C, O, or N.

4. The compound, the stereoisomer thereof, or the pharmaceutically acceptable salt thereof according to claim 1, wherein the Rc is

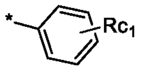 or 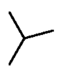,

the number of the $Rc_1$ is 0, 1, 2, or 3; each Rc1 is independently selected from at least one of F, Cl, and $C_{1-3}$ alkyl;

the Ra is a substituted or unsubstituted phenyl, or a substituted or unsubstituted

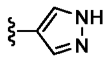
;

the substituent of the phenyl or the

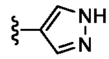

in the Ra is F, methyl, and methoxy; and the number of the respective substituents of the phenyl or the

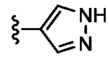

is 1;
the $R_1a$ is selected from H,

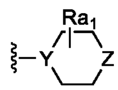
, 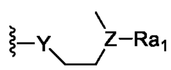
,

or $C_{1-3}$ alkyl; the Y is C or N; the Z is N or O and the $Ra_1$ is H, $CH_3$, or $CH_2CH_3$; t is selected from 0, 1, or 2; the Rb is selected from groups in one or more groups of the following 1)-5):

1) a substituted or unsubstituted 5- to 8-membered spiroheterocycle, wherein a heteroatom in the spiroheterocycle is O;
2) a substituted or unsubstituted C10 bridge ring, wherein the substituent of the bridge ring is one or more independent amino, $C_{1-3}$ alkyl, $C_{1-3}$ alkoxy, and OH;
3) a substituted or unsubstituted amino, wherein the substituent of the amino is one or two $C_{1-3}$ alkyls, or $-(CH_2)_a-E-(CH_2)_b-$, and N in the amino is linked with both ends of the $-(CH_2)_a-E-(CH_2)_b-$ to form a ring; the E is C or O, and the a is 1, 2, or 3; and the b is 1, 2, or 3;
4)

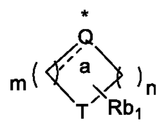
,

wherein the number of the double bonds a is 0, 1, 2, or 3; the position of the a is any position with a reasonable valence bond in the ring; the insufficient valence bonds are complemented by H; the m is 0, 1, 2, or 3; the n is 0, 1, 2, or 3; the Q and the T are each independently N, O, S, or C; and the insufficient valence bonds are complemented by H; and
5)

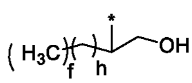
,

wherein the f is 1, 2, or 3; and the h is 0 or 1;

the number of the Rbi is 0 or 1; the Rbi is amino, hydroxy, $C_{1-3}$ alkyl, $C_{1-3}$ alkoxy or $C_{1-3}$ alkyl substituted by hydroxy, $SO_2R_2b$,

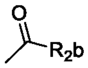, 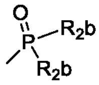, or 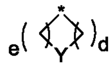;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

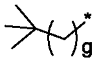;

the substituent of the $C_{1-3}$ alkyl or the phenyl is halogen or hydroxyl;

the g is 1, 2, or 3; the e and the d are each independently selected from 0, 1, 2, or 3; and the Y is selected from C, O, or N; and

the $R_1b$ is H, $NH_2$, hydroxymethyl, hydroxyethyl, hydroxypropyl, methyl, ethyl, propyl, isopropyl, OH, $SO_2R_2b$, $((CH_3)_2CH_2$,

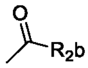, 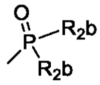, or 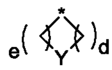;

each $R_2b$ is independently a substituted or unsubstituted $C_{1-3}$ alkyl, a substituted or unsubstituted phenyl, $C_{3-5}$ cycloalkyl, $C_{2-3}$ alkenyl, or

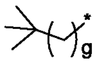;

the substituent of the phenyl is halogen or hydroxyl;

the g is 1, 2, or 3; the e and the d are each independently selected from 0, 1, 2, or 3; the Y is selected from C, O, or N; and the halogen is F, Cl, Br, or I.

**5.** The compound, the stereoisomer thereof, or the pharmaceutically acceptable salt thereof according to claim 1, wherein the Rc is

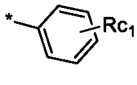 or 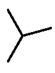,

the number of the $Rc_1$ is 0, 1, 2, or 3; each $Rc_1$ is independently selected from at least one of F, Cl, and $C_{1-3}$ alkyl;

the Ra is a substituted or unsubstituted phenyl, or a substituted or unsubstituted

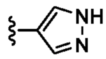;

each substituent of the phenyl or the

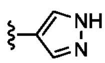

in the Ra is independently selected from F, methyl, and methoxy; and the number of the respective substituents of the phenyl or the

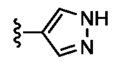

is independently 0, 1, or 2;
the $R_1a$ is selected from H,

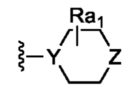, 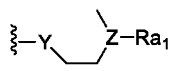,

or $C_{1-3}$ alkyl; the Y is N, O, or C; the Z is N or O;
the $Ra_1$ is H, $CH_3$, or $CH_2CH_3$; A is O or N, wherein the N is substituted by an H; t is selected from 0, 1, or 2;
the Rb is selected from groups in one or more groups of the following 1)-4):

1) a substituted or unsubstituted 7-membered spiroheterocycle, wherein a heteroatom in the spiroheterocycle is O;
2) a substituted or unsubstituted adamantyl, wherein the substituent of the adamantyl is one or more OHs;
3) a substituted or unsubstituted amino, wherein the substituent of the amino is one or two $C_{1-3}$ alkyls; and
4)

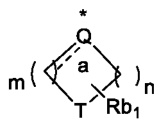,

wherein the number of the double bonds a is 0 or 1; the position of the a is any position with a reasonable valence bond in the ring; the insufficient valence bonds are complemented by H; the m is 0, 1, 2, or 3; the n is 0, 1, or 2; the Q is C; the T is N, O or C; and the insufficient valence bonds are complemented by H;

the number of the Rbi is 0 or 1; the Rbi is amino, hydroxy, $C_{1-3}$ alkyl, $C_{1-3}$ alkoxy or $C_{1-3}$ alkyl substituted by hydroxy, $SO_2R_2b$,

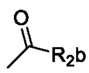, or 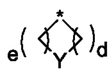;

each $R_2b$ is independently a substituted or unsubstituted $C_{1-3}$ alkyl, cyclopropyl, or $C_{2-3}$ alkenyl; the substituent of the $C_{1-3}$ alkyl is halogen or hydroxyl; the e and the d are each independently selected from 0, 1, 2, or 3; and the Y is selected from C, O, or N; and
the $R_1b$ is amino, hydroxy, $C_{1-3}$ alkyl substituted by hydroxy, $C_{1-3}$ alkoxy, $SO_2R_2b$, or

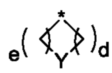;

the $R_2b$ is selected from $C_{1-3}$ alkyl or $C_{3-6}$ cycloalkyl; the e and the d are each independently selected from 1, 2, or 3; and the Y is selected from C, O, or N.

**6.** The compound, the stereoisomer thereof, or the pharmaceutically acceptable salt thereof according to claim 1, wherein the Rc is

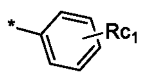 or 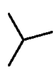,

the number of the $Rc_1$ is 0, 1, 2, or 3; each $Rc_1$ is independently selected from at least one of F, Cl, and $C_{1-3}$ alkyl;

the Ra is a substituted or unsubstituted phenyl, or a substituted or unsubstituted

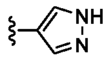 ;

the substituents are each independently selected from F, methyl, and methoxy; and the number of the respective substituents of the phenyl or the

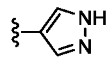

is independently 0, 1, or 2;
the $R_1$a is selected from H,

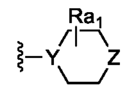, 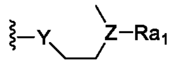,

or $C_{1-3}$ alkyl; the Y is N, O, or C; the Z is N or O;
the $Ra_1$ is H, $CH_3$, or $CH_2CH_3$; A is O or N, wherein the N is substituted by an H; t is selected from 0, 1, or 2;
the Rb is selected from groups in one or more groups of the following 1)-4):

1) a substituted or unsubstituted 7-membered spiroheterocycle, wherein a heteroatom in the spiroheterocycle is O;
2) a substituted or unsubstituted adamantyl, wherein the substituent of the adamantyl is one or more OHs;
3) a substituted or unsubstituted amino, wherein the substituent of the amino is one or two $C_{1-3}$ alkyls; and
4)

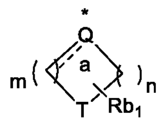,

wherein the number of the double bonds a is 0 or 1; the position of the a is any position with a reasonable valence bond in the ring; the insufficient valence bonds are complemented by H; the m is 0, 1, 2, or 3; the n is 0, 1, or 2; the Q is C; the T is N, O or C; and the insufficient valence bonds are complemented by H;

the number of the Rbi is 0, 1, or 2; the Rbi is amino, hydroxy, $C_{1-3}$ alkyl, $C_{1-3}$ alkoxy or $C_{1-3}$ alkyl substituted by hydroxy, $SO_2R_2b$,

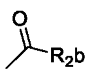, or 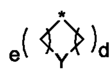;

each $R_2$b is independently a substituted or unsubstituted $C_{1-3}$ alkyl, cyclopropyl, or $C_{2-3}$ alkenyl; the substituent of the $C_{1-3}$ alkyl is halogen or hydroxyl; the e and the d are each independently selected from 0, 1, 2, or 3; and the Y is selected from C, O, or N; and
the $R_1$b is H, amino, hydroxy, $C_{1-3}$ alkyl substituted by hydroxy, $C_{1-3}$ alkoxy, $SO_2R_2b$, or

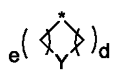;

the $R_2$b is selected from $C_{1-3}$ alkyl or $C_{3-6}$ cycloalkyl; the e and the d are each independently selected from 1, 2, or 3; and the Y is selected from C, O, or N.

7. The compound, the stereoisomer thereof, or the pharmaceutically acceptable salt thereof according to claim 1, wherein the Rc in formula I is a substituted or unsubstituted phenyl, as shown in formula II,

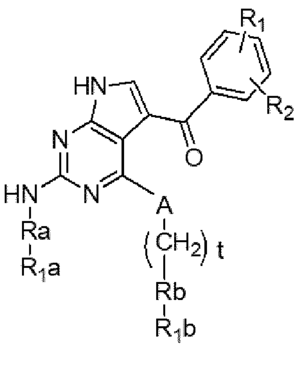

Formula II

,

the $R_1$ and the $R_2$ are each independently selected from at least one of H, halogen, and C1-3 alkyl.

8. The compound, the stereoisomer thereof, or the pharmaceutically acceptable salt thereof according to claim 1, wherein the Rc in formula I is a substituted or unsubstituted phenyl, the Ra is a substituted or unsubstituted benzene ring, and

the Rb is

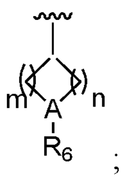

;

the $R_1$b is H, and as shown in formula III,

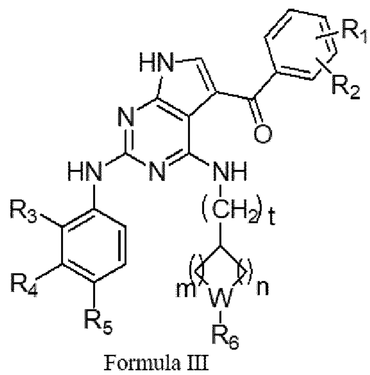

Formula III

,

wherein the m is selected from 0, 1, or 2; the n is selected from 0, 1, or 2; the t is selected from 0, 1, or 2; the W is C, O, or N; the R1 is selected from H, F, or Cl; the $R_2$ is selected from H, F, or Cl; the $R_3$ is selected from H or MeO; the $R_4$ is selected from H or F; the $R_5$ is selected from H,

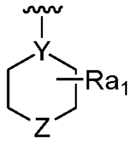 , 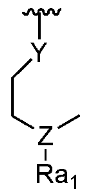 ,

or $C_{1-3}$ alkyl; the Y and the Z are each independently selected from N, O, and C, and the insufficient valence bonds are complemented by H; the $Ra_1$ is H and $C_{1-3}$ alkyl; and the $Ra_1$ is H or $C_{1-3}$ alkyl; and $R_6$ is selected from H, $NH_2$, hydroxyl, $C_{1-3}$ alkoxy, $C_{1-3}$ alkyl substituted by hydroxyl, $SO_2R_2b$, $C_{1-3}$ alkyl,

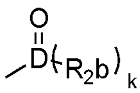 , or 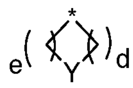 ;

each $R_2b$ is independently a substituted or unsubstituted $C_{1-3}$ alkyl, a substituted or unsubstituted phenyl, $C_{3-6}$ cycloalkyl, $C_{2-5}$ alkenyl, or

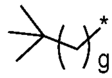 ;

the D is selected from C, P, or S; the k is 1 or 2; the substituent of the $C_{1-3}$ alkyl or the phenyl is halogen or hydroxyl; the g is 1, 2, or 3; the e and the d are each independently selected from 0, 1, 2, or 3; and the Y is selected from C, O, or N.

**9.** The compound, the stereoisomer thereof, or the pharmaceutically acceptable salt thereof according to claim 1, wherein in formula I, the Ra is a substituted or unsubstituted

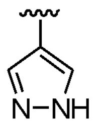 ,

and the substituent is at least one of $C_{1-3}$ alkoxy and $C_{1-3}$ alkyl; and further, the substituent is one, two or three of H, methoxy, ethoxy, propoxy, isopropoxy, methyl, ethyl, and propyl.

**10.** The compound, the stereoisomer thereof, or the pharmaceutically acceptable salt thereof according to claim 1, wherein in formula I, the Ra is

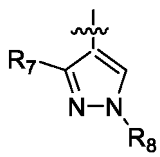

and the Rb is selected from

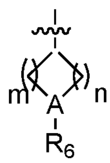 ;

the $R_1b$ is H, and as shown in formula IV,

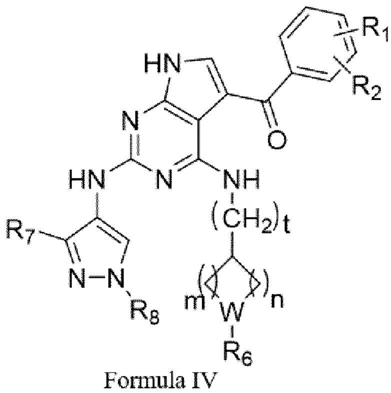

Formula IV

,

wherein the m is selected from 0, 1, or 2; the n is selected from 0, 1, or 2; the t is selected from 0, 1, or 2; the W is C, O, or N; the $R_1$ is selected from H, F, or Cl; the $R_2$ is selected from H, F, or Cl; the $R_6$ is selected from H, OH, $CH_2OH$,

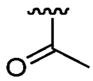, 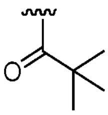, 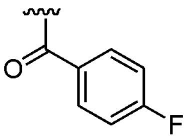,

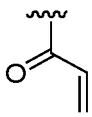, 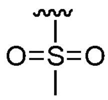, 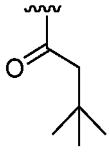, 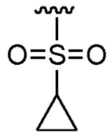, or 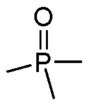;

$R_7$ is selected from H or $C_{1-3}$ alkoxy; and $R_8$ is selected from $C_{1-3}$ alkyl or piperidyl substituted by $C_{1-3}$ alkyl.

**11.** The compound, the stereoisomer thereof, or the pharmaceutically acceptable salt thereof according to claim 1, wherein the compound is selected from:

(S)-N-(1-(5-(4-fluorobenzoyl)-2-((5-(4-methylpiperazin-1-yl)pyridin-2-yl)amino)-7H-pyrrolo [2,3-d]pyrimidin-4-yl)piperidin-3-yl)cyclopropanesulfonamide,
(S)-N-(1-(5-(4-fluorobenzoyl)-2-((5-(4-methylpiperazin-1-yl)pyridin-2-yl)amino)-7H-pyrrolo [2,3-d]pyrimidin-4-yl)pyrrolidin-3-yl)cyclopropanesulfonamide,
(S)-N-(1-(5-(4-fluorobenzoyl)-2-((6-(4-methylpiperazin-1-yl)pyridin-3-yl)amino)-7H-pyrrolo [2,3-d]pyrimidin-4-yl)pyrrolidin-3-yl)cyclopropanesulfonamide,
(S)-N-(1-(5-(4-fluorobenzoyl)-2-((1-methyl-1H-pyrazol-4-yl)amino)-7H-pyrrolo[2,3-d]pyrimi din-4-yl)pyrrolidin-3-yl)cyclopropanesulfonamide,
(S)-N-(1-(5-(4-fluorobenzoyl)-2-((4-(4-methylpiperazin-1-yl)phenyl)amino)-7H-pyrrolo[2,3-d] pyrimidin-4-yl)piperidin-3-yl)cyclopropanesulfonamide,
(R)-(4-((1-(cyclopropylsulfonyl)piperidin-3-yl)amino)-2-((4-(4-methylpiperazin-1-yl)phenyl) amino)-7H-pyrrolo[2,3-d]pyrimidin-5-yl)(4-fluorophenyl)methanone,
(R)-(4-(1-(cyclopropylsulfonyl)piperidin-3-yl)amino)-2-((1-methyl-1H-pyrazol-4-yl)amino)-7 H-pyrrolo[2,3-d]pyrimidin-5-yl)(4-fluorophenyl)methanone,
(R)-1-(4-((1-(cyclopropylsulfonyl)piperidin-3-yl)amino)-2-((4-(4-methylpiperazin-1-yl)pheny l)amino)-7H-pyrrolo[2,3-d ]pyrimidin-5-yl)-2-methylpropan-1-one,
(S)-(4-(1-(cyclopropylsulfonyl)piperidin-3-yl)amino)-2-((4-(4-methylpiperazin-1-yl)phenyl)a mino)-7H-pyrrolo[2,3-d]pyrimidin-5-yl)(4-fluorophenyl)methanone,
(R)-(4-(1-(cyclopropylsulfonyl)pyrrolidin-3-yl)amino)2-((4-(4-methylpiperazin-1-yl)phenyl)a mino)-7H-pyrrolo[2,3-d]pyrimidin-5-yl)(4-fluorophenyl)methanone,

(S)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(4-fluorophenyl)methanone,
(S)-1-(3-((5-(4-fluorobenzoyl)-2-((4-(4-methylpiperazin-1-yl)phenyl)amino)-7H-pyrrolo[2,3-d]pyrimidin-4-yl)amino)piperidin-1-yl)prop-2-en-1-one,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1 -yl)prop-2-en-1 -one,
(S)-(4-(1-(cyclopropylsulfonyl)piperidin-3-yl)amino)-2-((4-(4-ethylpiperazin-1-yl)phenyl)ami no)-7H-pyrrolo[2,3-d]pyrimidin-5-yl)(4-fluorophenyl)methanone,
(S)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(phenyl)methanone,
(S)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(3-fluorophenyl)methanone,
(S)-(4-(1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(3,4-difluorophenyl)methanone,
(4-fluorophenyl)(2-((4-(4-methylpiperazin-1-yl)phenyl)amino)-4-(morpholinoamino)-7H-pyrr olo[2,3-d]pyrimidin-5-yl)methanone,
(4-fluorophenyl)(2-((4-(4-methylpiperazin-1-yl)phenyl)amino)-4-((tetrahydro-2H-pyran-4-yl) amino)-7H-pyrrolo[2,3-d]pyrimidin-5-yl)methanone,
(4-fluorophenyl)(4-(((1r,4r)-4-(hydroxymethyl)cyclohexyl)amino)-2-((4-(4-methylpiperazin-1 -yl)phenyl)amino)-7H-pyrrolo[2,3-d]pyrimidin-5-yl)methanone,
(S)-1-(3-((5-(2,4-difluorobenzoyl)-2-((4-(4-methylpiperazin-1-yl)phenyl)amino)-7H-pyrrolo[ 2,3 -d]pyrimidin-4-yl)amino)pyrrolidin-1 -yl)prop-2-en-1 -one,
(S)-1-(3-((5-(2-fluorobenzoyl)-2-((4-(4-methylpiperazin-1-yl)phenyl)amino)-7H-pyrrolo[2,3-d]pyrimidin-4-yl)amino)pyrrolidin-1 -yl)propan-2-en-1 -one,
(R)-1-(3-((5-(4-fluorobenzoyl)-2-((1-methyl-1H-pyrazol-4-yl)amino)-7H-pyrrolo[2,3-d]pyrim idin-4-yl)amino)pyrrolidin-1-yl)propan-2-en-1-one,
(S)-(4-((1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(2,4-difluorophenyl)methanone,
(R)-(4-fluorophenyl)(2-((4-(4-methylpiperazin-1-yl)phenyl)amino)-4-(1-(methylsulfonyl)pyrr olidin-3-yl)amino)-7H-pyrrolo[2,3-d]pyrimidin-5-yl)methanone,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1-yl)-3,3-dimethylbut-1-one,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1-yl)-2, 2-dimethylpropan-1-one,
(4-fluorophenyl)(4-(((1s,4s)-4-hydroxycyclohexyl)amino)-2-((4-(4-methylpiperazin-1-yl)phe nyl)amino)-7H-pyrrolo[2,3-d]pyrimidin-5-yl)methanone,
(S)-(4-fluorophenyl)(2-((4-(4-methylpiperazin-1-yl)phenyl)amino)-4-(tetrahydro-2H-pyran-3-yl)amino)-7H-pyrrolo[2,3-d]pyrimidin-5-yl)methanone,
(4-fluorophenyl)(2-((4-(4-methylpiperazin-1-yl)phenyl)amino)-4-((tetrahydro-2H-pyran-4-yl) methyl)amino)-7H-pyrrolo[2,3-d]pyrimidin-5-yl)methanone,
(4-fluorophenyl)(4-(methyl(tetrahydro-2H-pyran-4-yl)amino)-2-(4-((4-methylpiperazin-1-yl)p henyl)amino)-7H-pyrrolo[2,3-d]pyrimidin-5-yl)methanone,
1-(4-((5-(4-fluorobenzoyl)-2-((4-(4-methylpiperazin-1-yl)phenyl)amino)-7H-pyrrolo[2,3-d]py rimidin-4-yl)amino)piperidin-1-yl)prop-2-en-1-one,
(4-fluorophenyl)(2-((4-(4-methylpiperazin-1-yl)phenyl)amino)-4-((1-(methylsulfonyl)piperidi n-4-yl)amino)-7H-pyrrolo[2,3-d]pyrimidin-5-yl)methanone,
1-(4-((5-(4-fluorobenzoyl)-2-((4-(4-methylpiperazin-1-yl)phenyl)amino)-7H-pyrrolo[2,3-d]py rimidin-4-yl)amino)piperidin-1-yl)-3,3-dimethylbut-1-one,
(R)-(4-fluorophenyl)(2-((4-(4-methylpiperazin-1-yl)phenyl)amino)-4-(tetrahydrofuran-3-yl)a mino)-7H-pyrrolo[2,3-d]pyrimidin-5-yl)methanone,
(4-fluorophenyl)(4-((3-methyloxetan-3-yl)amino)-2-((4-(4-methylpiperazin-1-yl)phenyl)amin o)-7H-pyrrolo[2,3-d]pyrimidin-5-yl)methanone,
(4-fluorophenyl)(4-(((1s,3R,4s,5S,7s)-4-hydroxyadamantan-1-yl)amino)-2-((4-(4-methylpiper azin-1-yl)phenyl)amino)-7H-pyrrolo[2,3-d]pyrimidin-5-yl)methanone,
(R)-(4-fluorophenyl)(4-((1-hydroxy-4-methylpentan-2-yl)amino)-2-((4-(4-methylpiperazin-1-yl)phenyl)amino)-7H-pyrrolo[2,3-d]pyrimidin-5-yl)methanone,
(S)-(4-fluorophenyl)(4-((1-hydroxy-3,3-dimethylbut-2-yl)amino)-2-((4-(4-methylpiperazin-1-yl)phenyl)amino)-7H-pyrrolo[2,3-d]pyrimidin-5-yl)methanone,

(4-fluorophenyl)(2-((4-(4-methylpiperazin-1-yl)phenyl)amino)-4-((2-(tetrahydro-2H-pyran-4-yl)ethyl)amino)-7H-pyrrolo[2,3-d]pyrimidin-5-yl)methanone,
(4-fluorophenyl)(4-((1-(hydroxymethyl)cyclopentyl)amino)-2-((4-(4-methylpiperazin-1-yl)ph enyl)amino)-7H-pyrrolo[2,3 -d]pyrimidin-5-yl)methanone,
(4-fluorophenyl)(2-((4-(4-methylpiperazin-1-yl)phenyl)amino)-4-((tetrahydro-2H-pyran-3-yl) amino)-7H-pyrrolo[2,3-d]pyrimidin-5-yl)methanone,
(4-((2-(diethylamino)ethyl)amino)-2-((4-(4-methylpiperazin-1-yl)phenyl)amino)-7H-pyrrolo[ 2,3-d]pyrimidin-5-yl)(4-fluorophenyl)methanone,
(4-fluorophenyl)(2-((4-(4-methylpiperazin-1-yl)phenyl)amino)-4-((2-(pyrrolidin-1-yl)ethyl)a mino)-7H-pyrrolo[2,3 -d]pyrimidin-5-yl)methanone,
(4-fluorophenyl)(4-((4-hydroxytetrahydro-2H-pyran-4-yl)methyl)amino)-2-((4-(4-methylpipe razin-1-yl)phenyl)amino)-7H-pyrrolo[2,3-d]pyrimidin-5-yl)methanone,
(4-fluorophenyl)(4-(((1r,4r)-4-(hydroxymethyl)cyclohexyl)amino)-2-((4-morpholinophenyl)a mino)-7H-pyrrolo[2,3 -d]pyrimidin-5-yl)methanone,
(4-fluorophenyl)(4-(((1r,4r)-4-(hydroxymethyl)cyclohexyl)amino)-2-((2-methoxy-4-morpholi nophenyl)amino)-7H-pyrrolo[2,3-d]pyrimidin-5-yl)methanone,
(4-fluorophenyl)(4-(((1r,4r)-4-(hydroxymethyl)cyclohexyl)amino)-2-((1-methyl-1H-pyrazol-4 -yl)amino)-7H-pyrrolo[2,3-d]pyrimidin-5-yl)methanone,
(4-fluorophenyl)(2-((4-(4-methylpiperazin-1-yl)phenyl)amino)-4-((tetrahydro-2H-pyran-4-yl) oxy)-7H-pyrrolo[2,3-d]pyrimidin-5-yl)methanone,
(4-(((1s,4s)-4-aminocyclohexyl)amino)-2-((4-(4-methylpiperazin-1-yl)phenyl)amino)-7H-pyrr olo[2,3-d]pyrimidin-5-yl)(4-fluorophenyl)methanone,
(4-fluorophenyl)(4-((6-(hydroxymethyl)pyridin-3-yl)amino)-2-((4-(4-methylpiperazin-1-yl)ph enyl)amino)-7H-pyrrolo[2,3-d]pyrimidin-5-yl)methanone,
((1r,4r)-4-((2-((4-morpholinophenyl)amino)-7H-pyrrolo[2,3-d]pyrimidin-4-yl)amino)cyclohe xyl)methanol,
(4-fluorophenyl)(4-((1-isopropylpiperidin-4-yl)amino)-2-((4-(4-methylpiperazin-1-yl)phenyl) amino)-7H-pyrrolo[2,3-d]pyrimidin-5-yl)methanone,
(4-((3,5-bis(hydroxymethyl)phenyl)amino)-2-((4-(4-methylpiperazin-1-yl)phenyl)amino)-7H-pyrrolo[2,3-d]pyrimidin-5-yl)(4-fluorophenyl)methanone,
(4-((2-((4-(4-methylpiperazin-1-yl)phenyl)amino)-7H-pyrrolo[2,3-d]pyrimidin-4-yl)amino)pi peridin-1-yl)methanol,
(3-fluorophenyl)(4-(((1r,4r)-4-(hydroxymethyl)cyclohexyl)amino)-2-((4-morpholinophenyl)a mino)-7H-pyrrolo[2,3-d]pyrimidin-5-yl)methanone,
(4-fluorophenyl)(4-((3R,6S)-6-(hydroxymethyl)tetrahydro-2H-pyran-3-yl)amino)-2-((4-morp holinophenyl)amino)-7H-pyrrolo[2,3-d]pyrimidin-5-yl)methanone,
(2,4-difluorophenyl)(4-(((1r,4r)-4-(hydroxymethyl)cyclohexyl)amino)-2-((4-morpholinophen yl)amino)-7H-pyrrolo[2,3-d]pyrimidin-5-yl)methanone, (4-fluorophenyl)(4-(((1r,4r)-4-(hydroxymethyl)cyclohexyl)amino)-2-((3-methoxy-1-(1-methylpiperidin-4-yl)-1H-pyrazol-4-yl)amino)-7H-pyrrolo[2,3-d]pyrimidin-5-yl)methanone,
(4-((2-oxaspiro[3.3]hept-6-yl)amino)-2-((4-(4-methylpiperazin-1-yl)phenyl)amino)-7H-pyrrol o[2,3-d]pyrimidin-5-yl)(4-fluorophenyl)methanone,
((1r,4r)-4-((2-((1-methyl-1H-pyrazol-4-yl)amino)-7H-pyrrolo[2,3-d]pyrimidin-4-yl)amino)cy clohexyl)methanol,
(4-fluorophenyl)(4-((1-isopropylpiperidin-4-yl)amino)-2-((4-morpholinophenyl)amino)-7H-p yrrolo[2,3-d]pyrimidin-5-yl)methanone,
(3,5-difluorophenyl)(4-(((1r,4r)-4-(hydroxymethyl)cyclohexyl)amino)-2-((4-morpholinophen yl)amino)-7H-pyrrolo[2,3-d]pyrimidin-5-yl)methanone,
(2-((3-fluoro-4-(4-methylpiperazin-1-yl)phenyl)amino)-4-(((1s,4s)-4-(hydroxymethyl)cyclohe xyl)amino)-7H-pyrrolo[2,3-d]pyrimidin-5-yl)(4-fluorophenyl)methanone,
(4-fluorophenyl)(4-(((1 s,4s)-4-(hydroxymethyl)cyclohexyl)amino)-2-((1-methyl-1H-pyrazol-4-yl)amino)-7H-pyrrolo[2,3-d]pyrimidin-5-yl)methanone,
(4-fluorophenyl)(4-(((1s,4s)-4-(hydroxymethyl)cyclohexyl)amino)-2-((2-methoxy-4-(4-methy lpiperazin-1-yl)phenyl)amino)-7H-pyrrolo[2,3-d]pyrimidin-5-yl) methanone,
(2-((4-(2-(dimethylamino)ethoxy)phenyl)amino)-4-(((1s,4s)-4-(hydroxymethyl)cyclohexyl)a mino)-7H-pyrrolo[2,3-d]pyrimidin-5-yl)(4-fluorophenyl)methanone,
(4-fluorophenyl)(4-((1S,4R)-4-(hydroxymethyl)cyclopent-2-en-1-yl)amino)-2-((4-(4-methylpi perazin-1-yl)phenyl)amino)-7H-pyrrolo[2,3-d]pyrimidin-5-yl)methanone,
(4-(((1r,4r)-4-(hydroxymethyl)cyclohexyl)amino)-2-((4-morpholinophenyl)amino)-7H-pyrrol o[2,3-d]pyrimidin-5-yl)(2,3,4-trifluorophenyl)methanone,

(R)-1-(3-((5-(4-fluorobenzoyl)-2-((4-(4-methylpiperazin-1-yl)phenyl)amino)-7H-pyrrolo[2,3-d]pyrimidin-4-yl)amino)piperidin-1-yl)prop-2-en-1-one,
(R)-1-(3-((5-(4-fluorobenzoyl)-2-((4-(4-methylpiperazin-1-yl)phenyl)amino)-7H-pyrrolo[2,3-d]pyrimidin-4-yl)amino)piperidin-1-yl)prop-2-en-1-one,
(S)-(4-(1-(cyclopropylsulfonyl)piperidin-3-yl)amino)-2-((3-fluoro-4-(4-methylpiperazin-1-yl) phenyl)amino)-7H-pyrrolo[2,3-d]pyrimidin-5-yl)(4-fluorophenyl)methanone,
(S)-(4-(1-(cyclopropylsulfonyl)piperidin-3-yl)amino)-2-((4-morpholinophenyl)amino)-7H-pyrrolo[2,3-d]pyrimidin-5-yl)(4-fluorophenyl)methanone,
(S)-(4-(1-(cyclopropylsulfonyl)pyrrolidin-3-yl)amino)-2-((4-morpholinophenyl)amino)-7H-pyrrolo[2,3-d]pyrimidin-5-yl)(4-fluorophenyl)methanone,
(S)-1-(3-((5-benzoyl-2-((4-(4-methylpiperazin-1-yl)phenyl)amino)-7H-pyrrolo[2,3-d]pyrimidin-4-yl)amino)pyrrolidin-1-yl)prop-2-en-1-one,
(S)-1-(3-((5-(3-fluorobenzoyl)-2-((4-(4-methylpiperazin-1-yl)phenyl)amino)-7H-pyrrolo[2,3-d ]pyrimidin-4-yl)amino)pyrrolidin-1-yl)prop-2-en-1-one,
(S)-1-(3-((5-(3,4-difluorobenzoyl)-2-((4-(4-methylpiperazin-1-yl)phenyl)amino)-7H-pyrrolo[ 2,3-d]pyrimidin-4-yl)amino)pyrrolidin-1-yl)prop-2-en-1-one,
(4-fluorophenyl)(2-((4-(4-methylpiperazin-1 -yl)phenyl)amino)-4-(piperidin-1 -ylamino)-7H-pyrrolo[2,3-d]pyrimidin-5-yl)methanone,
(4-(cyclohexylamino)-2-((4-(4-methylpiperazin-1-yl)phenyl)amino)-7H-pyrrolo[2,3-d]pyrimidin-5-yl)(4-fluorophenyl)methanone,
(4-fluorophenyl)(4-((2-hydroxycyclohexyl)amino)-2-((4-(4-methylpiperazin-1-yl)phenyl)amino)-7H-pyrrolo[2,3-d]pyrimidin-5-yl)methanone,
(S)-(4-((1-(cyclopropylsulfonyl)pyrrolidin-3-yl)amino)-2-((4-(4-methylpiperazin-1-yl)phenyl) amino)-7H-pyrrolo[2,3-d]pyrimidin-5-yl)(2-fluorophenyl)methanone,
(4-((2-(dimethylphosphoryl)phenyl)amino)-2-((4-(4-methylpiperazin-1-yl)phenyl)amino)-7H-pyrrolo[2,3-d]pyrimidin-5-yl)(4-fluorophenyl)methanone,
(R)-(4-fluorophenyl)(2-((4-(4-methylpiperazin-1-yl)phenyl)amino)-4-(1-(methylsulfonyl)piperidin-3-yl)amino)-7H-pyrrolo[2,3-d]pyrimidin-5-yl)methanone,
(S)-(3-((5-(4-fluorobenzoyl)-2-(4-(4-methylpiperazin-1-yl)phenyl)amino)-7H-pyrrolo[2,3-d]pyrimidin-4-yl)amino)pyrrolidin-1-yl)(4-fluorophenyl)methanone,
(S)-1-(3-((5-(4-fluorobenzoyl)-2-((4-(4-methylpiperazin-1-yl)phenyl)amino)-7H-pyrrolo[2,3-d]pyrimidin-4-yl)amino)pyrrolidin-1-yl)ethyl-1-one,
(4-fluorophenyl)(4-(((1r,4r)-4-hydroxycyclohexyl)amino)-2-((4-(4-methylpiperazin-1-yl)phenyl)amino)-7H-pyrrolo[2,3-d]pyrimidin-5-yl)methanone,
(R)-(4-fluorophenyl)(2-((4-(4-methylpiperazin-1-yl)phenyl)amino)-4-(tetrahydro-2H-pyran-3-yl)amino)-7H-pyrrolo[2,3-d]pyrimidin-5-yl)methanone,
(4-fluorophenyl)(4-(((1s,4s)-4-(hydroxymethyl)cyclohexyl)amino)-2-((4-(4-methylpiperazin-1-yl)phenyl)amino)-7H-pyrrolo[2,3-d]pyrimidin-5-yl)methanone,
(4-fluorophenyl)(2-((4-(4-methylpiperazin-1-yl)phenyl)amino)-4-(1-(tetrahydro-2H-pyran-4-yl)piperidin-4-yl)amino)-7H-pyrrolo[2,3-d]pyrimidin-5-yl)methanone,
(4-((1-(cyclopropylsulfonyl)piperidin-4-yl)amino)-2-((4-(4-methylpiperazin-1-yl)phenyl)amino)-7H-pyrrolo[2,3-d]pyrimidin-5-yl)(4-fluorophenyl)methanone,
1-(4-((5-(4-fluorobenzoyl)-2-((4-(4-methylpiperazin-1-yl)phenyl)amino)-7H-pyrrolo[2,3-d]pyrimidin-4-yl)amino)piperidin-1-yl)-2,2-dimethylpropan-1-one,
(S)-(4-fluorophenyl)(2-((4-(4-methylpiperazin-1-yl)phenyl)amino)-4-(tetrahydrofuran-3-yl)amino)-7H-pyrrolo[2,3-d]pyrimidin-5-yl)methanone,
(4-fluorophenyl)(2-((4-(4-methylpiperazin-1-yl)phenyl)amino)-4-(oxetan-3-ylamino)-7H-pyrrolo[2,3-d]pyrimidin-5-yl)methanone,
(4-fluorophenyl)(4-(((1r,3s,5R,7S)-3-hydroxyadamantan-1-yl)amino)-2-((4-(4-methylpiperazin-1-yl)phenyl)amino)-7H-pyrrolo[2,3-d]pyrimidin-5-yl)methanone,
(S)-(4-fluorophenyl)(4-((1-hydroxy-4-methylpentan-2-yl)amino)-2-((4-(4-methylpiperazin-1-yl)phenyl)amino)-7H-pyrrolo[2,3-d]pyrimidin-5-yl)methanone,
(R)-(4-fluorophenyl)(4-(1-hydroxy-3,3-dimethylbut-2-yl)amino)-2-((4-(4-methylpiperazin-1-yl)phenyl)amino)-7H-pyrrolo[2,3-d]pyrimidin-5-yl)methanone,
(4-fluorophenyl)(2-((4-(4-methylpiperazin-1-yl)phenyl)amino)-4-((tetrahydro-2H-pyran-4-yl) methyl)amino)-7H-pyrrolo[2,3-d]pyrimidin-5-yl)methanone,
(4-fluorophenyl)(4-((1-(hydroxymethyl)cyclopropyl)amino)-2-((4-(4-methylpiperazin-1-yl)phenyl)amino)-7H-pyrrolo[2,3-d]pyrimidin-5-yl)methanone,

(4-fluorophenyl)(4-((1r,4r)-4-methoxycyclohexyl)amino)-2-((4-(4-methylpiperazin-1-yl)phen yl)amino)-7H-pyrrolo[2,3-d]pyrimidin-5-yl)methanone,
(4-fluorophenyl)(2-((4-(4-methylpiperazin-1-yl)phenyl)amino)-4-((2-morpholinoethyl)amino) -7H-pyrrolo[2,3-d]pyrimidin-5-yl)methanone,
(4-((2-(dimethylamino)ethyl)amino)-2-((4-(4-methylpiperazin-1-yl)phenyl)amino)-7H-pyrrol o[2,3-d]pyrimidin-5-yl)(4-fluorophenyl)methanone,
(4-fluorophenyl)(2-((4-(4-methylpiperazin-1 -yl)phenyl)amino)-4-((2-(piperidin-1 -yl)ethyl)am ino)-7H-pyrrolo[2,3-d]pyrimidin-5-yl)methanone,
(2-((3-fluoro-4-(4-methylpiperazin-1-yl)phenyl)amino)-4-(((1r,4r)-4-(hydroxymethyl)cyclohe xyl)amino)-7H-pyrrolo[2,3-d]pyrimidin-5-yl)(4-fluorophenyl)methanone,
(4-fluorophenyl)(4-(((1r,4r)-4-(hydroxymethyl)cyclohexyl)amino)-2-((2-methoxy-4-(4-methy lpiperazin-1-yl)phenyl)amino)-7H-pyrrolo[2,3-d]pyrimidin-5-yl)methanone,
(2-((3-fluoro-4-morpholinophenyl)amino)-4-(((1r,4r)-4-(hydroxymethyl)cyclohexyl)amino)-7 H-pyrrolo[2,3-d]pyrimidin-5-yl)(4-fluorophenyl)methanone,
(4-fluorophenyl)(4-((1-isopropylpiperidin-4-yl)amino)-2-((4-(4-methylpiperazin-1-yl)phenyl) amino)-7H-pyrrolo[2,3-d]pyrimidin-5-yl)methanone, (4-fluorophenyl)
(2-((4-(4-methylpiperazin-1-yl)phenyl)amino)-4-((1-methylpiperidin-4-yl)amino)-7H-pyrrolo [2,3-d]pyrimidin-5-yl)methanone,
(4-fluorophenyl)(4-((3-(hydroxymethyl)phenyl)amino)-2-((4-(4-methylpiperazin-1-yl)phenyl) amino)-7H-pyrrolo[2,3-d]pyrimidin-5-yl)methanone,
(2-((4-((2-(dimethylamino)ethyl)(methyl)amino)-2-methoxyphenyl)amino)-4-((1r,4r) -4-(hydroxymethyl)cyclohexyl)amino)-7H-pyrrolo[2,3-d]pyrimidin-5-yl)(4-fluorophenyl)met hanone,
(2-((4-(2-(dimethylamino)ethoxy)-2-methoxyphenyl)amino)-4-((1r,4r)-4-(hydroxymethyl)cyc lohexyl)amino)-7H-pyrrolo[2,3-d]pyrimidin-5-yl)(4-fluorophenyl)methanone,
(4-(((1r,4r)-4-aminocyclohexyl)amino)-2-(4-(4-methylpiperazin-1-yl)phenyl)amino)-7H-pyrr olo[2,3-d]pyrimidin-5-yl)(4-fluorophenyl)methanone,
(4-fluorophenyl)(4-(((1r,4r)-4-(hydroxymethyl)cyclohexyl)amino)-2-((1-(1-methylpiperidin-4 -yl)-1H-pyrazol-4-yl)amino)-7H-pyrrolo[2,3-d]pyrimidin-5-yl)methanone,
(3,4-difluorophenyl)(4-((1r,4r)-4-(hydroxymethyl)cyclohexyl)amino)-2-((4-morpholinophenyl )amino)-7H-pyrrolo[2,3-d]pyrimidin-5-yl)methanone,
(2-((4-(2-(dimethylamino)ethoxy)-2-methoxyphenyl)amino)-4-(((1r,4r)-4-(hydroxymethyl)cy clohexyl)amino)-7H-pyrrolo[2,3-d]pyrimidin-5-yl)(4-fluorophenyl)methanone,
(4-fluorophenyl)(4-((3R,6S)-6-(hydroxymethyl)tetrahydro-2H-pyran-3-yl)amino)-2-((4-(4-methylpiperazin-1-yl)phenyl)amino)-7H-pyrrolo[2,3-d]pyrimidin-5-yl)methanone,
(4-fluorophenyl)(4-(((1r,4r)-4-(hydroxymethyl)cyclohexyl)amino)-2-((3-methoxy-1-methyl-1 H-pyrazol-4-yl)amino)-7H-pyrrolo[2,3-d]pyrimidin-5-yl)methanone,
((1r,4r)-4-((2-((4-morpholinophenyl)amino)thieno[2,3-d]pyrimidin-4-yl)amino)cyclohexyl)m ethanol,
(4-(((1r,4r)-4-(hydroxymethyl)cyclohexyl)amino)-2-((4-morpholinophenyl)amino)-7H-pyrrol o[2,3-d]pyrimidin-5-yl)(p-tolyl)methanone,
(4-(((1r,4r)-4-(hydroxymethyl)cyclohexyl)amino)-2-((4-morpholinophenyl)amino)-7H-pyrrol o[2,3-d]pyrimidin-5-yl)(phenyl)methanone, (4-fluorophenyl)(4-
((1-methylpiperidin-4-yl)amino)-2-((4-morpholinophenyl)amino)-7H-pyrrolo[2,3-d]pyrimidin -5-yl)methanone,
(4-fluorophenyl)(4-(((1s,4s)-4-(hydroxymethyl)cyclohexyl)amino)-2-((4-morpholinophenyl)a mino)-7H-pyrrolo[2,3-d]pyrimidin-5-yl)methanone,(2-((4-(4-ethylpiperazin-1-yl)phenyl)amin o)-4-(((1s,4s)-4-(hydroxymethyl)cyclohexyl)amino)-7H-pyrrolo[2,3-d]pyrimidin-5-yl)(4-fluor ophenyl)methanone,
(2-chloro-4-fluorophenyl)(4-(((1r,4r)-4-(hydroxymethyl)cyclohexyl)amino)-2-((4-morpholino phenyl)amino)-7H-pyrrolo[2,3-d]pyrimidin-5-yl)methanone,
(4-fluorophenyl)(4-(((1s,4s)-4-(hydroxymethyl)cyclohexyl)amino)-2-((1-(1-methylpiperidin-4 -yl)-1H-pyrazol-4-yl)amino)-7H-pyrrolo[2,3-d]pyrimidin-5-yl)methanone,
(4-fluorophenyl)(4-(((1s,4s)-4-(hydroxymethyl)cyclohexyl)amino)-2-((3-methoxy-1-methyl-1 H-pyrazol-4-yl)amino)-7H-pyrrolo[2,3-d]pyrimidin-5-yl)methanone, and
(4-fluorophenyl)(4-((1R,4S)-4-hydroxymethyl)cyclopent-2-en-1-yl)amino)-2-((4-(4-methylpi perazin-1-yl)phenyl)amino)-7H-pyrrolo[2,3-d]pyrimidin-5-yl)methanone.

**12.** A method for synthesizing the compound of formula I according to claim 1, wherein the method is as follows:

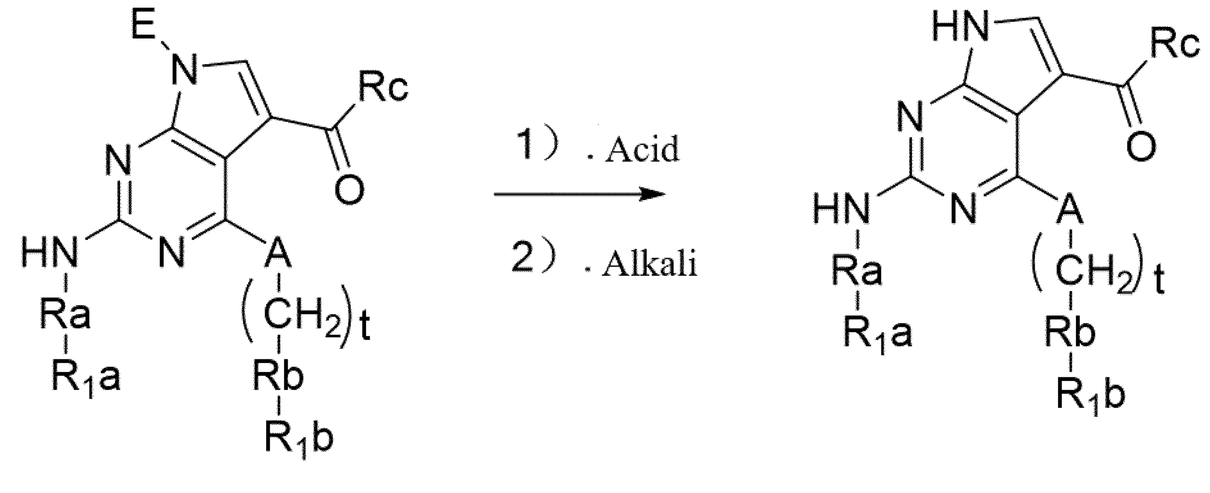


I-d

Formula I

1) removing the E group from the compound I-d under the acid condition, wherein the E is selected from one of trimethylsiloxyethyl methyl, benzyloxycarbonyl, p-toluenesulfonyl, benzenesulfonyl, acetyl, trifluoroacetyl, fluorenylmethoxycarbonyl, and benzyl; and

2) neutralizing the product obtained in step 1) under the alkali condition to obtain the compound of formula I.

**13.** The method for preparing the compound of formula I according to claim 12, wherein a method for preparing the I-d is as follows:

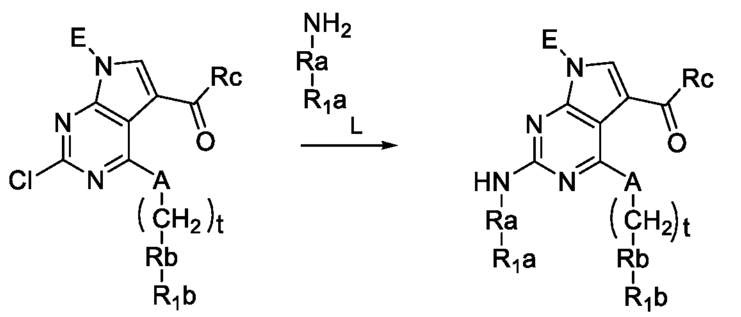


I-c

I-d

,

reacting the compound I-c with the amine L under the heating condition to generate the compound I-d.

**14.** The method for preparing the compound of formula I according to claim 13, wherein a method for preparing the I-c is as follows:

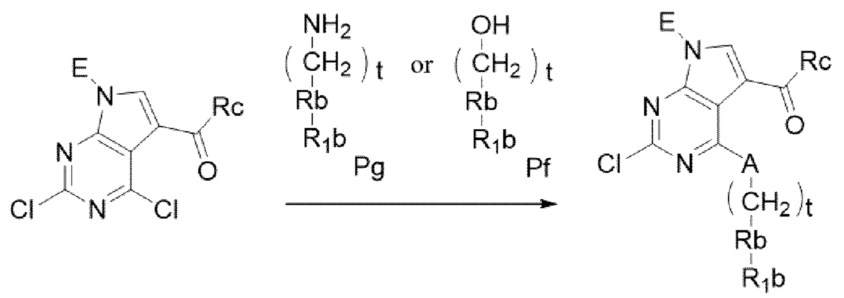


I-b

I-c

reacting the compound I-b with the amine Pg or the alcohol Pf under the heating condition to generate the compound I-c, wherein preferably, the heating is performed at temperature of 80-100°C.

**15.** The method for preparing the compound of formula I according to claim 14, wherein a method for preparing the I-b is as follows:

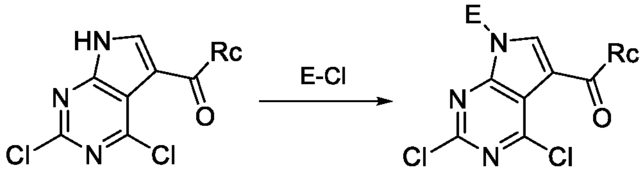


I-a I-b ,

reacting the compound I-a with the E-Cl under the heating condition to generate the compound I-b, wherein preferably, the heating is performed at temperature of 30-60°C.

**16.** The method for preparing the compound of formula I according to claim 15, wherein a method for preparing the I-a is as follows:

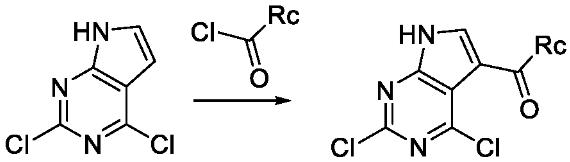

I-a ,

reacting the 2,4-dichloro-7H-pyrrolo[2,3-d]pyrimidine with the

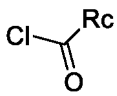

under the ice bath condition to obtain the compound I-a.

**17.** A pharmaceutical composition comprising the compound or the pharmaceutically acceptable salt thereof according to any one of claims 1-11, and one or more pharmaceutically acceptable pharmaceutical excipients.

**18.** Use of the compound or the pharmaceutically acceptable salt thereof according to any one of claims 1-11 or the pharmaceutical composition according to claim 18 in the preparation of an oral formulation or a parenteral formulation, wherein the oral formulation is a tablet, a pill, a granule, a powder, a capsule, a syrup, an emulsion, and a microemulsion; and the parenteral formulation is an injection.

**19.** Use of the compound or the pharmaceutically acceptable salt thereof according to any one of claims 1-11 or the pharmaceutical composition according to claim 18 in regulating and controlling an EGFR tyrosine kinase activity or preventing and treating an EGFR-related disease.

**20.** The use according to claim 19, wherein the regulating and controlling an EGFR tyrosine kinase activity or preventing and treating an EGFR-related disease comprises tumor, diabetes, atherosclerosis, immune system diseases, neurodegenerative diseases, or cardiovascular diseases.

**21.** The use according to claim 20, wherein the tumor is selected from lung cancer, liver cancer, ascitic tumor, metastatic encephaloma, colon cancer, pancreatic cancer, breast cancer, prostate cancer, brain cancer, ovarian cancer, cervical cancer, testicular cancer, kidney cancer, head and neck cancer, lymphoma, melanoma, leukemia, esophageal cancer, stomach cancer, skin cancer, rectal cancer, adrenal cancer, thyroid tumor, and complications thereof.

**22.** The use according to claim 21, wherein the tumor is selected from lung cancer, liver cancer, breast cancer, ascitic tumor, pancreatic cancer, and metastatic encephaloma.

**23.** The use according to claim 22, wherein the lung cancer is selected from non-small cell lung cancer or small cell lung cancer.

**24.** The use according to claim 23, wherein the non-small cell lung cancer is selected from lung adenocarcinoma, lung squamous cell carcinoma, or large cell lung cancer.

**25.** The use according to claim 24, wherein the non-small cell lung cancer is lung adenocarcinoma.

**26.** The use according to claim 21, wherein the lung cancer is selected from EGFR-mutant lung cancer, KRAS-mutant lung cancer, HER2-mutant lung cancer, PIK3CA-mutant lung cancer, BRAF-mutant lung cancer, *MET* gene-mutant lung cancer, *ALK* gene rearrangement lung cancer, *ROS1* gene rearrangement lung cancer, and *RET* gene rearrangement lung cancer.

**27.** The use according to claim 27, wherein the EGFR-mutant lung cancer is selected from EGFR-Del19-mutant lung cancer, EGFR-L858R-mutant lung cancer, EGFR-C797S-mutant lung cancer, EGFR-C797S/T790M-mutant lung cancer, EGFR-L858R/T790M-mutant lung cancer, EGFR-Del19/T790M-mutant lung cancer, EGFR-L858R/T790M/C797S-mutant lung cancer, EGFR-Del 19/T790MIC 797 S -mutant lung cancer, EGFR D770-N771 ins NPG-mutant lung cancer, or EGFR D770-N771 ins NPG/T790M-mutant lung cancer.

**28.** The use according to claim 27, wherein non-small cell lung cancer of the EGFR-mutant lung cancer is EGFR Del19/T790M/C797S-mutant lung adenocarcinoma.

**29.** The use according to claim 27, wherein the non-small cell lung cancer of the EGFR-mutant lung cancer is selected from EGFR D770-N771 ins NPG mutation or EGFR D770-N771 ins NPG/T790M mutation.

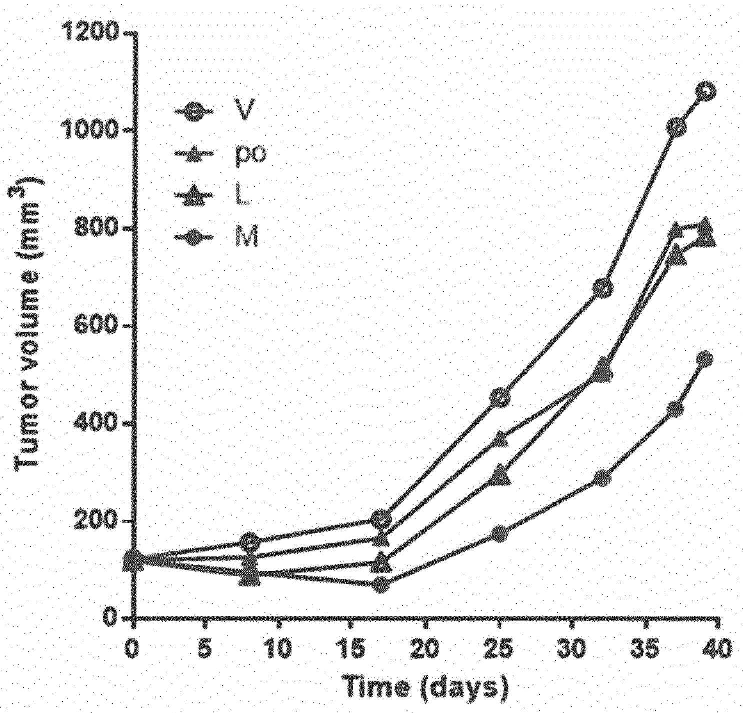


Fig. 1 shows a tumor volume curve of mice in each group

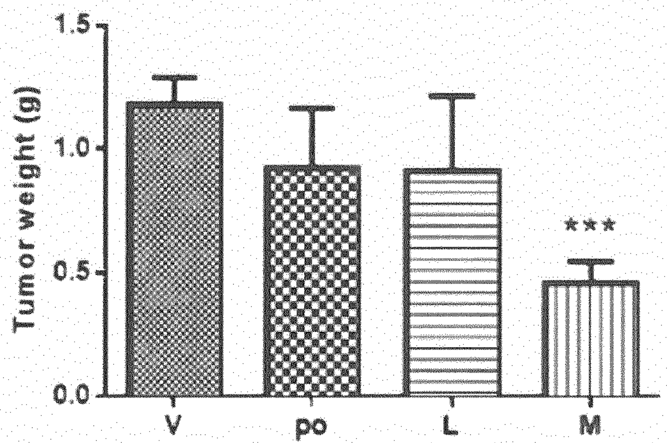


Fig. 2 shows a tumor weight curve of mice in each group

## INTERNATIONAL SEARCH REPORT

International application No. **PCT/CN2022/119451**

**A. CLASSIFICATION OF SUBJECT MATTER**

C07D 487/04(2006.01)i; A61P 35/00(2006.01)i; A61K 31/519(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNABS; DWPI; VEN; CNKI; CNTXT; USTXT; WOTXT; EPTXT; STN-REGISTRY; STN-CAPLUS; PATENTICS; 超星读秀, DUXIU; ISI-Web of Science: 山东新时代药业有限公司, 张贵民, 张浩, 姚景春, 袁将, 赵桂芳, 李蕊, 李光艳, 赵云, 梁红宝, 张振军, 朱祥霞, structural formula (I), 吡咯并嘧啶, EGFR, 激酶抑制剂, 肿瘤, 癌, +pyrrol+, +pyrimidine+, kinase inhibit+, cancer, tumour, tumor

**C. DOCUMENTS CONSIDERED TO BE RELEVANT**

| Category* | Citation of document, with indication, where appropriate, of the relevant passages | Relevant to claim No. |
|---|---|---|
| A | CN 103814030 A (PFIZER INC.) 21 May 2014 (2014-05-21) claim 1, and description, paragraphs [1321], [1398] and [1409] | 1-29 |
| A | WO 2020149723 A1 (VORONOI CO., LTD. et al.) 23 July 2020 (2020-07-23) claim 1, and description, paragraphs [560] and [657] | 1-29 |
| A | CN 110526941 A (BEIJING SCITECH-MQ PHARMACEUTICALS LIMITED) 03 December 2019 (2019-12-03) entire document | 1-29 |
| A | WO 2019223777 A1 (BEIJING SCITECH-MQ PHARMACEUTICALS LIMITED) 28 November 2019 (2019-11-28) entire document | 1-29 |
| A | CN 111825719 A (BEIJING SCITECH-MQ PHARMACEUTICALS LIMITED) 27 October 2020 (2020-10-27) entire document | 1-29 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 November 2022** | **30 November 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/119451**

| Patent document cited in search report | Publication date (day/month/year) | Patent family member(s) | Publication date (day/month/year) |
|---|---|---|---|
| CN 103814030 A | 21 May 2014 | CA 2847540 A1 | 28 March 2013 |
| | | US 2013079324 A1 | 28 March 2013 |
| | | WO 2013042006 A1 | 28 March 2013 |
| | | TW 201313723 A | 01 April 2013 |
| | | AU 2012311184 A1 | 06 March 2014 |
| | | PH 12014500638 A1 | 05 May 2014 |
| | | KR 20140059246 A | 15 May 2014 |
| | | IL 231592 A1 | 28 May 2014 |
| | | MD 20140023 A2 | 30 June 2014 |
| | | MX 2014003501 A1 | 22 July 2014 |
| | | EP 2758402 A1 | 30 July 2014 |
| | | VN 39121 A | 25 September 2014 |
| | | SG 201401445 A1 | 26 September 2014 |
| | | HK 1196354 A0 | 12 December 2014 |
| | | US 9040547 B2 | 26 May 2015 |
| | | ID 201503058 A | 10 July 2015 |
| | | TW 492946 B1 | 21 July 2015 |
| | | EP 2758402 B1 | 27 April 2016 |
| | | JP 5914667 B2 | 11 May 2016 |
| | | IN 201401874 P1 | 13 May 2016 |
| | | CA 2847540 C | 17 May 2016 |
| | | ES 2575710 T3 | 30 June 2016 |
| | | EP 2758402 B9 | 14 September 2016 |
| | | ES 2575710 T9 | 17 October 2016 |
| | | BR 112014006840 A2 | 04 April 2017 |
| | | JP 2014526549 A | 06 October 2014 |
| WO 2020149723 A1 | 23 July 2020 | KR 20200090636 A | 29 July 2020 |
| | | EP 3915991 A1 | 01 December 2021 |
| | | AU 2020209789 A1 | 16 September 2021 |
| | | CN 113614086 A | 05 November 2021 |
| | | IN 202137037338 A | 01 October 2021 |
| | | JP 2022523477 W | 25 April 2022 |
| CN 110526941 A | 03 December 2019 | None | |
| WO 2019223777 A1 | 28 November 2019 | CN 111836819 A | 27 October 2020 |
| CN 111825719 A | 27 October 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CAREY ; SUNDBERG.** ADVANCED ORGANIC CHEMISTRY. Plenum Press, 2000, vol. A,B
- Remingtong's Pharmaceutical Scicences. Mack Publishing Company, 1985, 1418
- *Journal of Pharmaceutical Science,* 1977, vol. 66, 2